(19) Europäisches Patentamt
European Patent Office
Office européen des brevets

(11) EP 2 289 525 A1

(12) EUROPEAN PATENT APPLICATION

(43) Date of publication:
**02.03.2011 Bulletin 2011/09**

(21) Application number: **10171625.6**

(22) Date of filing: **06.06.2003**

(51) Int Cl.:
***A61K 35/14*** *(2006.01)* ***A61K 35/28*** *(2006.01)*
***C12N 5/00*** *(2006.01)* ***A61P 35/00*** *(2006.01)*

(84) Designated Contracting States:
**AT BE BG CH CY CZ DE DK EE ES FI FR GB GR HU IE IT LI LU MC NL PT RO SE SI SK TR**

(30) Priority: **07.06.2002 US 387127 P**

(62) Document number(s) of the earlier application(s) in accordance with Art. 76 EPC:
**03757366.4 / 1 531 850**

(71) Applicant: **ZymoGenetics, Inc.**
**Seattle, WA 98102 (US)**

(72) Inventors:
- **Nelson, Andrew J.**
**Outremont, Québec H12V 1V5 (CA)**
- **Hughes, Steven D.**
**Seattle, WA 98028 (US)**
- **Holly, Richard D.**
**Seattle, WA 98177 (US)**
- **Kindsvogel, Wayne, R.**
**Seattle, WA 98115 (US)**

(74) Representative: **Griffin, Philippa Jane et al**
**Mathys & Squire LLP**
**120 Holborn**
**London**
**EC1N 2SQ (GB)**

Remarks:
This application was filed on 02-08-2010 as a divisional application to the application mentioned under INID code 62.

# (54) Use of IL-21 for expanding lymphoid cells and for anticancer adoptive immunotherapy

(57) There is provided an *in vitro* method for expansion of hematopoietic cells and hematopoietic cell progenitors comprising: culturing bone marrow or peripheral blood cells with a composition comprising an amount of IL-21 sufficient to produce an increase in the number of lymphoid cells in the bone marrow or peripheral blood cells as compared to bone marrow or peripheral blood cells cultured in the absence of IL-21. There are also provided anti-cancer effector cells for use in the treatment of cancer in a patient by adoptive immunotherapy, wherein said anti-cancer effector cells have been obtained from the patient, and wherein the use comprises an initial *ex vivo* step of expanding the anti-cancer effector cells in culture in the presence of IL-21.



Printed by Jouve, 75001 PARIS (FR)

**Description**

REFERENCE TO RELATED APPLICATIONS

This patent application is a continuation-in-part which claims benefit of U.S. Provisional Application Serial No. 60/387,127, filed on June 7, 2002, and is incorporated by reference herein.

BACKGROUND OF THE INVENTION

Cytokines generally stimulate proliferation or differentiation of cells of the hematopoietic lineage or participate in the immune and inflammatory response mechanisms of the body. Examples of cytokines which affect hematopoiesis are erythropoietin (EPO), which stimulates the development of red blood cells; thrombopoietin (TPO), which stimulates development of cells of the megakaryocyte lineage; and granulocyte-colony stimulating factor (G-CSF), which stimulates development of neutrophils. These cytokines are useful in restoring normal blood cell levels in patients suffering from anemia, thrombocytopenia, and neutropenia or receiving chemotherapy for cancer.

The interleukins are a family of cytokines that mediate immunological responses. Central to an immune response is the T cell, which produce many cytokines and adaptive immunity to antigens. Cytokines produced by the T cell have been classified as type 1 and type 2 (Kelso, A. Immun. Cell Biol. 76:300-317, 1998). Type 1 cytokines include IL-2, IFN-$\gamma$, LT-$\alpha$, and are involved in inflammatory responses, viral immunity, intracellular parasite immunity and allograft rejection. Type 2 cytokines include IL-4, IL-5, IL-6, IL-10 and IL-13, and are involved in humoral responses, helminth immunity and allergic response. Shared cytokines between Type 1 and 2 include IL-3, GM-CSF and TNF-$\alpha$. There is some evidence to suggest that Type 1 and Type 2 producing T cell populations preferentially migrate into different types of inflamed tissue.

Mature T cells can activated, i.e., by an antigen or other stimulus, to produce, for example, cytokines, biochemical signaling molecules, or receptors that further influence the fate of the T cell population.

B cells can be activated via receptors on their cell surface including B cell receptor and other accessory molecules to perform accessory cell functions, such as production of cytokines.

Natural killer (NK) cells have a common progenitor cell with T cells and B cells, and play a role in immune surveillance. NK cells, which comprise up to 15% of blood lymphocytes, do not express antigen receptors, and therefore do not use MHC recognition as requirement for binding to a target cell. NK cells are involved in the recognition and killing of certain tumor cells and virally infected cells. *In vivo,* NK cells are believed to require activation, however, *in vitro,* NK cells have been shown to kill some types of tumor cells without activation.

Lymphomas are malignancies of the lymphatic system, that are heterogenous in etiology, morphology, and clinical course. Lymphomas are generally classified as either Hodgkins disease or Non-Hodgkins lymphomas. Hodgkins disease is characterized by giant histocytes, whereas absence of the cells encompasses all non-Hodgkins lymphomas. Lymphocytes, which are the primary component of lymph, can be B cell lymphocytes or T cell lymphocytes. Generally, when a lymphoma arises early in cell maturation, the malignancy is more aggressive than malignancies arising from mature cells. Chemotherapy is usually more effective in treating aggressive lymphoma, whereas indolent lymphomas cannot be treated as easily and therefore are likely never to be cured as long as the disease remains indolent. For a survey of information relating to lymphoma, see, e.g. Lymphoma Treatments and Managing Their Side Effects, Lymphoma Res. Found. Of Amer., Los Angeles, 2001.

In particular, interleukins are a family of cytokines that mediate immunological responses. Central to an immune response is the T cell, which produce many cytokines and effect adaptive immunity to antigens. Mature T cells can be activated, i.e., by an antigen or other stimulus, to produce, for example, cytokines, biochemical signaling molecules, or receptors that further influence the fate of the T cell population.

Viral infections can be classified in various ways. For example, viruses may be classified phylogenetically, according to the infected target cell or organ, or by the disease state they induce. However, not all viruses and viral diseases are treated identically because additional factors, such as whether an infection is acute or chronic and the patient's underlying health, influence the type of treatment that is recommended. Generally, treatment of acute infections in immunocompetent patients should reduce the disease's severity, complications, and decrease the rate of transmission, making safety, cost, and convenience essential considerations in recommending an antiviral agent. Treatments for chronic infections should prevent viral damage to organs such as liver, lungs, heart, central nervous system, and gastrointestinal sytem, making efficacy the primary consideration.

There are few effective treatments for hepatitis. For the treatment of hepatitis B virus (HBV) and hepatitus C virus (HCV), the FDA has approved administration of recombinant interferon alpha (IFN-$\alpha$). However, IFN-$\alpha$ is associated with a number of dose-dependent adverse effects, including thrombocytopenia, leukopenia, bacterial infections, and influenza-like symptoms. Other agents used to treat chronic HBV or HCV include the nucleoside analog RIBAVIRIN™ and ursodeoxycholic acid; however, neither has been shown to be very effective. RIBAVIRIN™ + IFN combination therapy results in 47% rate of sustained viral clearance (Lanford, R.E. and Bigger, C. Virology 293: 1-9 (2002). (See, Medicine, (D. C. Dale and D. D. Federman, eds.) (Scientific American, Inc., New York), 4:VIII: 1-8 (1995)).

Progressive chronic liver disease as a result of chronic infections, such as HCV and HBV, and tumorigenesis associated with HIV are three examples of diseases that can be treated with intervention therapy and/or preventative therapy using the methods of the present invention. The present invention provides methods for treating infections, particularly viral infections, by administering IL-21 to the subject. In certain embodiments, the IL-21 can be administered in conjunction with other antiviral compounds.

In other aspects, the present invention provides such methods for treating solid tumors and lymphomas by administrating IL-21 compositions that may used as a monotherapy or in combination with chemotherapy, radiation therapy, or other biologics. These and other uses should be apparent to those skilled in the art from the teachings herein.

These and other uses should be apparent to those skilled in the art from the teachings herein.

## SUMMARY OF THE INVENTION

Within one aspect, the present invention provides a method of treating Non-Hodgskins lymphoma comprising administering to a subject in need thereof a therapeutically effective amount of a polypeptide having a functional activity of IL-21. In certain embodiments, the polypeptide has been shown to not cause proliferation of isolated cancer cells prior to administration to the subject.

In another aspect, the present invention provides a method of treating cancer comprising administering to subject a therapeutically effective amount of a polypeptide having a functional activity of IL-21, wherein the cancer is selected from the group of renal cell carcinoma, epithelial carcinoma, breast cancer, prostate cancer, ovarian cancer and colon cancer. In one embodiment, there is a tumor response. In another embodiment, the tumor response is measured as complete response, partial response or reduction in time to progression.

In another aspect, the present invention provides a method of treating Non-Hodgskins lymphoma comprising administering to a subject in need thereof a therapeutically effective amount of a fusion protein comprising a first polypeptide having a functional activity of IL-21 and a second polypeptide. In other embodiments, the methods provide the cancer is selected from the group of renal cell carcinoma, epithelial carcinoma, breast cancer, prostate cancer, ovarian cancer and colon cancer. In one embodiment, there is a tumor response. In another embodiment, the tumor response is measured as complete response, partial response or reduction in time to progression.

In another aspect, the methods of the present invention provide a method of treating an infection comprising administering a therapeutically effective amount of a polypeptide having a functional activity of IL-21, wherein the infection is selected from the group of hepatitis B virus, hepatitis C virus, human immunodeficiency virus, sudden acute respiratory syndrome caused by a coronovirus, Herpes Simplex viruses, Epstein-Barr virus, Cytomegalovirus; Pox viruses; Papilloma virus; Adenovirus, Poliovirus; Orthomyxoviruses, Paramyxoviruses, Influenza viruses; caliciviruses; rabies viruses, and rinderpest viruses.

In another aspect, the present invention provides a method of treating a viral infection in a mammal comprising administering a therapeutically effective amount of a polypeptide having a functional activity of IL-21, wherein the viral infection results in a disease selected from the group consisting of Acquired immunodeficiency; Hepatitis; Gastroenteritis; Hemorrhagic diseases; Enteritis; Carditis; Encephalitis; Paralysis; Brochiolitis; Upper or lower respiratory disease; Respiratory Papillomatosis; Arthritis; Disseminated disease, Meningitis, and Mononucleosis.

In one aspect, the present invention provides a method of treating an infection comprising administering a therapeutically effective amount of a fusion protein comprising a first polypeptide having a functional activity of IL-21 and a second polypeptide, wherein the infection is selected from the group of hepatitis B virus, hepatitis C virus, human immunodeficiency virus, sudden acute respiratory syndrome caused by a coronovirus, Herpes Simplex viruses, Epstein-Barr virus, Cytomegalovirus; Pox viruses; Papilloma virus; Adenovirus, Poliovirus; Orthomyxoviruses, Paramyxoviruses, Influenza viruses; caliciviruses; rabies viruses, and rinderpest viruses.

In certain embodiments, the methods of treating viral infections using polypeptides and fusion proteins having a functional activity of IL-21, the level of viral infection is reduced. In other embodiments, the reduction in the level of viral infection is measured as reduction in viral load, increased viral-specific antibodies, reduction in alanine aminotransferase level (ALT), or histologic improvement in a target tissue as measured by immununohistochemistry.

The present invention also provides a method of treating a bacterial infection in a mammal comprising administering a therapeutically effective amount of a polypeptide having a functional activity of IL-21, wherein the bacterial infection is an infection by a bacteria selected from the group consisting of *chlamydiae, listeriae, helicobacter pylori, mycobacterium, mycoplasma, salmonella, and shigella.*

In other aspects, the present invention provides a method of treating a bacterial infection in a mammal comprising administering a therapeutically effective of a fusion protein comprising a first polypeptide that has the functional activity of IL-21 and a second polypeptide, wherein the bacterial infection is an infection by a bacteria selected from the group consisting of *chlamydiae, listeriae, helicobacter pylori, mycobacterium, mycoplasma, salmonella, and shigella.*

For all aspects and embodiments of the present invention, the polypeptide or first polypeptide in a fusion protein can comprise a polypeptide has at least 80%, 90%, 95% or complete identity to an IL-21 polypeptide comprising residues 41 (Gln) to 148 (Ile) of SEQ ID NO:2 or residues 32 (Gln) to 162 (Ser) of SEQ ID NO:2.

DESCRIPTION OF THE INVENTION

Prior to setting forth the invention in detail, it may be helpful to the understanding thereof to define the following terms:

The term "affinity tag" is used herein to denote a polypeptide segment that can be attached to a second polypeptide to provide for purification or detection of the second polypeptide or provide sites for attachment of the second polypeptide to a substrate. In principal, any peptide or protein for which an antibody or other specific binding agent is available can be used as an affinity tag. Affinity tags include a poly-histidine tract, protein A (Nilsson et al., EMBO J. 4:1075, 1985; Nilsson et al., Methods Enzymol. 198:3, 1991), glutathione S transferase (Smith and Johnson, Gene 67:31, 1988), Glu-Glu affinity tag (Grussenmeyer et al., Proc. Natl. Acad. Sci. USA 82:7952-4, 1985), substance P, Flag™ peptide (Hopp et al., Biotechnology 6:1204-10, 1988), streptavidin binding peptide, or other antigenic epitope or binding domain. See, in general, Ford et al., Protein Expression and Purification 2: 95-107, 1991. DNAs encoding affinity tags are available from commercial suppliers (e.g., Pharmacia Biotech, Piscataway, NJ).

The term "allelic variant" is used herein to denote any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term allelic variant is also used herein to denote a protein encoded by an allelic variant of a gene.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "cancer" or "cancer cell" is used herein to denote a tissue or cell found in a neoplasm which possesses characteristics which differentiate it from normal tissue or tissue cells. Among such characteristics include but are not limited to: degree of anaplasia, irregularity in shape, indistinctness of cell outline, nuclear size, changes in structure of nucleus or cytoplasm, other phenotypic changes, presence of cellular proteins indicative of a cancerous or pre-cancerous state, increased number of mitoses, and ability to metastasize. Words pertaining to "cancer" include carcinoma, sarcoma, tumor, epithelioma, leukemia, lymphoma, polyp, and scirrus, transformation, neoplasm, and the like.

The term "complementlanti-complement pair" denotes non-identical moieties that form a non-covalently associated, stable pair under appropriate conditions. For instance, biotin and avidin (or streptavidin) are prototypical members of a complement/anti-complement pair. Other exemplary complemerit/anti-complement pairs include receptor/ligand pairs, antibody/antigen (or hapten or epitope) pairs, senselantisense polynucleotide pairs, and the like. Where subsequent dissociation of the complement/anti-complement pair is desirable, the complement/anti-complement pair preferably has a binding affinity of < $10^9$ $M^{-1}$.

The term "complements of a polynucleotide molecule" denotes a polynucleotide molecule having a complementary base sequence and reverse orientation as compared to a reference sequence.

The term "degenerate nucleotide sequence" denotes a sequence of nucleotides that includes one or more degenerate codons (as compared to a reference polynucleotide molecule that encodes a polypeptide). Degenerate codons contain different triplets of nucleotides, but encode the same amino acid residue (i.e., GAU and GAC triplets each encode Asp).

The term "expression vector" is used to denote a DNA molecule, linear or circular, that comprises a segment encoding a polypeptide of interest operably linked to additional segments that provide for its transcription. Such additional segments include promoter and terminator sequences, and may also include one or more origins of replication, one or more selectable markers, an enhancer, a polyadenylation signal, etc. Expression vectors are generally derived from plasmid or viral DNA, or may contain elements of both.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, Nature 316:774-78, 1985).

An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. When used in this context, the term "isolated" does not exclude the presence of the same polypeptide in alternative physical forms, such as dimers or alternatively glycosylated or derivatized forms.

The term "level" when referring to immune cells, such as NK cells, T cells, in particular cytotoxic T cells, B cells and the like, an increased level is either increased number of cells or enhanced activity of cell function.

The term "level" when referring to viral infections refers to a change in the level of viral infection and includes, but is not limited to, a change in the level of CTLs or NK cells (as described above), a decrease in viral load, an increase antiviral antibody titer, decrease in serological levels of alanine aminotransferase, or improvement as determined by histological examination of a target tissue or organ. Determination of whether these changes in level are significant differences or changes is well within the skill of one in the art.

The term "neoplastic", when referring to cells, indicates cells undergoing new and abnormal proliferation, particularly in a tissue where in the proliferation is uncontrolled and progressive, resulting in a neoplasm. The neoplastic cells can be either malignant, i.e. invasive and metastatic, or benign.

The term "operably linked", when referring to DNA segments, indicates that the segments are arranged so that they function in concert for their intended purposes, e.g., transcription initiates in the promoter and proceeds through the coding segment to the terminator.

A "polynucleotide" is a single- or double-stranded polymer of deoxyribonucleotide or ribonucleotide bases read from the 5' to the 3' end. Polynucleotides include RNA and DNA, and may be isolated from natural sources, synthesized *in vitro,* or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired.

A "polypeptide" is a polymer of amino acid residues joined by peptide bonds, whether produced naturally or synthetically. Polypeptides of less than about 10 amino acid residues are commonly referred to as "peptides".

The term "promoter" is used herein for its art-recognized meaning to denote a portion of a gene containing DNA sequences that provide for the binding of RNA polymerase and initiation of transcription. Promoter sequences are commonly, but not always, found in the 5' non-coding regions of genes.

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

The term "receptor" denotes a cell-associated protein that binds to a bioactive molecule (i.e., a ligand) and mediates the effect of the ligand on the cell. Membrane-bound receptors are characterized by a multi-peptide structure comprising an extracellular ligand-binding domain and an intracellular effector domain that is typically involved in signal transduction. Binding of ligand to receptor results in a conformational change in the receptor that causes an interaction between the effector domain and other molecule(s) in the cell. This interaction in turn leads to an alteration in the metabolism of the cell. Metabolic events that are linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids. In general, receptors can be membrane bound, cytosolic or nuclear; monomeric (e.g., thyroid stimulating hormone receptor, beta-adrenergic receptor) or multimeric (e.g., PDGF receptor, growth hormone receptor, IL-3 receptor, GM-CSF receptor, G-CSF receptor, erythropoietin receptor and IL-6 receptor).

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

Molecular weights and lengths of polymers determined by imprecise analytical methods (e.g., gel electrophoresis) will be understood to be approximate values. When such a value is expressed as "about" X or "approximately" X, the stated value of X will be understood to be accurate to $\pm 10\%$.

All references cited herein are incorporated by reference in their entirety.

The present invention is based in part upon the discovery that administration of a therapeutically effective amount of IL-21 results in inhibiting proliferation of certain neoplastic or cancerous cells, either directly or indirectly, thereby limiting the pathological effects caused by cancer. The neoplastic cells include, but are not limited to, certain lymphocytic cells and metastatic cells originating from solid tumors. The present invention also is based on the discovery that administration of a therapeutically effective amount of IL-21 to specific lymphomas and specific solid tumors results in a tumor response. In the examples which follow, animal models and *in vitro* assays demonstrate the activity of IL-21 on biological samples, in particular solid tumors, neoplastic B-lymphocytes and T-lymphocytes.

The present invention is also based on the discovery that IL-21 has antiviral activity against specific acute infections such as influenza and specific chronic infections such as hepatitis. These antiviral effects may be mediated through immune system cells, such as cytotoxic T cells and NK cells. In the description and examples which follow, animals models and *in vitro* assay demonstrate the antiviral activities of I1-21.

A. Description of IL-21 and its receptor.

Human IL-21 (SEQ ID NO:1 and SEQ ID NO:2) was designated IL-21, and is described in commonly-owned U.S. Patent No. 6,307,024, which is incorporated herein by reference. The IL-21 receptor, (previously designated zalphall) now designated IL-21R (SEQ ID NO:5 and SEQ ID NO:6), and heterodimeric receptor IL-21R/IL-2Rγ are described in commonly-owned WIPO Publication No.s WO 0/17235 and WO 01/77171, which are incorporated herein by reference. As described in these publications, IL-21 was isolated from a cDNA library generated from activated human peripheral blood cells (hPBCs), which were selected for CD3. CD3 is a cell surface marker unique to cells of lymphoid origin, particularly T cells.

The amino acid sequence for the IL-21R indicated that the encoded receptor belonged to the Class I cytokine receptor subfamily that includes, but is not limited to, the receptors for IL-2, IL-4, IL-7, IL-15, EPO, TPO, GM-CSF and G-CSF (for a review see, Cosman, "The Hematopoietin Receptor Superfamily" in Cytokine 5(2): 95-106, 1993). The tissue distribution of the receptor suggests that a target for IL-21 is hematopoietic lineage cells, in particular lymphoid progenitor cells and lymphoid cells. Other known four-helical-bundle cytokines that act on lymphoid cells include IL-2, IL-4, IL-7, and IL-15. For a review of four-helical-bundle cytokines, see, Nicola et al., Advances in Protein Chemistry 52: 1-65, 1999 and Kelso, A., Immunol. Cell Biol. 76:300-317, 1998.

For IL-21, the secretory signal sequence is comprised of amino acid residues 1 (Met) to 31 (Gly), and the mature polypeptide is comprised of amino acid residues 32 (Gln) to 162 (Ser) (as shown in SEQ ID NO: 2). In general, cytokines are predicted to have a four-alpha helix structure, with helices A, C and D being most important in ligand-receptor interactions, and are more highly conserved among members of the family. Referring to the human IL-21 amino acid sequence shown in SEQ ID NO:2, an alignment of human IL-21, human IL-15, human IL-4, and human GM-CSF amino acid sequences predicted that IL-21 helix A is defined by amino acid residues 41-56; helix B by amino acid residues 69-84; helix C by amino acid residues 92-105; and helix D by amino acid residues 135-148; as shown in SEQ ID NO: 2. Structural analysis suggests that the A/B loop is long, the B/C loop is short and the C/D loop is parallel long. This loop structure results in an up-up-down-down helical organization. The cysteine residues are absolutely conserved between IL-21 and IL-15. The cysteine residues that are conserved between IL-15 and IL-21 correspond to amino acid residues 71, 78, 122 and 125 of SEQ ID NO: 2. Conservation of some of the cysteine residues is also found in 1L-2, IL-4, GM-CSF and IL-21 corresponding to amino acid residues 78 and 125 of SEQ ID NO: 2. Consistent cysteine placement is further confirmation of the four-helical-bundle structure. Also highly conserved in the family comprising IL-15, IL-2, IL-4, GM-CSF and IL-21 is the Glu-Phe-Leu sequence as shown in SEQ ID NO: 2 at residues 136-138. Further analysis of IL-21 based on multiple alignments predicts that amino acid residues 44, 47 and 135 (as shown in SEQ ID NO: 2) play an important role in IL-21 binding to its cognate receptor. Moreover, the predicted amino acid sequence of murine IL-21 (SEQ ID NO:4) shows 57% identity to the predicted human protein. Based on comparison between sequences of human and murine IL-21 well-conserved residues were found in the regions predicted to encode alpha helices A and D.

The corresponding polynucleotides encoding the IL-21 polypeptide regions, domains, motifs, residues and sequences described herein are as shown in SEQ ID NO:1. The amino acid residues comprising helices A, B, C, and D, and loops A/B, B/C and C/D for IL-21 , IL-2, IL-4, IL-15 and GM-CSF are shown in Table 1.

Table 1

| | Helix A | A/B Loop | Helix B | B/C Loop | Helix C | C/D Loop | Helix D | |
|---|---|---|---|---|---|---|---|---|
| IL-21residue es | 41-56 | 57-68 | 69-84 | 85-91 | 92-105 | 106-134 | 135-148 | SEQ ID NO:2 |

(continued)

| | Helix A | A/B Loop | Helix B | B/C Loop | Helix C | C/D Loop | Helix D | |
|---|---|---|---|---|---|---|---|---|
| IL-2 residues | 36-46 | 47-52 | 53-75 | 76-86 | 87-99 | 100-102 | 103-121 | SEQ ID NO:7 |
| IL-4 residues | 29-43 | 44-64 | 65-83 | 84-94 | 95-118 | 119-133 | 134-151 | SEQ ID NO:8 |
| IL-15 residues | 45-68 | 69-83 | 84-101 1 | 102-106 | 107-119 | 120-133 | 134-160 | SEQ ID NO:9 |
| GM-CSF residues | 30-44 | 45-71 | 72-81 | 82-90 | 91-102 | 103-119 | 120-131 | SEQ ID NO: 10 |

Those skilled in the art will recognize that the sequence disclosed in SEQ ID NO:1 represents a single allele of human IL-21 and that allelic variation and alternative splicing are expected to occur. Allelic variants of this sequence can be cloned by probing cDNA or genomic libraries from different individuals according to standard procedures. Allelic variants of the DNA sequence shown in SEQ ID NO:1, including those containing silent mutations and those in which mutations result in amino acid sequence changes, are within the scope of the present invention, as are proteins which are allelic variants of SEQ ID NO:2. cDNAs generated from alternatively spliced mRNAs, which retain the properties of the IL-21 polypeptide, are included within the scope of the present invention, as are polypeptides encoded by such cDNAs and mRNAs. Allelic variants and splice variants of these sequences can be cloned by probing cDNA or genomic libraries from different individuals or tissues according to standard procedures known in the art.

The present invention also provides isolated IL-21 polypeptides that have a substantially similar sequence identity to the polypeptides of SEQ ID NO:2, or their orthologs. The term "substantially similar sequence identity" is used herein to denote polypeptides comprising at least 70%, at least 80%, at least 90%, at least 95%, or greater than 95% sequence identity to the sequences shown in SEQ ID NO:2, or their orthologs. The present invention also includes polypeptides that comprise an amino acid sequence having at least 70%, at least 80%, at least 90%, at least 95% or greater than 95% sequence identity to the sequence of amino acid residues 1 to 162 or 33 to 162 of SEQ B7 NO:2. The present invention further includes nucleic acid molecules that encode such polypeptides. Methods for determining percent identity are described below.

Percent sequence identity is determined by conventional methods. See, for example, Altschul et al., Bull. Math. Bio. 48:603 (1986), and Henikoff and Henikoff, Proc. Natl. Acad. Sci. USA 89:10915 (1992). Briefly, two amino acid sequences are aligned to optimize the alignment scores using a gap opening penalty of 10, a gap extension penalty of 1, and the "BLOSUM62" scoring matrix of Henikoff and Henikoff (*ibid.*) as shown in Table 2 (amino acids are indicated by the standard one-letter codes).

$$\frac{\text{Total number of identical matches}}{\text{[length of the longer sequence plus the number of gaps introduced into the longer sequence in order to align the two sequences]}} \times 100$$

Table 2

| | | | | | | | | | | | | | | | | | | | | |
|---|---|---|---|---|---|---|---|---|---|---|---|---|---|---|---|---|---|---|---|---|
| A | A4 | R | N | D | C | Q | E | G | H | I | L | K | M | F | P | S | T | W | Y | V |
| R | -1 | 5 | | | | | | | | | | | | | | | | | | |
| N | -2 | 0 | 6 | | | | | | | | | | | | | | | | | |

(continued)

| | | | | | | | | | | | | | | | | | | | | |
|---|---|---|---|---|---|---|---|---|---|---|---|---|---|---|---|---|---|---|---|---|
| D | -2 | -2 | 1 | 6 | | | | | | | | | | | | | | | | |
| C | 0 | -3 | -3 | -3 | 9 | | | | | | | | | | | | | | | |
| Q | -1 | 1 | 0 | 0 | -3 | 5 | | | | | | | | | | | | | | |
| E | -1 | 0 | 0 | 2 | -4 | 2 | 5 | | | | | | | | | | | | | |
| G | 0 | -2 | 0 | -1 | -3 | -2 | -2 | 6 | | | | | | | | | | | | |
| H | -2 | 0 | 1 | -1 | -3 | 0 | 0 | -2 | 8 | | | | | | | | | | | |
| I | -1 | -3 | -3 | -3 | -1 | -3 | -3 | -4 | -3 | 4 | | | | | | | | | | |
| L | -1 | -2 | -3 | -4 | 1 | -2 | -3 | -4 | -3 | 2 | 4 | | | | | | | | | |
| K | -1 | 2 | 0 | -1 | -3 | 1 | 1 | -2 | -1 | -3 | -2 | 5 | | | | | | | | |
| M | -1 | -1 | -2 | -3 | -1 | 0 | -2 | -3 | -2 | 1 | 2 | -1 | 5 | | | | | | | |
| F | -2 | -3 | -3 | -3 | -2 | -3 | -3 | -3 | -1 | 0 | 0 | -3 | 0 | 6 | | | | | | |
| P | -1 | -2 | -2 | -1 | -3 | -1 | -1 | -2 | -2 | -3 | -3 | -1 | -2 | -4 | 7 | | | | | |
| S | 1 | -1 | | 0 | -1 | 0 | 0 | 0 | -1 | -2 | -2 | 0 | -1 | -2 | -1 | 4 | | | | |
| T | 0 | -1 | 0 | -1 | -1 | -1 | -1 | -2 | -2 | -1 | -1 | -1 | -1 | -2 | -1 | 1 | 5 | | | |
| W | -3 | -3 | -4 | -4 | -2 | -2 | -3 | -2 | -2 | -3 | -2 | -3 | -1 | 1 | -4 | -3 | -2 | 11 | | |
| Y | -2 | -2 | -2 | -3 | -2 | -1 | -2 | -3 | 2 | -1 | -1 | -2 | -1 | 3 | -3 | -2 | -2 | 2 | 7 | |
| V | 0 | -3 | -3 | -3 | -1 | -2 | -2 | -3 | -3 | 3 | 1 | -2 | 1 | -1 | -2 | -2 | 0 | -3 | -1 | 4 |

Those skilled in the art appreciate that there are many established algorithms available to align two amino acid sequences. The "FASTA" similarity search algorithm of Pearson and Lipman is a suitable protein alignment method for examining the level of identity shared by an amino acid sequence disclosed herein and the amino acid sequence of a putative variant IL-21. The FASTA algorithm is described by Pearson and Lipman, Proc. Nat'1 Acad. Sci. USA 85:2444 (1988), and by Pearson, Meth. Enzymol. 183:63 (1990).

Variant IL-21 polypeptides or polypeptides with substantially similar sequence identity are characterized as having one or more amino acid substitutions, deletions or additions. These changes are preferably of a minor nature, that is conservative amino acid substitutions (see Table 3) and other substitutions that do not significantly affect the folding or activity of the polypeptide; small deletions, typically of one to about 30 amino acids; and amino- or carboxyl-terminal extensions, such as an amino-terminal methionine residue, a small linker peptide of up to about 20-25 residues, or an affinity tag. The present invention thus includes polypeptides of from about 108 to 216 amino acid residues that comprise a sequence that is at least 80%, preferably at least 90%, and more preferably 95%, 96%, 97%, 98%, 99% or more identical to the corresponding region of SEQ ID NO:2. Polypeptides comprising affinity tags can further comprise a proteolytic cleavage site between the IL-21 polypeptide and the affinity tag. Preferred such sites include thrombin cleavage sites and factor Xa cleavage sites.

Table 3
Conservative amino acid substitutions

| | |
|---|---|
| Basic: | arginine |
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

Determination of amino acid residues that comprise regions or domains that are critical to maintaining structural integrity can be determined. Within these regions one can determine specific residues that will be more or less tolerant of change and maintain the overall tertiary structure of the molecule. Methods for analyzing sequence structure include, but are not limited to alignment of multiple sequences with high amino acid or nucleotide identity, secondary structure propensities, binary patterns, complementary packing and buried polar interactions (Barton, Current Opin. Struct. Biol. 5:372-376, 1995 and Cordes et al., Current Opin. Struct. Biol. 6:3-10, 1996). In general, when designing modifications to molecules or identifying specific fragments determination of structure will be accompanied by evaluating activity of modified molecules.

Amino acid sequence changes are made in IL-21 polypeptides so as to minimize disruption of higher order structure essential to biological activity. For example, where the IL-21 polypeptide comprises one or more helices, changes in amino acid residues will be made so as not to disrupt the helix geometry and other components of the molecule where changes in conformation abate some critical function, for example, binding of the molecule to its binding partners, e.g., A and D helices, residues 44, 47 and 135 of SEQ ID NO: 2. The effects of amino acid sequence changes can be predicted by, for example, computer modeling as disclosed above or determined by analysis of crystal structure (see, e.g., Lapthorn et al., Nat. Struct. Biol. 2:266-268, 1995). Other techniques that are well known in the art compare folding of a variant protein to a standard molecule (e.g., the native protein). For example, comparison of the cysteine pattern in a variant and standard molecules can be made. Mass spectrometry and chemical modification using reduction and alkylation provide methods for determining cysteine residues which are associated with disulfide bonds or are free of such associations (Bean et al., Anal. Biochem. 201:216-226, 1992; Gray, Protein Sci. 2:1732-1748, 1993; and Patterson et al., Anal. Chem. 66:3727-3732, 1994). It is generally believed that if a modified molecule does not have the same cysteine pattern as the standard molecule folding would be affected. Another well known and accepted method for measuring folding is circular dichrosism (CD). Measuring and comparing the CD spectra generated by a modified molecule and standard molecule is routine (Johnson, Proteins 7:205-214, 1990). Crystallography is another well known method for analyzing folding and structure. Nuclear magnetic resonance (NMR), digestive peptide mapping and epitope mapping are also known methods for analyzing folding and structurally similarities between proteins and polypeptides (Schaanan et al., Science 257:961-964, 1992).

A Hopp/Woods hydrophilicity profile of the IL-21 protein sequence as shown in SEQ ID NO:2 can be generated (Hopp et al., Proc. Natl. Acad. Sci.78:3824-3828, 1981; Hopp, J. Immun. Meth. 88:1-18, 1986 and Triquier et al., Protein Engineering 11:153-169,1998). The profile is based on a sliding six-residue window. Buried G, S, and T residues and exposed H, Y, and W residues were ignored. For example, in IL-21, hydrophilic regions include amino acid residues 114-119 of SEQ ID NO: 2, amino acid residues 101-105 of SEQ ID NO: 2, amino acid residues 126-131 of SEQ ID NO: 2, amino acid residues 113-118 of SEQ ID NO: 2, and amino acid residues 158-162 of SEQ ID NO: 2.

Those skilled in the art will recognize that hydrophilicity or hydrophobicity will be taken into account when designing modifications in the amino acid sequence of a IL-21 polypeptide, so as not to disrupt the overall structural and biological profile. Of particular interest for replacement are hydrophobic residues selected from the group consisting of Val, Leu and Ile or the group consisting of Met, Gly, Ser, Ala, Tyr and Trp. For example, residues tolerant of substitution could include residues 100 and 103 as shown in SEQ ID NO: 2. Cysteine residues at positions 71, 78, 122 and 125 of SEQ ID NO: 2, will be relatively intolerant of substitution.

The identities of essential amino acids can also be inferred from analysis of sequence similarity between IL-15, IL-2, IL-4 and GM-CSF with IL-21. Using methods such as "FASTA" analysis described previously, regions of high similarity are identified within a family of proteins and used to analyze amino acid sequence for conserved regions. An alternative approach to identifying a variant IL-21 polynucleotide on the basis of structure is to determine whether a nucleic acid molecule encoding a potential variant IL-21 gene can hybridize to a nucleic acid molecule having the nucleotide sequence of SEQ ID NO:1, as discussed above.

Other methods of identifying essential amino acids in the polypeptides of the present invention are procedures known in the art, such as site-directed mutagenesis or alanine-scanning mutagenesis (Cunningham and Wells, Science 244:1081 (1989), Bass et al., Proc. Natl Acad. Sci. USA 88:4498 (1991), Coombs and Corey, "Site-Directed Mutagenesis and Protein Engineering," in Proteins: Analysis and Design, Angeletti (ed.), pages 259-311 (Academic Press, Inc. 1998)). In the latter technique, single alanine mutations are introduced at every residue in the molecule, and the resultant mutant molecules are tested for biological or biochemical activity as disclosed below to identify amino acid residues that are critical to the activity of the molecule. See also, Hilton et al., J. Biol. Chem. 271:4699 (1996).

The present invention also includes administration of molecules having the functional activity of IL-21. Thus, administration of functional fragments and functional modified polypeptides of IL-21 polypeptides and nucleic acid molecules encoding such functional fragments and modified polypeptides. A "functional" IL-21 or fragment thereof as defined herein is characterized by its proliferative or differentiating activity, by its ability to induce or inhibit specialized cell functions, in particular for immune effector cells, such as NK cells, T cells, B cells and dendritic cells. Functional IL-21 also includes the ability to exhibit anti-cancer and anti-viral effects *in vitro* or *in vivo,* or by its ability to bind specifically to an anti- IL-21 antibody or IL-21 receptor (either soluble or immobilized). As previously described herein, IL-21 is characterized by a four-helical-bundle structure comprising helix A (amino acid residues 41-56), helix B (amino acid residues 69-84), helix C (amino acid residues 92-105) and helix D (amino acid residues 135-148), as shown in SEQ ID NO: 2. Thus, the present invention further provides fusion proteins encompassing: (a) polypeptide molecules comprising one or more of the helices described above; and (b) functional fragments comprising one or more of these helices. The other polypeptide portion of the fusion protein can contributed by another four-helical-bundle cytokine, such as IL-15, IL-2, IL-4 and GM-CSF, or by a non-native and/or an unrelated secretory signal peptide that facilitates secretion of the fusion protein.

Routine deletion analyses of nucleic acid molecules can be performed to obtain functional fragments of a nucleic acid molecule that encodes a IL-21 polypeptide. As an illustration, DNA molecules having the nucleotide sequence of SEQ ID NO:1 or fragments thereof, can be digested with *Bal*31 nuclease to obtain a series of nested deletions. These DNA fragments are then inserted into expression vectors in proper reading frame, and the expressed polypeptides are isolated and tested for IL-21 activity, or for the ability to bind anti-IL-21 antibodies or zalphal 1 receptor. One alternative to exonuclease digestion is to use oligonucleotide-directed mutagenesis to introduce deletions or stop codons to specify production of a desired IL-21 fragment. Alternatively, particular fragments of a IL-21 gene can be synthesized using the polymerase chain reaction.

Standard methods for identifying functional domains are well-known to those of skill in the art. For example, studies on the truncation at either or both termini of interferons have been summarized by Horisberger and Di Marco, Pharmac. Ther. 66:507 (1995). Moreover, standard techniques for functional analysis of proteins are described by, for example, Treuter et al., Molec. Gen. Genet. 240:113 (1993); Content et al., "Expression and preliminary deletion analysis of the 42 kDa 2-5A synthetase induced by human interferon," in Biological Interferon Systems, Proceedings of ISIR-TNO Meeting on Interferon Systems, Cantell (ed.), pages 65-72 (Nijhoff 1987); Herschman, "The EGF Receptor," in Control of Animal Cell Proliferation 1, Boynton et al., (eds.) pages 169-199 (Academic Press 1985); Coumailleau et al., J. Biol. Chem. 270:29270 (1995); Fukunaga et al., J. Biol. Chem. 270:25291 (1995); Yamaguchi et al., Biochem. Pharmacol. 50:1295 (1995); and Meisel et al., Plant Molec. Biol. 30:1 (1996).

Multiple amino acid substitutions can be made and tested using known methods of mutagenesis and screening, such as those disclosed by Reidhaar-Olson and Sauer (Science 241:53 (1988)) or Bowie and Sauer (Proc. Nat'1 Acad. Sci. USA 86:2152 (1989)). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (*e.g.,* Lowman et al., Biochem. 30:10832 (1991), Ladner et al., U.S. Patent No. 5,223,409, Huse, international publication No. WO 92/06204), and region-directed mutagenesis (Derbyshire et al., Gene 46:145 (1986), and Ner et al., DNA 7:127, (1988)).

Variants of the disclosed IL-21 nucleotide and polypeptide sequences can also be generated through DNA shuffling as disclosed by Stemmer, Nature 370:389 (1994), Stemmer, Proc. Natl Acad. Sci. USA 91:10747 (1994), and international publication No. WO 97/20078. Briefly, variant DNA molecules are generated by *in vitro* homologous recombination by random fragmentation of a parent DNA followed by reassembly using PCR, resulting in randomly introduced point mutations. This technique can be modified by using a family of parent DNA molecules, such as allelic variants or DNA molecules from different species, to introduce additional variability into the process. Selection or screening for the desired activity, followed by additional iterations of mutagenesis and assay provides for rapid "evolution" of sequences by selecting for desirable mutations while simultaneously selecting against detrimental changes.

Mutagenesis methods as disclosed herein can be combined with high-throughput, automated screening methods to detect activity of cloned, mutagenized polypeptides in host cells. Mutagenized DNA molecules that encode biologically active polypeptides, or polypeptides that bind with anti-IL-21 antibodies or soluble zalpha11receptor, can be recovered from the host cells and rapidly sequenced using modem equipment. These methods allow the rapid determination of the importance of individual amino acid residues in a polypeptide of interest, and can be applied to polypeptides of unknown structure.

In addition, the proteins of the present invention (or polypeptide fragments thereof) can be joined to other bioactive molecules, particularly other cytokines, to provide multi-functional molecules. For example, one or more helices from IL-21 can be joined to other cytokines to enhance their biological properties or efficiency of production.

The present invention thus provides a series of novel, hybrid molecules in which a segment comprising one or more of the helices of IL-21 is fused to another polypeptide. Fusion is preferably done by splicing at the DNA level to allow expression of chimeric molecules in recombinant production systems. The resultant molecules are then assayed for such properties as improved solubility, improved stability, prolonged clearance half-life, improved expression and secretion levels, and pharmacodynamics. Such hybrid molecules may further comprise additional amino acid residues (e.g. a polypeptide linker) between the component proteins or polypeptides.

Non-naturally occurring amino acids include, without limitation, *trans*-3-methylproline, 2,4-methanoproline, *cis*-4-hydroxyproline, *trans*-4-hydroxyproline, *N*-methylglycine, *allo*-threonine, methylthreonine, hydroxyethylcysteine, hydroxyethylhomocysteine, nitroglutamine, homoglutamine, pipecolic acid, thiazolidine carboxylic acid, dehydroproline, 3- and 4-methylproline, 3,3-dimethylproline, *tert*-leucine, norvaline, 2-azaphenylalanine, 3-azaphenylalanine, 4-azaphenylalanine, and 4-fluorophenylalanine. Several methods are known in the art for incorporating non-naturally occurring amino acid residues into proteins. For example, an *in vitro* system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is typically carried out in a cell-free system comprising an E. *coli* S30 extract and commercially available enzymes and other reagents. Proteins are purified by chromatography. See, for example, Robertson et al., J. Am. Chem. Soc. 113:2722 (1991), Ellman et al., Methods Enzymol. 202:301 (1991), Chung et al., Science 259:806 (1993), and Chung et al., Proc. Nat'l Acad. Sci. USA 90:10145 (1993).

In a second method, translation is carried out in *Xenopus* oocytes by microinjection of mutated mRNA and chemically aminoacylated suppressor tRNAs (Turcatti et al., J. Biol. Chem. 271:19991 (1996)). Within a third method, E. *coli* cells are cultured in the absence of a natural amino acid that is to be replaced (*e.g.,* phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the protein in place of its natural counterpart. See, Koide et al., Biochem. 33:7470 (1994). Naturally occurring amino acid residues can be converted to non-naturally occurring species by *in vitro* chemical modification. Chemical modification can be combined with site-directed mutagenesis to further expand the range of substitutions (Wynn and Richards, Protein Sci. 2:395 (1993). It can advantageous to stabilize IL-21 to extend the half-life of the molecule, particularly for extending metabolic persistence in an active state. To achieve extended half-life, IL-21 molecules can be chemically modified using methods described herein. PEGylation is one method commonly used that has been demonstrated to increase plasma half-life, increased solubility, and decreased antigenicity and immunogenicity (Nucci et al., Advanced Drug Delivery Reviews 6: 133-155, 1991 and Lu et al., Int. J. Peptide Protein Res. 43:127-138,1994).

A limited number of non-conservative amino acids, amino acids that are not encoded by the genetic code, non-naturally occurring amino acids, and unnatural amino acids can substituted for IL-21 amino acid residues.

The present invention also provides polypeptide fragments or peptides comprising an epitope-bearing portion of a IL-21 polypeptide described herein. Such fragments or peptides may comprise an "immunogenic epitope," which is a part of a protein that elicits an antibody response when the entire protein is used as an immunogen. Immunogenic epitope-bearing peptides can be identified using standard methods (see, for example, Geysen et al., Proc. Nat'l Acad. Sci. USA 81:3998 (1983)).

In contrast, polypeptide fragments or peptides may comprise an "antigenic epitope," which is a region of a protein molecule to which an antibody can specifically bind. Certain epitopes consist of a linear or contiguous stretch of amino acids, and the antigenicity of such an epitope is not disrupted by denaturing agents. It is known in the art that relatively short synthetic peptides that can mimic epitopes of a protein can be used to stimulate the production of antibodies against the protein (see, for example, Sutcliffe et al., Science 219:660 (1983)). Accordingly, antigenic epitope-bearing peptides and polypeptides of the present invention are useful to raise antibodies that bind with the polypeptides described herein. Hopp/Woods hydrophilicity profiles can be used to determine regions that have the most antigenic potential (Hopp et al., 1981, *ibid,* and Hopp, 1986, *ibid*.). In IL-21 these regions include: amino acid residues 114-119, 101-105, 126-131, 113-118, and 158-162 of SEQ ID NO: 2.

Antigenic epitope-bearing peptides and polypeptides preferably contain at least four to ten amino acids, at least ten to fourteen amino acids, or about fourteen to about thirty amino acids of SEQ ID NO:2 or SEQ ID NO:4. Such epitope-bearing peptides and polypeptides can be produced by fragmenting a IL-21 polypeptide, or by chemical peptide synthesis, as described herein. Moreover, epitopes can be selected by phage display of random peptide libraries (see, for example, Lane and Stephen, Curr. Opin. Immunol. 5:268 (1993); and Cortese et al., Curr. Opin. Biotechnol. 7:616 (1996)). Standard methods for identifying epitopes and producing antibodies from small peptides that comprise an epitope are described, for example, by Mole, "Epitope Mapping," in Methods in Molecular Biology, Vol. 10, Manson (ed.), pages 105-116 (The Humana Press, Inc. 1992); Price, "Production and Characterization of Synthetic Peptide-Derived Antibodies," in Monoclonal Antibodies: Production. Engineering, and Clinical Application, Ritter and Ladyman (eds.), pages 60-84 (Cambridge University Press 1995), and Coligan et al. (eds.), Current Protocols in Immunology. pages 9.3.1 - 9.3.5 and pages 9.4.1 - 9.4.11 (John Wiley & Sons 1997).

Regardless of the particular nucleotide sequence of a variant IL-21 polynucleotide, the polynucleotide encodes a polypeptide that is characterized by its proliferative or differentiating activity, its ability to induce or inhibit specialized cell functions, or by the ability to bind specifically to an anti-IL-21 antibody or zalpha11 I receptor. More specifically, variant IL-21 polynucleotides will encode polypeptides which exhibit at least 50% and preferably, greater than 70%, 80% or 90%, of the activity of the polypeptide as shown in SEQ ID NO: 2.

For any IL-21 polypeptide, including variants and fusion proteins, one of ordinary skill in the art can readily generate a fully degenerate polynucleotide sequence encoding that variant using the genetic code and methods known in the art.

The present invention further provides a variety of other polypeptide fusions (and related multimeric proteins comprising one or more polypeptide fusions). For example, a IL-21 polypeptide can be prepared as a fusion to a dimerizing protein as disclosed in U.S. Patents Nos. 5,155,027 and 5,567,584. Preferred dimerizing proteins in this regard include immunoglobulin constant region domains. Immunoglobulin- IL-21 polypeptide fusions can be expressed in genetically engineered cells (to produce a variety of multimerie IL-21 analogs). Auxiliary domains can be fused to IL-21 polypeptides to target them to specific cells, tissues, or macromolecules. For example, a IL-21 polypeptide or protein could be targeted to a predetermined cell type by fusing a IL-21 polypeptide to a ligand that specifically binds to a receptor on the surface of that target cell. In this way, polypeptides and proteins can be targeted for therapeutic or diagnostic purposes. A IL-21 polypeptide can be fused to two or more moieties, such as an affinity tag for purification and a targeting domain. Polypeptide fusions can also comprise one or more cleavage sites, particularly between domains. See, Tuan et al., Connective Tissue Research 34:1-9, 1996.

Using the methods discussed herein, one of ordinary skill in the art can identify and/or prepare a variety of polypeptides that have substantially similar sequence identity to residues 1-162 or 33-162 of SEQ ID NO: 2, or functional fragments and fusions thereof, wherein such polypeptides or fragments or fusions retain the properties of the wild-type protein such as the ability to stimulate proliferation, differentiation, induce specialized cell function or bind the IL-21 receptor or IL-21 antibodies.

The IL-21 polypeptides used in the present invention can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., Molecular Cloning: A Laboratory Manual, 2nd ed., Cold Spring Harbor Laboratory Press, Cold Spring Harbor, NY, 1989, and Ausubel et al., eds., Current Protocols in Molecular Biology, John Wiley and Sons, Inc., NY, 1987.

In general, a DNA sequence encoding a IL-21 polypeptide is operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers.

To direct a IL-21 polypeptide or fragment thereof into the secretory pathway of a host cell, a secretory signal sequence (also known as a leader sequence, prepro sequence or pre sequence) is provided in the expression vector. The secretory signal sequence may be that of IL-21, or may be derived from another secreted protein (e.g., t-PA) or synthesized *de novo.* The secretory signal sequence is operably linked to the IL-21 DNA sequence, i.e., the two sequences are joined in the correct reading frame and positioned to direct the newly synthesized polypeptide into the secretory pathway of the host cell. Secretory signal sequences are commonly positioned 5' to the DNA sequence encoding the polypeptide of interest, although certain secretory signal sequences may be positioned elsewhere in the DNA sequence of interest (see, e.g., Welch et al., U.S. Patent No. 5,037,743; Holland et al., U.S. Patent No. 5,143,830).

Cultured mammalian cells are suitable hosts within the present invention. Methods for introducing exogenous DNA into mammalian host cells include calcium phosphate-mediated transfection (Wigler et al., Cell 14:725, 1978; Corsaro and Pearson, Somatic Cell Genetics 7:603, 1981: Graham and Van der Eb, Virology 52:456, 1973), electroporation (Neumann et al., EMBO J. 1:841-5, 1982), DEAE-dextran mediated transfection (Ausubel et al., ibid.), and liposome-mediated transfection (Hawley-Nelson et al., Focus 15:73, 1993; Ciccarone et al., Focus 15:80, 1993, and viral vectors (Miller and Rosman, BioTechniques 7:980-90, 1989; Wag and Finer, Nature Med. 2:714-6, 1996).

A wide variety of suitable recombinant host cells includes, but is not limited to, gram-negative prokaryotic host organisms. Suitable strains of *E. coli* include W3110, K12-derived strains MM294, TG-1, JM-107, BL21, and UT5600. Other suitable strains include: BL21(DE3), BL21(DE3)pLysS, BL21(DE3)pLysE, DH1, DH4I, DH5, DH5I, DH5IF', DH5IMCR, DH10B, DH10B/p3, DH11S, C600, HB101, JM101, JM105, JM109, JM110, K38, RR1, Y1088, Y1089, CSH18, ER1451, ER1647, *E. coli* K12, *E. coli* K12 RV308, *E. coli* K12 C600, *E. coli*HB101, *E. coli* K12 C600 R.sub.k-M.sub.k-, E. *coli* K12 RR1 (see, for example, Brown (ed.), Molecular Biology Labfax (Academic Press 1991)). Other gram-negative prokaryotic hosts can include *Serratia, Pseudomonas, Caulobacter.* Prokaryotic hosts can include gram-positive organisms such as *Bacillus,* for example, *B. subtilis* and *B. thuringienesis,* and *B. thuringienesis* var. *israelensis,* as well as *Streptomyces,* for example, *S. lividans, S. arrabofaciens, S. fradiae,* and *S. griseofuscus.* Suitable strains of *Bacillus subtilus* include BR151, YB886, MI119, MI120, and B170 (see, for example, Hardy, "Bacillus Cloning Methods," in DNA Cloning: A Practical Approach, Glover (ed.) (IRL Press 1985)). Standard techniques for propagating vectors in prokaryotic hosts are well-known to those of skill in the art (see, for example, Ausubel et al. (eds.), Short Protocols in Molecular Biology, 3rd Edition (John Wiley & Sons 1995); Wu et al-, Methods in Gene Biotechnology (CRC Press, Inc. 1997)). In one embodiment, the methods of the present invention use IL-21 expressed in the W3110 strain, which has been deposited at the American Type Culture Collection (ATCC) as ATCC # 27325.

When large scale production of IL-21 using the expression system of the present invention is required, batch fermentation can be used. Generally, batch fermentation comprises that a first stage seed flask is prepared by growing *E. coli* strains expressing IL-21 in a suitable medium in shake flask culture to allow for growth to an optical density (OD) of between 5 and 20 at 600 nm. A suitable medium would contain nitrogen from a source(s) such as ammonium sulfate, ammonium phosphate, ammonium chloride, yeast extract, hydrolyzed animal proteins, hydrolyzed plant proteins or hydrolyzed caseins. Phosphate will be supplied from potassium phosphate, ammonium phosphate, phosphoric acid or sodium phosphate. Other components would be magnesium chloride or magnesium sulfate, ferrous sulfate or ferrous chloride, and other trace elements. Growth medium can be supplemented with carbohydrates, such as fructose, glucose, galactose, lactose, and glycerol, to improve growth. Alternatively, a fed batch culture is used to generate a high yield of IL-21 protein. The IL-21 producing E. coli strains are grown under conditions similar to those described for the first stage vessel used to inoculate a batch fermentation.

Following fermentation the cells are harvested by centrifugation, resuspended in homogenization buffer and homogenized, for example, in an APV-Gaulin homogenizer (Invensys APV, Tonawanda, New York) or other type of cell disruption equipment, such as bead mills or sonicators. Alternatively, the cells are taken directly from the fermentor and homogenized in an APV-Gaulin homogenizer. The washed inclusion body prep can be solubilized using guanidine hydrochloride (5-8 M) or urea (7 - 8 M) containing a reducing agent such as beta mercaptoethanol (10 - 100 mM) or dithiothreitol (5-50 mM). The solutions can be prepared in Tris, phopshate, HEPES or other appropriate buffers. Inclusion bodies can also be solubilized with urea (2-4 M) containing sodium lauryl sulfate (0.1-2%). In the process for recovering purified IL-21 from transformed E. coli host strains in which the IL-21 is accumulates as refractile inclusion bodies, the cells are disrupted and the inclusion bodies are recovered by centrifugation. The inclusion bodies are then solubilized and denatured in 6 M guanidine hydrochloride containing a reducing agent. The reduced IL-21 is then oxidized in a controlled renaturation step. Refolded IL-21 can be passed through a filter for clarification and removal of insoluble protein. The solution is then passed through a filter for clarification and removal of insoluble protein. After the IL-21 protein is refolded and concentrated, the refolded IL-21 protein is captured in dilute buffer on a cation exchange column and purified using hydrophobic interaction chromatography.

It is preferred to purify the polypeptides of the present invention to ≥ 80% purity, more preferably to ≥90% purity, even more preferably ≥95% purity, and particularly preferred is a pharmaceutically pure state, that is greater than 99.9% pure with respect to contaminating macromolecules, particularly other proteins and nucleic acids, and free of infectious and pyrogenic agents. Preferably, a purified polypeptide is substantially free of other polypeptides, particularly other polypeptides of animal origin.

A variety of assays known to those skilled in the art can be utilized to detect antibodies which bind to IL-21 proteins or polypeptides. Exemplary assays are described in detail in Antibodies: A Laboratory Manual, Harlow and Lane (Eds.), Cold Spring Harbor Laboratory Press, 1988. Representative examples of such assays include: concurrent immunoelectrophoresis, radioimmunoassay, radioimmunoprecipitation, enzyme-linked immunosorbent assay (ELISA), dot blot or Western blot assay, inhibition or competition assay, and sandwich assay. In addition, antibodies can be screened for binding to wild-type versus mutant IL-21 protein or polypeptide.

The methods of the present invention also contemplate using chemically modified IL-21 compositions, in which a IL-21 polypeptide is linked with a polymer. Illustrative IL-21 polypeptides are soluble polypeptides that lack a functional transmembrane domain, such as a mature IL-21 polypeptide. Typically, the polymer is water soluble so that the IL-21 conjugate does not precipitate in an aqueous environment, such as a physiological environment. An example of a suitable polymer is one that has been modified to have a single reactive group, such as an active ester for acylation, or an aldehyde for alkylation, In this way, the degree of polymerization can be controlled. An example of a reactive aldehyde is polyethylene glycol propionaldehyde, or mono-(C1-C10) alkoxy, or aryloxy derivatives thereof (see, for example, Harris, et al., U.S. Patent No. 5,252,714). The polymer may be branched or unbranched. Moreover, a mixture of polymers can be used to produce IL-21 conjugates.

IL-21 conjugates used for therapy can comprise pharmaceutically acceptable water-soluble polymer moieties. Suitable water-soluble polymers include polyethylene glycol (PEG), monomethoxy-PEG, mono-(C1-C10)alkoxy-PEG, aryloxy-PEG, poly-(N-vinyl pyrrolidone)PEG, tresyl monomethoxy PEG, PEG propionaldehyde, *bis*-succinirnidyl carbonate PEG, propylene glycol homopolymers, a polypropylene oxide/ethylene oxide co-polymer, polyoxyethylated polyols (*e.g.,* glycerol), polyvinyl alcohol, dextran, cellulose, or other carbohydrate-based polymers. Suitable PEG may have a molecular weight from about 600 to about 60,000, including, for example, 5,000, 12,000, 20,000 and 25,000. A IL-21 conjugate can also comprise a mixture of such water-soluble polymers.

B. The Use of IL-21 for Treating Cancer

Differentiation is a progressive and dynamic process, beginning with pluripotent stem cells and ending with terminally differentiated cells. Pluripotent stem cells that can regenerate without commitment to a lineage express a set of differentiation markers that are lost when commitment to a cell lineage is made. Progenitor cells express a set of differentiation markers that may or may not continue to be expressed as the cells progress down the cell lineage pathway toward maturation. Differentiation markers that are expressed exclusively by mature cells are usually functional properties such as cell products, enzymes to produce cell products, and receptors. The stage of a cell population's differentiation is monitored by identification of markers present in the cell population.

There is evidence to suggest that factors that stimulate specific cell types down a pathway towards terminal differentiation or dedifferentiation affect the entire cell population originating from a common precursor or stem cell. Thus, the present invention includes stimulating or inhibiting the proliferation of lymphoid cells, hematopoietic cells and epithelial cells.

IL-21 was isolated from tissue known to have important immunological function and which contain cells that play a role in the immune system. IL-21 is expressed in CD3+ selected, activated peripheral blood cells, and it has been shown that IL-21 expression increases after T cell activation. Moreover, results of experiments described in the Examples section herein demonstrate that polypeptides of the present invention have an effect on the growth/expansion and/or differentiated state of NK cells or NK progenitors. Factors that both stimulate proliferation of hematopoietic progenitors and activate mature cells are generally known. NK cells are responsive to IL-2 alone, but proliferation and activation generally require additional growth factors. For example, it has been shown that IL-7 and Steel Factor (c-kit ligand) were required for colony formation of NK progenitors. IL-15 + IL-2 in combination with IL-7 and Steel Factor was more effective (Mr6zek et al., Blood 87:2632-2640, 1996). However, unidentified cytokines may be necessary for proliferation of specific subsets of NK cells and/or NK progenitors (Robertson et. al., Blood 76:2451-2438, 1990). A composition comprising IL-21 and IL-15 stimulates NK progenitors and NK cells, with evidence that this composition is more potent than previously described factors and combinations of factors. Moreover, IL-21 promotes NK-cell expansion, and IL-21 can largely overcome the inhibitory effects of IL-4 on NK-cell growth, it synergizes with IL-2 to promote NK cell growth, and IL-21 selectively promotes the expression of IFN-γ and depresses IL-13 expression. These data suggest that IL-21 have an indirect role in treating solid tumors, metastatic tumors and lymphomas by stimulating the immune effector cells resulting in anti-lymphoma activity. In addition, for certain cancerous cells where the IL-21 receptor is expressed, the anticancer effect of IL-21 can be direct.

Additional evidence demonstrates that IL-21 affects proliferation and/or differentiation of T cells and B cells *in vivo.* It is shown that IL-21 can either inhibit or enhance the proliferation of normal B cells depending on the nature of the co-stimulus provided the cells. IL-21 inhibits the proliferation of some B cell lines, but not others even though most non-responder cell lines express IL-21R as measured by specific IL-21 binding. Many human B cell lines will grow in and kill SCID mice Bonnefoix et al., Leukemia and Lymphoma 25:169-178, 1997). Examples herein describe three B-cell lines which are inhibited by IL-21 and three B cell lines which did not respond to IL-21. All of the cell lines were IL-21R positive, and were put into SCID mice to determine if IL-21 could prolong the survival of lymphoma bearing animals. IL-21 exhibited significant efficacy against the three cell lines whose proliferation was inhibited *in vitro.* In a separate experiment, NK-cell depletion of the SCID mice failed to abrogate the IL-21 effect in the IM-9 model, suggesting that NK-cells are not required for the efficacy of IL-21 in this model.

Assays measuring differentiation include, for example, measuring cell markers associated with stage-specific expression of a tissue, enzymatic activity, functional activity or morphological changes (Watt, FASEB, 5:281-284, 1991; Francis, Differentiation 57:63-75, 1994; Raes, Adv. Anim. Cell Biol. Technol. Bioprocesses, 161-171, 1989; all incorporated herein by reference). Alternatively, IL-21 polypeptide itself can serve as an additional cell-surface or secreted marker associated with stage-specific expression of a tissue. As such, direct measurement of IL-21 polypeptide or its receptors expressed on cancer cells, or its loss of expression in a tissue as it differentiates, can serve as a marker for differentiation of tissues.

The classification of lymphomas most commonly used is the REAL classification system (Ottensmeier, Chemico-Biological Interactions 135-136:653-664, 2001.) Specific immunological markers have been identified for classifications of lymphomas. For example, follicular lymphoma markers include CD20+, CD3-, CD10+, CD5-; Small lymphocytic lymphoma markers include CD20+, CD3-, CD10-, CD5+, CD23+; marginal zone B cell lymphoma markers include CD20+, CD3-, CD10-, CD23-; diffuse large B cell lymphoma markers include CD20+, CD3-; mantle cell lymphoma markers include CD20+, CD3-, CD10-, CD5+, CD23+; peripheral T cell lymphoma markers include CD20-, CD3+; primary mediastinal large B cell lymphoma markers include CD20+, CD3-, lymphoblastic lymphoma markers include CD20-, CD3+, Tdt+, and Burkitt's lymphoma markers include CD20+, CD3-, CD10+, CD5- (Decision Resourses, Non-Hodgkins Lymphoma, Waltham, MA., Feb. 2002).

Primary lymphoma specimens are routinely acquired by biopsy of nodal or extra nodal tumors in the diagnosis of lymphoma. For some lymphoid neoplasms, in particular Chronic Lymphocytic leukemia (CLL), the malignant cells can be acquired from the patient's blood. One method for testing whether a specific lymphoma or patient is amenable to treatment with IL-21 is culturing lymphoma cells. Biopsy or blood specimens can be prepared for tissue culture by a combination of methods well known to those skilled in the art. For example, the samples can be prepared by mincing, teasing, enzymatic digestion or density gradient centrifugation (ficoll) (Jacob et al., Blood 75(5):1154-1162, 1990). The tumor cells are then labeled with a fluorescent DNA stain such as carboxyfluorescein diacetate succinimidyl ester (CFSE; Molecular Probes, Eugene, OR) and cultured in IL-21. The distribution of tumor cells that have undergone 1 or more rounds of cell division can be quatitated by flow cytometry, with the cells losing 1/2 of their CFSE intensity with each round of replication. The proportion of inviable cells and the number of apoptotic cells are also analysed analyzed for the effect of IL-21 using 7-AAD and annexin-V staining. The number of surviving cells, the distribution of CFSE staining and the per cent of cells that are apoptotic can all be used to determine whether IL-21 promotes or inhibits the growth and survival of a given malignant specimen. In such an analysis the lymphoma cells are distinguished from normal cells contaminating the specimen by a combination of a B-cell lineage specific marker, immunoglobulin light chain lambda and kappa specific antibody and light scatter properties. For CLL specimens CD5 staining can also be utilized as an aid in defining the malignant cells. As the proportion of cells proliferating *in vitro* is likely to be very low, it is essential that the tumor cells be distinguished from normal cells. A data analysis method such as flow cytometry that can measure multiple parameters on individual cells is preferred.

One exemplary method for determination specific lymphoma sensitivity to IL-21 uses biopsy or blood cells cultured in serum free medium or in medium containing serum or plasma, preferably fetal bovine serum or human serum, at varying doses of IL-21, generally in a range from 0.1 to 10 nM, and including a negative control. At various time points , for example 1 ,2, 4 and 7 days cells are harvested and subjected to flow cytometric methods to determine the distribution of cells that have divided 1 or more times (CFSE intensity), the proportion of inviable cells( 7-AAD staining; Hausner et al., J. Immunol. Methods 247 (1-2):175-186, 2001) and the number of apoptotic cells (annexin-V staining; Lagneaux et al., Br. J. Hematol. 112 (2):344-352, 2001). The number of surviving cells, the distribution of CFSE staining and the per cent of cells that are apoptotic can all be used to determine whether IL-21 promotes or inhibits the growth and survival of a given malignant specimen. In such an analysis the lymphoma cells are distinguished from normal cells contaminating the specimen by a combination of a B-cell lineage specific marker; immunoglobulin light chain lambda and kappa specific antibody and light scatter properties. For CLL specimens CD5 staining can also be utilized as an aid in defining the malignant cells. As the proportion of cells proliferating *in vitro* may be very low, it is critical that the tumor cells be distinguished from normal cells in the data analysis and is why a method such as flow cytometry that can measure multiple parameters on individual cells is useful.

Such testing of individual tumor specimens will provide basis to choose which patients are likely to respond to IL-21 favorably and for which patients IL-21 may be contraindicated. Patients whose malignant lymphocytes proliferate more slowly in response to IL-21 than in control cultures or die more rapidly than control cultures would be considered as candidates for IL-21 therapy. Similarly, a patient whose malignant cells proliferation rate or survival is enhanced by IL-21 in *vitro* would, in general, not be candidates for IL-21 therapy (except as noted below). Once data are accumulated that demonstrates a strong correlation between a particular type of lymphoma (for example, follicular lymphoma or CLL) and sensitivity to IL-21 *in vitro,* the need to test all patients within such a subgroup for their response to IL-21 may be obviated, provided that no patients are found within the group that exhibit increased proliferation in response to IL-21 *in vitro.*

Similarly, direct measurement of IL-21 polypeptide, or its loss of expression in a tissue can be determined in a tissue or in cells as they undergo tumor progression. Increases in invasiveness and motility of cells, or the gain or loss of expression of IL-21 in a pre-cancerous or cancerous condition, in comparison to normal tissue, can serve as a diagnostic for transformation, invasion and metastasis in tumor progression. As such, knowledge of a tumor's stage of progression or metastasis will aid the physician in choosing the most proper therapy, or aggressiveness of treatment, for a given individual cancer patient. Methods of measuring gain and loss of expression (of either mRNA or protein) are well known in the art and described herein and can be applied to IL-21 expression. For example, appearance or disappearance of polypeptides that regulate cell motility can be used to aid diagnosis and prognosis of prostate cancer (Banyard, J. and Zetter, B.R., Cancer and Metast. Rev. 17:449-458, 1999). As an effector of cell motility, IL-21 gain or loss of expression may serve as a diagnostic for lymphoid cancers.

As discussed above, the IM-9 mouse model for cancer demonstrated that antitumor activity is not NK cell dependent. There are several syngeneic mouse models that have been developed to study the influence of polypeptides, compounds or other treatments on tumor progression. In these models, tumor cells passaged in culture are implanted into mice of the same strain as the tumor donor. The cells will develop into tumors having similar characteristics in the recipient mice, and metastasis will also occur in some of the models. Appropriate tumor models for our studies include the Lewis lung carcinoma (ATCC No. CRL-1642) and B16 melanoma (ATCC No. CRL-6323), amongst others. These are both commonly used tumor lines, syngeneic to the C57BL6/J mouse, that are readily cultured and manipulated *in vitro.* Tumors resulting from implantation of either of these cell lines are capable of metastasis to the lung in C57BL6/J mice. The Lewis lung carcinoma model has recently been used in mice to identify an inhibitor of angiogenesis (O'Reilly MS, et al. Cell 79: 315-328,1994). C57BL6/J mice are treated with an experimental agent either through daily injection of recombinant protein, agonist or antagonist or a one time injection of recombinant adenovirus. Three days following this treatment, $10^5$ to $10^6$ cells are implanted under the dorsal skin. Alternatively, the cells themselves can be infected with recombinant adenovirus, such as one expressing IL-21, before implantation so that the protein is synthesized at the tumor site or intracellularly, rather than systemically. The mice normally develop visible tumors within 5 days. The tumors are allowed to grow for a period of up to 3 weeks, during which time they may reach a size of 1500 - 1800 $mm^3$ in the control treated group. Tumor size and body weight are carefully monitored throughout the experiment. At the time of sacrifice, the tumor is removed and weighed along with the lungs and the liver. The lung weight has been shown to correlate well with metastatic tumor burden. As an additional measure, lung surface metastases are counted. The resected tumor, lungs and liver are prepared for histopathological examination, immunohistochemistry, and *in situ* hybridization, using methods known in the art and described herein. The influence of the expressed polypeptide in question, e.g., IL-21, on the ability of the tumor to recruit vasculature and undergo metastasis can thus be assessed. In addition, aside from using adenovirus, the implanted cells can be transiently transfected with IL-21. Use of stable IL-21 transfectants as well as use of induceable promoters to activate IL-21 expression *in vivo* are known in the art and can be used in this system to assess IL-21 induction of metastasis. Moreover, purified IL-21 or IL-21 conditioned media can be directly injected in to this mouse model, and hence be used in this system. For general reference see, O'Reilly MS, et al. Cell 79:315-328, 1994; and Rusciano D, et al. Murine Models of Liver Metastasis. Invasion Metastasis 14:349-361, 1995.

The activity of IL-21 and its derivatives (conjugates) on growth and dissemination of tumor cells derived from human hematologic malignancies can be measured *in vivo.* Several mouse models have been developed in which human tumor cells are implanted into immunodeficient mice (collectively referred to as xenograft models); see, for example, Cattan AR, Douglas E, Leuk. Res. 18:513-22, 1994 and Flavell, DJ, Hematological Oncology 14:67-82, 1996. The characteristics of the disease model vary with the type and quantity of cells delivered to the mouse, and several disease models are known in the art. In an example of this model, tumor cells (e.g. Raji cells (ATCC No. CCL-86)) would be passaged in culture and about $1X10^6$ cells injected intravenously into severe combined immune deficient (SCID) mice. Such tumor cells proliferate rapidly within the animal and can be found circulating in the blood and populating numerous organ systems. Therapies designed to kill or reduce the growth of tumor cells using IL-21 or its derivatives, agonists, conjugates or variants can be tested by administration of IL-21 compounds to mice bearing the tumor cells. Efficacy of treatment is measured and statistically evaluated as increased survival within the treated population over time. Tumor burden may also be monitored over time using well-known methods such as flow cytometry (or PCR) to quantitate the number of tumor cells present in a sample of peripheral blood. For example, therapeutic strategies appropriate for testing in such a model include direct treatment with IL-21 or related conjugates or antibody-induced toxicity based on the interaction of IL-21 with its receptor(s), or for cell-based therapies utilizing IL-21 or its derivatives, agonists, conjugates or variants. The latter method, commonly referred to as adoptive immunotherapy, would involve treatment of the animal with components of the human immune system (i.e. lymphocytes, NK cells, bone marrow) and may include *ex vivo* incubation of cells with IL-21 with or without other immunomodulatory agents described herein or known in the art.

The activity of IL-21 on immune (effector) cell-mediated tumor cell destruction can be measured *in vivo,* using the murine form of the IL-21 protein (SEQ m NO:2) in syngeneic mouse tumor models. Several such models have been developed in order to study the influence of polypeptides, compounds or other treatments on the growth of tumor cells and interaction with their natural host, and can serve as models for therapeutics in human disease. In these models, tumor cells passaged in culture or in mice are implanted into mice of the same strain as the tumor donor. The cells will develop into tumors having similar characteristics in the recipient mice. For reference, see, for example, van Elsas et al., J. Exp. Med. 190:355-66, 1999; Shrikant et al., Immuni 11:483-93, 1999; and Shrikant et al., J. Immunol. 162:2858-66, 1999. Appropriate tumor models for studying the activity of IL-21 on immune (effector) cell-mediated tumor cell destruction include the B16-F10 melanoma (ATCC No. CRL-6457), and the EG.7 thymoma (ATCC No. CRL-2113), described herein, amongst others. These are both commonly used tumor cell lines, syngeneic to the C57BL6 mouse, which are readily cultured and manipulated *in vitro.*

In an example of an *in vivo* model, the tumor cells (e.g. B16-F10 melanoma (ATCC No. CRL-6475) are passaged in culture and about 100,000 cells injected intravenously into C57BL6 mice. In this mode of administration, B16-F10 cells will selectively colonize the lungs. Small tumor foci are established and will grow within the lungs of the host mouse. Therapies designed to kill or reduce the growth of tumor cells using IL-21 or its derivatives, agonists, conjugates or variants can be tested by administration of compounds to mice bearing the tumor cells. Efficacy of treatment is measured and statistically evaluated by quantitation of tumor burden in the treated population at a discrete time point, two to three weeks following injection of tumor cells. Therapeutic strategies appropriate for testing in such a model include direct treatment with IL-21 or its derivatives, agonists, conjugates or variants, or cell-based therapies utilizing IL-21 or its derivatives, agonists, conjugates or variants. The latter method, commonly referred to as adoptive immunotherapy, would involve treatment of the animal with immune system components (i.e. lymphocytes, NK cells, dendritic cells or bone marrow, and the like) and may include *ex* vivo incubation of cells with IL-21 with or without other immunomodulatory agents described herein or known in the art.

Another syngeneic mouse tumor cell line can used to test the anti-cancer efficacy of IL-21 and to identify the immune (effector) cell population responsible for mediating this effect. EG.7ova is a thymoma cell line that has been modified (transfected) to express ovalbumin, an antigen foreign to the host. Mice bearing a transgenic T cell receptor specific for EG.7ova are available (OT-1 transgenics, Jackson Laboratory). CD8 T cells isolated from these animals (OT-I T cells) have been demonstrated to kill EG.7 cells *in vitro* and to promote rejection of the tumor *in vivo.* EG.7ova cells can be passaged in culture and about 1,000,000 cells injected intraperitoneal into C57BL6 mice. Multiple tumor sites are established and grow within the peritoneal cavity. Therapies designed to kill or reduce the growth of tumor cells using IL-21 or its derivatives, agonists, conjugates or variants can be tested by administration of compounds to mice bearing the tumor cells. OT-1 T cells can be administered to the mice to determine if their activity is enhanced in the presence of IL-21. Efficacy of treatment is measured and statistically evaluated by time of survival in the treated populations. Therapeutic strategies appropriate for testing in such models include direct treatment with IL-21 or its derivatives, agonists, conjugates or variants, or cell-based therapies utilizing IL-21 or its derivatives, agonists, conjugates or variants. Ex vivo treatment of cytotoxic T-lymphocytes (CTL) could also be used to test the IL-21 in the cell-based strategy.

Analysis of IL-21 efficacy for treating certain specific types of cancers are preferably made using animals that have been shown to correlate to other mammalian disease, particularly human disease. After IL-21 is administered in these models evaluation of the effects on the cancerous cells or tumors is made. Xenografts are used for most preclinical work, using immunodeficient mice. For example, a syngeneic mouse model for ovarian carcinoma utilizes a C57BL6 murine ovarian carcinoma cell line stably overexpressing VEGF16 isoform and enhanced green fluorescent protein (Zhang et al., Am. J. Pathol. 161:2295-2309, 2002). Renal cell carcinoma mouse models using Renca cell injections have been shown to establish renal cell metastatic tumors that are responsive to treatment with immunotherapeutics such as IL-12 and IL-2 (Wigginton et al., J. of Nat. Cancer Inst. 88:38-43, 1996). A colorectal carcinoma mouse model has been established by implanting mouse colon tumor MC-26 cells into the splenic subcapsule of BALB/c mice (Yao et al., Cancer Res. 63 (3):586-586-592, 2003). An immunotherapeutic-responsive mouse model for breast cancer has been developed using a mouse that spontaneously develops tumors in the mammary gland and demonstrates peripheral and central tolerance to MUC1 (Mukherjee et al., J. Immunotherapy 26:47-42, 2003). To test the efficacy of IL-21 in prostate cancer, animal models that closely mimic human disease have been developed. A transgenic adenocarcinoma of the mouse prostate model (TRAMP) is the most commonly used syngeneic model (Kaplan-Lefko et al., Prostate 55 3 :219-237, 2003; Kwon et al., PNAS 96:15074-15079, 1999; Arap et al., PNAS 99:1527-1531,2002).

IL-21 will be useful in treating tumorgenesis, enhancing CTLs and NK activity, and therefore are useful in the treatment of cancer. In addition to direct and indirect effects on CTLs and NK cells, as shown in several tumor models described herein, IL-21 inhibits IL-4 stimulated proliferation of anti-IgM stimulated normal B-cells and a similar effect is observed in B-cell tumor lines suggesting that there can be therapeutic benefit in treating patients with the IL-21 in order to induce the B cell tumor cells into a less proliferative state.

The ligand could be administered in combination with other agents already in use including both conventional chemotherapeutic agents as well as immune modulators such as interferon alpha. Alpha/beta interferons have been shown to be effective in treating some leukemias and animal disease models, and the growth inhibitory effects of INF-$\alpha$ and IL-21 are additive for at least one B-cell tumor-derived cell line. Establishing the optimal dose level and scheduling for IL-21 is done by a combination of means, including the pharmacokinetics and pharmacodynamics of IL-21, the sensitivity of human B-cell lines and primary lymphoma specimens to IL-21 *in vitro,* effective doses in animal models and the toxicity of IL-21. Optimally, to have a direct anti-tumor effect the concentration of IL-21 in plasma should reach a level that in vitro is maximally active against B-cell lymphoma cell lines and primary lymphomas. In addition the optimal and minimum times of exposure to IL-21 to elicit a growth inhibitory or apoptotic responses can be modeled in vitro with cell lines and primary tumor cells. Direct pharmacokinetic measurements done in primates and clinical trials can then be used to predict theoretical doses in patients that achieve plasma IL-21 levels that are of sufficient magnitude and duration to achieve a biological response in patients. In addition IL-21 stimulates a variety of responses in normal lymphocytes, such that surrogate markers can be employed to measure the biological activty of IL-21 on effector cells in patients.

Since lymphoma patients are treated with a variety of chemotherapeutic drugs and drug combinations, developing a protocol to integrate IL-21 into an existing standard treatment regimen may result in improved therapeutic outcome. The effect of combining chemotherapy drugs and IL-21 is primarily modeled with IL-21 sensitive human B-cell lines *in vitro,* measuring cell proliferation, cell viability, and apoptosis. Time and dose dependent response curves to chemotherapeutic drugs (e.g. chlorambucil, etoposide, or fludaribine) are established for individual cell lines. IL-21 is then tested over a wide range of concentrations under suboptimal conditions of each chemotherapy drug. The order of exposure of the cells to IL-21 versus a chemotherapy agent may significantly affect the outcome of the interaction with the cell line tested. As such, the IL-21 should be introduced to the cultures in several manners to find the optimal mode of treatment. This should include, for example, prior treatment with IL-21 for several hours to several days (0, 4, 24, 48 and 72 hours ), followed by a wash out of IL-21 and the addition of a suboptimal dose/exposure time of a chemotherapy drug. After 1-3 days, analysis of the culture for cell viability, proliferation and apoptosis is made. In a variation to the above experiment, the IL-21 is not washed out prior to the addition of the chemotherpy drug. The complete set of conditions to test would also include the simultaneous treatment of cells with IL-21 and a chemotherapy drug with varying time of IL-21 wash out, as well as delayed (from a few hours to several days) addition of IL-21 until after exposure to a chemotherapy drug. The timing and concentration of IL-21 exposure that gives a maximal reduction in target cell outgrowth/viability or maximum increase in apoptotic response will then be considered optimal for further testing in animal models or the design of a clinical protocol. The combination of IL-21 with chemotherapeutic drugs in vitro using cell lines whose growth is stimulated by IL-21, such as RPMI-1788, could be done to identify drugs that eliminate the potential adverse affects of IL-21 that might be encountered in a subset of patients. Such drugs would be identified from those known to have activity against lymphomas and selected on the basis of their ability in vitro to prevent enhanced growth and or survival of RPMI 1788 or similarly IL-21 responsive cell lines. In this way IL-21 therapy, when combined with selected chemotherapeutic regimens would be of benefit to those patients whose malignancy is sensitve to IL-21 mediated growth suppression, while protecting any patients that might otherwise respond unfavorably to IL-21 monotherapy.

Lymphoma patients are also treated with biologics, such as RITUXAN™, IL-2 and interferon. Those biologic agents that have a direct inhibitory effect on the tumor and are not largely dependent on effector cells for their activity can be modeled in a manner similar to the *in vitro* experiments above for their interaction with IL-21. For example, RITUXAN™ binds to lymphoma cells and can induce apoptosis directly *in vitro,* but is also capable of inducing a variety of effector mechanisms such as complement dependent cytotoxity and antibody dependent cell-mediated cytotoxicity (ADCC). Therefore, it is possible to define conditions *in vitro* in which IL-21 and RITUXAN™ interact in synergy to inhibit lymphoma growth or stimulate apoptosis. The use of a xenogeneic human lymphoma model in SCID mice has the potential to measure a broader range of potential interactions that involve host effector mechanisms between RITUYAN™ (or some other biologic agent) and IL-21. To determine if there is significant synergy between IL-21 and another anti-tumor biologic in a xenogeneic lymphoma SCID mouse model, IL-21 and the other biologic are tested under conditions that yield marginal therapeutic results with either agent alone.

IL-21 and IL-2 exhibit synergy in their effects on NK-cells *in vitro* with respect to IFN-γ production and proliferation. In addition, high dose IL-2 therapy is highly toxic and requires extensive hospitalization. Many low dose regimens of IL-2 have been tested, and found to be well tolerated, but with little evidence of anti-tumor efficacy (Atkins, Semin. Oncol. 29 (3 Suppl. 7):12, 2002). The combination of low dose IL-2 with IL-21 therefore may be clinically useful by augmenting the immune system stimulation of low dose IL-2 while providing a direct anti-lymphoma effect. The effects of combining IL-2 and IL-21 are studied in mouse syngeneic lymphoma models or in SCID mouse xenogeneic human lymphoma models as described herein. The relative effects on effector cell activation and toxicity of combining IL-2 and IL-21 at different doses can be determined in normal primates to optimize a dosing level and schedule to avoid the need to hospitalize patients.

For those patients whose malignant lymphocytes are stimulated to proliferate *in vitro* in response to IL-21, a course of IL-21 could be contraindicated (in the absence of combination with other drugs as discussed above) unless the malignant cells have a very low turn over rate *in vivo,* such as CLL. The relatively quiescent state of CLL cells may be related to the resistance of this disease to chemotherapy. In such cases, patients could be treated by pulsing them with IL-21 just prior to the administration of chemotherapeutic drug(s). The optimal timing of dosing of IL-21 and chemotherapy could be modeled *in vitro* to predict how long after exposure to IL-21 the malignant cells become maximally sensitive to specific chemotherapeutic drugs.

The present invention provides a method of reducing proliferation of a neoplastic B or T cells comprising administering to a mammal with a B or T cell lymphoma an amount of a composition of IL-21 sufficient to reduce proliferation of the B or T lymphoma cells. In other embodiments, the composition can comprise at least one other cytokine selected from the group consisting of IL-2, IL-15, IL-4, IL-18, GM-CSF, Flt3 ligand, interferon, or stem cell factor.

In another aspect, the present invention provides a method of reducing proliferation of a neoplastic B or T cells comprising administering to a mammal with a B or T cell neoplasm an amount of a composition of IL-21 antagonist sufficient to reducing proliferation of the neoplastic B or T cells. In other embodiments, the composition can comprise at least one other cytokine selected from the group consisting of IL-2, IL-15, IL-4, IL-18, GM-CSF, Flt3 ligand, interferon, or stem cell factor. Furthermore, the IL-21 antagonist can be a ligand/toxin fusion protein.

A IL-21-saporin fusion toxin, or other IL-21-toxin fusion, can be employed against a similar set of leukemias and lymphomas, extending the range of leukemias that can be treated with IL-21 . Moreover, such IL-21-toxin fusions can be employed against other cancers wherein IL-21 binds its receptors. Fusion toxin mediated activation of the IL-21 receptor provides two independent means to inhibit the growth of the target cells, the first being identical to the effects seen by the ligand alone, and the second due to delivery of the toxin through receptor internalization. The lymphoid restricted expression pattern of the IL-21 receptor suggests that the ligand-saporin conjugate can be tolerated by patients.

When treatment for malignancies includes allogeneic bone marrow or stem cell transplantion, IL-21 can be valuable in enhancement of the graft-vs-tumor effect. IL-21 stimulates the generation of lytic NK cells from marrow progenitors and stimulates the proliferation of T-cells following activation of the antigen receptors. Therefore, when patients receive allogenic marrow transplants, IL-21 will enhance the generation of anti-cancer responses, with or without the infusion of donor lymphocytes.

Modem methods for cancer immunotherapy are based on the principle that the immune system can detect and defend against spontaneous tumors. Evidence supporting the concept of "Immunological surveillance" (see, Burnet FM Lancet 1:1171-4, 1967), comes in part from epidemiological studies indicating that the incidence of cancer increases in patients that are immunocomprised by disease, such as infection (see, Klein G. Harvey Lect. 69:71-102, 1975; and Kuper et al., J. Intern. Med. 248:171-83, 2000), or following medical interventions such as bone marrow ablation (see, Birkeland et al., Lancet 355:1886-7, 2000; and Penn I, Cancer Detect Prev. 18:241-52, 1994). Experiments performed in gene-targeted mice also show that the immune system modulates susceptibility to spontaneous tumors in aged mice (see, Smyth, M. et al., J. Exp. Med. 192:755-760, 2000; and Davidson, W. et al., J. Exp. Med. 187: 1825-1838, 1998) or following exposure to chemical carcinogens (see, Peng, S et al., J. Exp. Med. 184: 1149-1154, 1996; Kaplan, D. et al., Proc. Nat. Acad Sci. USA 95:7556-7561, 1998; and Shankaran V. et al., Nature 410:1107-1111, 2001). Proof that immune recognition of tumors occurs frequently in tumor bearing hosts comes from the identification of T-cells that are reactive to a broad range of tumor associated antigens including differentiation antigens, mutational antigens, tissue-specific antigens, cancer-testis antigens, self antigens that are over expressed in tumors, and viral antigens (Boon T. et al., Immunol. Today 18:267-8, 1997.). In addition, B-cells are known to produce high titers of circulating IgG antibodies that recognize these same classes of tumor antigens (Stockert E. et al., J. Exp. Med. 187:1349-54, 1998;. Sahin U et al.,. Curr. Opin. Immunol. 9:709-16, 1997; and Jäger, E. et al., Proc. Nat. Acad. Sci. USA 97:12198-12203, 2000), and NK cells have been isolated that can recognize and kill tumor cells that express various stress-related genes (Bauer, S et al., Science 285:727-729, 1999).

The concept that immunotherapy can be an effective method for treating cancer is firmly established in experimental animal models, and while the methodologies are much less advanced for human subjects, there is a strong suggestion that the immune system can be stimulated to reject established disease. The very first attempt at cancer immunotherapy was reported by William Coley in 1893 who, using extracts of pyrogenic bacteria, achieved anti-cancer responses most likely through the induction of systemic inflammation and cell-mediated immunity (Coley WB. The treatment of malignant tumors by repeated inoculations of erysipelas. With a report of ten original cases. 1893, Clin Orthop. 262:3-11, 1991). In more modem times five generalized strategies have been employed to increase the numbers of effector cells and/or modulate their anti-cancer activity (reviewed in Rosenberg, SA. (Ed.), Principles and practice of the biologic therapy of cancer., 3rd edition, Lippincott Williams & Wilkins, Philadelphia, PA, 2000): cytokine therapy, cell transfer therapy, monoclonal antibody therapy, cancer vaccines, and gene therapy. To date, each method has shown effectiveness in mediating an anti-cancer response although the durability of these responses, with a few exceptions, is mostly temporary. This fact reflects our limited understanding of tumor immunology and argues that improvements in the technology await the utilization of previously unrecognized elements of the anti-cancer response. The present invention provides such an element to improve our understanding of tumor immunology as well as provide polypeptides that are therapeutically useful in treating and preventing human cancers.

One requirement for achieving sustained immunity and durable clinical responses is the amplification in the level, i.e., in the numbers and activity of the cells that mediate tumor killing. Thus, new factors that mediate their effects on lymphocytes including cytotoxic T-cells (CTLs), NK cells, and B-cells, as well myeloid cells such as neutrophils and monocytic cells will improve anti-cancer activity. IL-21 is a product of activated $CD4^+$ "helper" T-cells which are required for both humoral and cell-mediated immunity and for sustaining long-term memory to antigenic re-challenge (U.S. Patent No. 6,307,024; Parrish-Novak J et al., Nature 408:57-63, 2000). The receptor for IL-21 is expressed on cells that mediate anti-cancer responses and previous experiments have shown that IL-21 can stimulate the proliferation of these cell types *in vitro* (commonly-owned WIPO Publication No.s WO 0/17235 and WO 01/77171). Additional experiments affirm these IL-21 activities *in vivo.*

IL-21 polypeptides for the methods of the present invention are shown to stimulate CTL and NK cells against tumors *in vivo* in animal models resulting in decreased tumor burden and tumor cells, and increased survival. IL-21 can hence be used in therapeutic anti-cancer applications in humans. As such, IL-21 anti-cancer activity is useful in the treatment and prevention of human cancers. Such indications include but are not limited to the following: Carcinomas (epithelial tissues), Sarcomas of the soft tissues and bone (mesodermal tissues), Adenomas (glandular tissues), cancers of all organ systems, such as liver (hepatoma) and kidney (renal cell carcinomas), CNS (gliomas, neuroblastoma), and hematological cancers, viral associated cancers (e.g., associated with retroviral infections, HPV, hepatitis B and C, and the like), lung cancers, endocrine cancers, gastrointestinal cancers (e.g., biliary tract cancer, liver cancer, pancreatic cancer, stomach cancer and colorectal cancer), genitourinary cancers (e.g., prostate cancer bladder cancer, renal cell carcinoma), gynecologic cancers (e.g., uterine cancer, cervical cancer, ovarian cancer) breast, and other cancers of the reproductive system, head and neck cancers, and others. Of particular interest are hematopoietic cancers, including but not limited to, lymphocytic leukemia, myeloid leukemia, Hodgkin's lymphoma, Non-Hodgkins lymphomas, chronic lymphocytic leukemia, and other leukemias and lymphomas. Moreover IL-21 can be used therapeutically in cancers of various non-metastatic as wells as metastatic stages such as "Stage 1" Localized (confined to the organ of origin); "Stage 2" Regional; "Stage 3" Extensive; and "Stage 4" Widely disseminated cancers. In addition, IL-21 can be used in various applications for cancer, immunotherapy, and in conjunction with chemotherapy and the like.

Administration of IL-21 using the methods of the present invention will result in a tumor response. While each protocol may define tumor response accessments differently, exemplary guidelines can be found in Clinical Research Associates Manual, Southwest Oncology Group, CRAB, Seattle, WA, October 6, 1998, updated August 1999. According to the CRA Manual (see, chapter 7 "Response Accessment"), tumor response means a reduction or elimination of all measurable lesions or metastases. Disease is generally considered measurable if it comprises bidimensionally measurable lesions with clearly defined margins by medical photograph or X-ray, computerized axial tomography (CT), magnetic resonance imaging (MRI), or palpation.. Evaluable disease means the disease comprises unidimensionally measurable lesions, masses with margins not clearly defined, lesion with both diameters less than 0.5 cm, lesions on scan with either diameter smaller than the distance between cuts, palpable lesions with diameter less than 2 cm, or bone disease. Non-evaluable disease includes pleural effusions, ascites, and disease documented by indirect evidence. Previously radiated lesions which have not progressed are also generally considered non-evaluable.

The criteria for objective status are required for protocols to access solid tumor response. A representative criteria includes the following: (1) Complete Response (CR) defined as complete disappearance of all measurable and evaluable disease. No new lesions. No disease related symptoms. No evidence of non-evaluable disease; (2) Partial Response (PR) defined as greater than or equal to 50% decrease from baseline in the sum of products of perpendicular diameters of all measureable lesions. No progression of evaluable disease. No new lesions. Applies to patients with at least one measurable lesion; (3) Progression defined as 50% or an increase of 10 $cm^2$ in the sum of products of measurable lesions over the smallest sum observed using same techniques as baseline, or clear worsening of any evaluable disease, or reappearance of any lesion which had disappeared, or appearance of any new lesion, or failure to return for evaluation due to death or deteriorating condition (unless unrelated to this cancer); (4) Stable or No Response defined as not qualifying for CR, PR, or Progression. (See, Clinical Research Associates Manual, supra.)

Examples of methods for using IL-21 in the treatment of cancer include, but are not limited to, the following:

1) IL-21 can be used as a single agent for direct inhibitory activity against tumors that express the IL-21 receptor (U.S. Patent No. 6,307,024; WIPO Publication No.s WO 0117235 and WO 01/77171). Such activity is shown herein. Administration in a pharmaceutical vehicle for therapeutic use can be achieved using methods in the art and described herein.

2) IL-21 can be conjugated to a toxic compound that binds and kills tumor cells that express IL-21 receptor such as B-cell lymphomas, T-cell lymphomas and NK cell lymphomas. The toxic compound can be a small molecule drug like calichaemicin used in a manner similar to the anti-CD33 antibody + drug conjugate, MYLOTARG™, that is used to treat acute myelogeous leukemia (for example, See, Sievers EL et al.,. J Clin Oncol. 19:3244-54, 2001; and Bernstein ID Clin. Lymphoma Suppl 1:S9-S11, 2002); or a radioisotope like $^{125}I$ (Kaminski MS, et al.. J. Clin. Oncol. 19:3918-28,2001) or $^{90}Y$ (Reviewed in Gordon LI et al., . Semin. Oncol. (1 Suppl 2):87-92, 2002) that has been attached to an anti-CD20 antibody used for the treatment of Non-Hogkin's lymophoma; or a naturally occurring protein toxin such as Ricin A (Lynch TJ Jr, et al., J. Clin. Oncol. 15:723-34, 1997) or diphtheria B toxin that was made as a fusion protein with IL-2 for the treatment of cutaneous T-cell lymphoma (Talpur R et al., Leuk. Lymphoma 43:121-6, 2002). The attachment of these toxic compounds to IL-21 might occur through chemical conjugation (Rapley R. Mol. Biotechnol. 3:139-54, 1995) or genetic recombination (Foss FM. Clin. Lymphoma Suppl 1:S27-31, 2000). Such toxin conjugates with IL-21, for example IL-21 -saporin conjugates, are shown to kill various tumors *in vivo* and *in vitro* (U.S. Patent No. 6,307,024; and described herein).

3) IL-21 can be used as an immunostimulatory agent for cancer monotherapy. A variety of cytokines such as IL-2, IL-4, IL-6, IL-12, IL-15, and interferon, are known to stimulate anti-cancer responses in animal models via stimulation of the immune system (reviewed in Rosenberg, SA ibid.). Moreover IL-21 is shown to also stimulate the immune system (U.S. Patent No. 6,307,024; and described herein). Cytokine monotherapy is an accepted practice for human cancer patients. For example, the use of IL-2 and IFN-α are used for the treatments of metastatic melanoma and renal cell carcinoma (e.g., see, Atkins MB et al., J. Clin. Oncol. 17:2105-16, 1999; Fyfe G et al., J. Clin. Oncol. 13: 688-96, 1995; and Jonasch E, and Haluska FG, Oncologist 6:34-55, 2001). The mechanism of action of these cytokines includes, but is not limited to, an enhancement of a Th1 cell-mediated responses including direct tumor cell killing by CD8+ T-cells and NK cells. IL-21 is shown to similarly enhance Th1 cell-mediated responses including direct tumor cell killing by CTLs, e.g., CD8+ T-cells, and NK cells *in vivo* and *in vitro* as described herein. Thus, IL-21 of the present invention can be used therapeutically or clinically to actively kill tumor cells in human disease, and to regulate these activities, as well as in additional anti-cancer responses.

4) IL-21 can be used as an immunostimulatory agent in combination with chemotherapy, radiation, and myeloablation. In addition to working alone to boost anti-cancer immunity in patients, IL-21 can work in synergy with standard types of chemotherapy or radiation. For instance, in preclinical models of lymphoma and renal cell carcinoma, the combination of IL-2 with doxorubicin (Ehrke MJ et al., Cancer Immunol. Immunother. 42:221-30, 1996), or the combinations of IL-2 (Younes E et al., Cell Immunol. 165:243-51, 1995) or IFN-α (Nishisaka N et al., Cytokines Cell Mol Ther. 6:199-206, 2000) with radiation provided superior results over the use of single agents. In this setting, IL-21 can further reduce tumor burden and allow more efficient killing by the chemotherapeutic. Additionally, lethal doses of chemotherapy or radiation followed by bone marrow transplantation or stem cell reconstitution could reduce tumor burden to a sufficiently small level (ie. minimal residual disease) to better allow an IL-21 mediated anti-cancer effect. Examples of this type of treatment regimen include the uses of IL-2 and IFN-α to modify anti-cancer responses following myeloablation and transplantation (Porrata LF et al., Bone Marrow Transplant. 28:673-80, 2001; Slavin S, and Nagler A. Cancer J. Sci. Am. Suppl 1:S59-67, 1997; and Fefer A et al., Cancer J. Sci. Am. Suppl 1:S48-53, 1997). In the case of lymphoma and other cancers, depending on when IL-21 is used relative to the chemotherapeutic agents, IL-21 may be employed to directly synergize with the chemotherapeutic agent's effect on the tumor cells or alternatively employed after the chemotherapy to stimulate the immune system. Those skilled in the art would design a protocol to take advantage of both possibilities.

5) IL-21 can be used as a tissue protective agent in combination with standard forms of chemotherapy or methods that ablate bone marrow. IL-21 regulates the proliferation and differentiation of cells. As a result, IL-21 can protect various tissues and organs from the toxicities associated with commonly used chemotherapies and radiation. As an example, gut epithelium expresses IL-15 receptor and experiments in animal models show that IL-15 protects intestinal epithelium from chemotherapy induced toxicity and prevents morbidity (Shinohara H et al., Clin. Cancer Res. 5:2148-56, 1999; Cao S et al., Cancer Res. 58:3270-4, 1998; and Cao S et al., Cancer Res. 58: 1695-9, 1998). In addition to protecting against damage, the proliferative effects of IL-21 can accelerate tissue regeneration following drug-induced toxicity. Relevant examples of this type of activity include the enhanced reconstitution of the immune system stimulated by IL-7 following bone marrow transplantation (Alpdogan O et al., Blood 98:2256-65, 2001; and Mackall CL et al., Blood 97:1491-7, 2001) and the use of G-CSF to treat neutropenia following chemotherapy (Lord, BI et al., Clin. Cancer Res. 7:2085-90, 2001; and Holmes FA et al., J. Clin. Oncol. 20:727-31, 2002). Because IL-21 is shown to enhance proliferation and differentiation of hematopoietic and lymphoid cells, IL-21 of the present invention can be used therapeutically or clinically to aid in recovery as well as enhance the chemotherapeutic dosage schemes upon administration of chemotherapeutic agents in human disease.

6) IL-21 can be used in combination with other immunomodulatory compounds including various cytokines and co-stimulatory/inhibitory molecules. The immunostimulatory activity of IL-21 in mediating an anti-cancer response can be enhanced in patients when IL-21 is used with other classes of immunomodulatory molecules. These could include, but are not limited to, the use of additional cytokines. For instance, the combined use of IL-2 and IL-12 shows beneficial effects in T-cell lymphoma, squamous cell carcinoma, and lung cancer (Zaki MH et al., J. Invest. Dermatol. 118:366-71, 2002; Li D et al., Arch. Otolaryngol. Head Neck Surg. 127:1319-24, 2001; and Hiraki A et al., Lung Cancer 35:329-33, 2002). In addition IL-21 could be combined with reagents that co-stimulate various cell surface molecules found on immune-based effector cells, such as the activation of CD137 (Wilcox RA et al., J. Clin. Invest. 109:651-9, 2002) or inhibition of CTLA4 (Chambers CA et al., Ann. Rev. Immunol. 19:565-94, 2001). Alternatively, IL-21 could be used with reagents that induce tumor cell apoptosis by interacting with TRAIL-related receptors (Takeda K et al., J. Exp. Med. 195:161-9, 2002; and Srivastava RK, Neoplasia 3:535-46, 2001). Such reagents include TRAIL ligand, TRAIL ligand-Ig fusions, anti-TRAIL antibodies, and the like.

7) IL-21 can be used in combination with Monoclonal Antibody Therapy. Treatment of cancer with monoclonal antibodies is becoming a standard practice for many tumors including Non-Hodgkins lymphoma (RITUXAN™), forms of leukemia (MYLOTARG™), breast cell carcinoma (HERCEPTIN™), and colon carcinoma (ERBITUX™). One mechanism by which antibodies mediate an anti-cancer effect is through a process referred to as antibody-dependent cell-mediated cytotoxicity (ADCC) in which immune-based cells including NK cells, macrophages and neutrophils kill those cells that are bound by the antibody complex. Due to its immunomodulatory activity, IL-21 can be used to enhance the effectiveness of antibody therapy. Examples of this type of treatment paradigm include the combination use of RITUXAN™ and either IL-2, IL-12, or IFN-$\alpha$ for the treatment of Hodgkin's and Non-Hodgkin's lymphoma (Keilholz U et al., Leuk. Lymphoma 35:641-2,. 1999; Ansell SM et al., Blood 99:67-74, 2002; Carson WE et al., Eur. J. Immunol. 31:3016-25, 2001; and Sacchi S et al., Haematologica 86:951-8., 2001). Similarly, Because IL-21 is shown to enhance proliferation and differentiation of hematopoietic and lymphoid cells, as well as NK cells, IL-21 of the present invention can be used therapeutically or clinically to enhance the enhance the activity and effectiveness of antibody therapy in human disease.

8) IL-21 can be used in combination with cell adoptive therapy. One method used to treat cancer is to isolate anti-cancer effector cells directly from patients, expand these in culture to very high numbers, and then to reintroduce these cells back into patients. The growth of these effector cells, which include NK cells, LAK cells, and tumor-specific T-cells, requires cytokines such as IL-2 (Dudley ME et al., J. Immunother. 24:363-73, 2001). Given its growth stimulatory properties on lymphocytes, IL-21 could also be used to propagate these cells in culture for subsequent re-introduction into patients in need of such cells. Following the transfer of cells back into patients, methods are employed to maintain their viability by treating patients with cytokines such as IL-2 (Bear HD et al., Cancer Immunol. Immunother. 50:269-74, 2001; and Schultze JL et al., Br. J. Haematol. 113:455-60, 2001). Again, IL-21 can be used following adoptive therapy to increase effector cell function and survival.

9) IL-21 can be used in combination with tumor vaccines. The major objective of cancer vaccination is to elicit an active immune response against antigens expressed by the tumor. Numerous methods for immunizing patients with cancer antigens have been employed, and a variety of techniques are being used to amplify the strength of the immune response following antigen delivery (reviewed in Rosenberg, SA ibid). Methods in which IL-21 can be used in combination with a tumor vaccine include, but are not limited to, the delivery of autologous and allogeneic tumor cells that either express the IL-21 gene or in which IL-21 is delivered in the context of a adjuvant protein. Similarly, IL-21 can be delivered in combination with injection of purified tumor antigen protein, tumor antigen expressed from injected DNA, or tumor antigen peptides that are presented to effector cells using dendritic cell-based therapies. Examples of these types of therapies include the use of cytokines like IL-2 in the context of vaccination with modified tumor cells (Antonia SJ et al., J.Urol. 167:1995-2000, 2002; and Schrayer DP et al., Clin. Exp. Metastasis 19:43-53, 2002), DNA (Niethammer AG et al., Cancer Res. 61:6178-84, 2001), and dendritic cells (Shimizu K et al., Proc. Nat. Acad. Sci U S A 96:2268-73, 1999). Similarly, IL-21 can be used as an anti-cancer vaccine adjuvant.

10) IL-21 can be used in the context of gene therapy. Gene therapy can be broadly defined as the transfer of genetic material into a cell to transiently or permanently alter the cellular phenotype. Numerous methods are being developed for delivery of cytokines, tumor antigens, and additional co-stimulatory molecules via gene therapy to specific locations within tumor patients (reviewed in Rosenberg, SA ibid). These methodologies could be adapted to use IL-21 DNA or RNA, or IL-21 could be used as a protein adjuvant to enhance immunity in combination with a gene therapy approach as described herein.

The tissue distribution of a receptor for a given cytokine offers a strong indication of the potential sites of action of that cytokine. Northern analysis of IL-21 receptor revealed transcripts in human spleen, thymus, lymph node, bone marrow, and peripheral blood leukocytes. Specific cell types were identified as expressing IL-21 receptors, and strong signals were seen in a mixed lymphocyte reaction (MLR) and in the Burkitt's lymphoma Raji. The two monocytic cell lines, THP-1 (Tsuchiya et al., Int. J. Cancer 26:171-176, 1980) and U937 (Sundstrom et al., Int. J. Cancer 17:565-577, 1976), were negative.

IL-21 receptor is expressed at relatively high levels in the MLR, in which peripheral blood mononuclear cells (PBMNC) from two individuals are mixed, resulting in mutual activation. Detection of high levels of transcript in the MLR but not in resting T or B cell populations suggests that IL-21 receptor expression may be induced in one or more cell types during activation. Activation of isolated populations of T and B cells can be artificially achieved by stimulating cells with PMA and ionomycin. When sorted cells were subjected to these activation conditions, levels of IL-21 receptor transcript increased in both cell types, supporting a role for this receptor and IL-21 in immune responses, especially in autocrine and paracrine T and B cell expansions during activation. IL-21 may also play a role in the expansion of more primitive progenitors involved in lymphopoiesis.

IL-21 receptor was found to be present at low levels in resting T and B cells, and was upregulated during activation in both cell types. Interestingly, the B cells also down-regulate the message more quickly than do T cells, suggesting that amplitude of signal and timing of quenching of signal are important for the appropriate regulation of B cell responses.

IL-21 in concert with IL-15 expands NK cells from bone marrow progenitors and augments NK cell effector function. IL-21 also co-stimulates mature B cells stimulated with anti-CD40 antibodies, but inhibits B cell proliferation to signals through IgM. IL-21 enhances T cell proliferation in concert with a signal through the T cell receptor, and overexpression in transgenic mice leads to lymphopenia and an expansion of monocytes and granulocytes, as described herein.

IL-21 polypeptides and proteins can also be used *ex vivo*, such as in autologous marrow culture. Briefly, bone marrow is removed from a patient prior to chemotherapy or organ transplant and treated with IL-21, optionally in combination with one or more other cytokines. The treated marrow is then returned to the patient after chemotherapy to speed the recovery of the marrow or after transplant to suppress graft vs. Host disease. In addition, the proteins of the present invention can also be used for the ex vivo expansion of marrow or peripheral blood progenitor (PBPC) cells. Prior to treatment, marrow can be stimulated with stem cell factor (SCF) to release early progenitor cells into peripheral circulation. These progenitors can be collected and concentrated from peripheral blood and then treated in culture with IL-21, optionally in combination with one or more other cytokines, including but not limited to SCF, IL-2, IL-4, IL-7, IL-15, IL-18, or interferon, to differentiate and proliferate into high-density lymphoid cultures, which can then be returned to the patient following chemotherapy or transplantation.

The present invention provides a method for expansion of hematopoietic cells and hematopoietic cell progenitors comprising culturing bone marrow or peripheral blood cells with a composition comprising an amount of IL-21 sufficient to produce an increase in the number of lymphoid cells in the bone marrow or peripheral blood cells as compared to bone marrow or peripheral blood cells cultured in the absence of IL-21. In other embodiments, the hematopoietic cells and hematopoietic progenitor cells are lymphoid cells. In another embodiment, the lymphoid cells are NK cells or cytotoxic T cells. Furthermore, the composition can also comprise at least one other cytokine selected from the group consisting of IL-2, IL-15, IL-4, GM-CSF, Flt3 ligand and stem cell factor.

C. The Use of IL-21 for Infections

One requirement for achieving sustained immunity and durable clinical responses is the amplification in the numbers and activity of the cells that suppress viral replication, prevent reinfection and kill infected cells. Thus, new factors that mediate effects on lymphocytes including cytotoxic T-cells (CTLs), NK cells, and B-cells, as well myeloid cells such as neutrophils and monocytic cells will improve antiviral activity by the immune system. IL-21 is a product of activated $CD4^+$ "helper" T-cells which are required for both humoral and cell-mediated immunity and for sustaining long-term memory to antigenic re-challenge (U.S. Patent No. 6,307,024; Parrish-Novak J et al., Nature 408:57-63, 2000). The receptor for IL-21 is expressed on cells that mediate antiviral responses and previous experiments have shown that IL-21 can stimulate the proliferation of these cell types *in vitro* (WIPO Publication No.s WO 0/17235 and WO 01/77171). Additional experiments affirm these IL-21 activities *in vivo.*

A central role of the immune system is to protect against microbial infection (reviewed by Paul, WE (Ed), Fundamental Immunology. Lippincott-Raven, New York, NY, 1999). The immune system makes a rapid and highly specific response to a broad spectrum of bacteria, parasites and viruses through an orchestration of cellular interactions and synthesis of soluble factors including cytokines. Two categories of activity describe this protective response: Innate and adaptive immunity. The innate immune response is an acute response and functions to limit pathogen replication. Macrophages, dendritic cells, NK cells and neutrophilic granulocytes constitute some of the cell type responsible for this activity. Innate immunity is accompanied by a more protracted response, the adaptive immune response, whereby an antigen-specific effector system regulated by dendritic cells, T-cells and B-cells mediates resolution of infection and long term memory.

Cytokines play a major role in regulating immune responses to infection. For instance, IFN-(α and -γ are critical for inhibiting virus replication and preventing the replication of cells that harbor virus (reviewed by Vilcek J, and Sen GC, BN, Knipe DM, Howley PM (Eds.), Interferons and other cytokines. Fields Fundamental Virology., 3rd ed., Lippincott-Raven Publishers Philadelphia, PA, 1996, pages 341-365). IFN's also stimulate cells regulating innate immunity and are required for the initiation of the acquired immune response. Additional cytokines produced at early times during viral infection include NK cell-produced IFN-γ, tumor necrosis factor-α, and IL-15. These molecules help shape inflammatory responses to infection as well as stimulate the proliferation and differentiation of lymphocytes critical for adaptive immunity. With respect to viral infection, the CD4+ T-cells that become activated in response to viral antigens initiate a T-helper type I (TH1) response and the subsequent cascade required for cell-mediated immunity. That is, following their expansion by specific growth factors like the cytokine IL-2, these T-cells stimulate antigen-specific CD8+ T-cells, macrophages, and NK cells to kill virally infected host cells. Again, cytokines play an important role in mediating these types of responses. IFN-γ, for example, is critical for regulating the differentiation of a Th1 T-cells and for stimulating cell-mediated immunity (reviewed by Paul, WE supra).

Strategies for treating infectious disease often focus on ways to enhance immunity. For instance, the most common method for treating viral infection involves prophylactic vaccines that induce immune-based memory responses. Many endemic childhood diseases such as measles, chicken pox, and mumps are now quite rare in the U.S. thanks to aggressive vaccination programs (Dowdle WR, and Orenstein WA, Proc Nat. Acad. Sci. USA. 91:2464-8, 1994). Another method for treating viral infection includes passive immunization via immunoglobulin therapy such as infusing antibodies to respiratory sincytial virus (RSV), into high risk patients (Meissner HC, J. Pediatr. 124:S 17-21, 1994). IFN-$\alpha$ is another method for treating viral infections such as genital warts (Reichman RC et al., Ann. Intem. Med. 108:675-9, 1988) and chronic diseases like HCV (Davis GL et al., New Engl. J. Med. 339:1493-9, 1998) and HBV. Although oftentimes efficacious, these methods have limitations in clinical use. For instance, many viral infections are not amenable to vaccine development, nor are they treatable with antibodies alone. In addition, IFN's are not extremely effective and they can cause significant toxicities; thus, there is a need for improved therapies.

Improved methods for treating diseases such as viral infections are dependent on the isolation of reagents that specifically enhance innate and acquired immunity; compounds that stimulate the effector cells of an antiviral response. We have previously reported the discovery of IL-21 (Parrish-Novak J et al., supra; U.S. Patent No. 6,307,024). This cytokine is produced by activated CD4+ T-cells. The IL-21 receptor is expressed by a variety of cells within the immune system such as CD4+ and CD8+ T-cells, B-cells, NK cells, and dendritic cells. Like IL-2, IL-21 acts as an autocrine factor that drives T-cell proliferation, a critical requirement for amplifying an antiviral response. In addition, IL-21 enhances the proliferation and killing activities of both CD8 T-cells and NK cells, thus demonstrating an important role in cell-mediated immunity (Parrish-Novak, J. et al., supra.; U.S. Patent No. 6,307,024; and Kasaian MT et al., Immunity 16: 559-69, 2002). Finally, IL-21 enhances the proliferation of B-cells in the presence of T-cell help, thereby suggesting an important role in stimulating antibody-mediated processes (Parrish-Novak, J. et al.., supra.; U.S. Patent No. 6,307,024). In addition, data described herein have shown not only the immunostimulatory effects of IL-21 on the immune system in regard to CTLs, but also IL-21 is immunostimulatory and represents a novel treatment for viral disease.

The IL-21 polypeptides of the present invention are shown to stimulate CTL and NK cells. IL-21 can hence be used in therapeutic antiviral applications in humans. As such, IL-21 antiviral activity is useful in the treatment and prevention of human viral infections. Examples of the types of viral infections for IL-21 use include, but are not limited to: infections caused by DNA Viruses (e.g., Herpes Viruses such as Herpes Simplex viruses, Epstein-Barr virus, Cytomegalovirus; Pox viruses such as Variola (small pox) virus; Hepadnaviruses (e.g, Hepatitis B virus); Papilloma viruses; Adenoviruses); RNA Viruses (e.g., HIV I, II; HTLV I, II; Poliovirus; Hepatitis A; coronoviruses, such as sudden acute respiratory syndrome (SARS); Orthomyxoviruses (e.g., Influenza viruses); Paramyxoviruses (e.g., Measles virus); Rabies virus; Hepatitis C virus), Flaviviruses, Influenza viruses; caliciviruses; rabies viruses, rinderpest viruses, Arena virus, and the like. Moreover, examples of the types of virus-related diseases for which IL-21 could be used include, but are not limited to: Acquired immunodeficiency; Hepatitis; Gastroenteritis; Hemorrhagic diseases; Enteritis; Carditis; Encephalitis; Paralysis; Brochiolitis; Upper and lower respiratory disease; Respiratory Papillomatosis; Arthritis; Disseminated disease, Meningitis, Mononucleosis. In addition, IL-21 can be used in various applications for antiviral immunotherapy, and in conjunction with other cytokines, other protein or small molecule antivirals, and the like.

Moreover, IL-21 will be useful treatment of other microbial infections. These types of microbes include bacteria and fungus. Specific bacterial infections that can be treated with IL-21, include but are not limited to, *chlamydiae, listeriae, helicobacter pylori, mycobacterium, mycoplasma, bacillus anthracis, salmonella, and shigella.* An example of a fungal infection include, but are not limited to, *candidiasis.*

For example:

(1) IL-21 can be used as a monotherapy for acute and chronic viral infections and for immunocompromised patients. Methods that enhance immunity can accelerate the recovery time in patients with unresolved infections. A partial list of the types of infections is provided (see above). Immunotherapies can have an even greater impact on subsets of immunocompromised patients such as the very young or elderly as well as patients that suffer immunodeficiencies acquired through infection, or induced following medical interventions such as chemotherapy or bone marrow ablation. Examples of the types of indications being treated via immune-modulation include; the use of IFN-$\alpha$ for chronic hepatitis (Perry CM, and Jarvis B, Drugs 61:2263-88, 2001), the use of IL-2 following HIV infection (Mitsuyasu R., J Infect Dis. 185 Suppl 2:5115-22, 2002; and Ross RW et al., Expert Opin Biol Ther. 1:413-24, 2001), and the use of either interferon (Faro A, Springer Semin Immunopathol.20:425-36, 1998) for treating Epstein Barr Virus infections following transplantation. Experiments performed in animal models indicate that IL-2 and GM-CSF may also be efficacious for treating EBV related diseases (Baiocchi RA et al., J Clin. Invest. 108:887-94, 2001).

(2) IL-21 can be used in combination with antiviral agents. Some of the more common treatments for viral infection include drugs that inhibit viral replication such as ACYCLOVIR™ In addition, the combined use of some of these agents form the basis for highly active antiretroviral therapy (HAART) used for the treatment of HIV. Examples in which the combination of immunotherapy (ie cytokines) and antiviral drugs shows improved efficacy include the use of interferon plus RIBAVIRIN™ for the treatment of chronic HCV infection (Maddrey WC, Semin. Liver. Dis. 19 Suppl 1:67-75, 1999) and the combined use of IL-2 and HAART (Ross, RW et al, ibid..) Thus, as IL-21 can stimulate the immune system against disease, it can similarly be used in combination with HAARTand other antiviral drugs. In particular, IL-21 may be useful in monotherapy or combination therapy with IFN-α (with or without RIBAVIRIN™) in patients who do not respond well to IFN therapy. These patients may not respond to IFN therapy due to having less type I interferon receptor on the surface of their cells (Yatsuhashi et al. J Hepatol Jun.30(6):995-1003, 1999; Mathai et al., J. Interferon Cytokine Res Sep.19(9):1011-8, 1999; Fukuda et al. J Med Virol Mar. 63(3):220-7, 2001). IL-21 may also be useful in monotherapy or combination therapy with IFN-α, (with or without RIBAVIRIN™) in patients who have less type I interferon receptor on the surface of their cells due to down-regulation of the type I interferon receptor after type I interferon treatment (Dupont SA, et al. J. Interferon Cytokine Res. Apr;22(4):491-501, 2002).

(3) IL-21 can be used in combination with other immunotherapies including cytokines, immunoglobulin transfer, and various co-stimulatory molecules. In addition to antiviral drugs, IL-21 could be used in combination with any other immunotherapy that is intended to stimulate the immune system. Thus, IL-21 could be used with other cytokines such as Interferon or IL-2. IL-21 could also be added to methods of passive immunization that involve immunoglobulin transfer, one example bring the use of antibodies to treat RSV infection in high risk patients (Meissner HC, ibid.). In addition, IL-21 could be used with additional co-stimulatory molecules such as 4-1BB ligand that recognize various cell surface molecules like CD137 (Tan, JT et al., J Immunol. 163:4859-68, 1999).

(4) IL-21 can be used as an adjuvant for antiviral vaccines. The use of prophylactic vaccines for the prevention of viral disease is well known. IL-21 could be used as an adjuvant with these vaccines for indications where the efficacy of the vaccine is reduced, one example being the hepatitis B vaccine (Hasan MS et al., J Infect Dis. 180:2023-6, 1999; and Evans TG et aL, Clin Nephrol. 54:138-42, 2000). In addition to prophylactic vaccines, therapeutic vaccines are being developed that are intended to arrest an ongoing infection. The methodologies for these vaccines are quite varied and include, but are not limited to, viral antigens delivered via DNA, viral peptides, viral proteins, portions of viral particles, and viral antigens loaded into cell-based therapies such as dendritic cells. Similar to the combined treatment of a therapeutic cancer with a cytokine like IL-2 (Shimizu K et al., Proc. Nat. Acad. Sci. U S A. 96:2268-73, 1999), IL-21 could be used in combination with a therapeutic antiviral vaccine.

The tissue distribution of a receptor for a given cytokine offers a strong indication of the potential sites of action of that cytokine. Northern analysis of IL-21 receptor revealed transcripts in human spleen, thymus, lymph node, bone marrow, and peripheral blood leukocytes. Specific cell types were identified as expressing IL-21 receptors, and strong signals were seen in a mixed lymphocyte reaction (MLR) and in the Burkitt's lymphoma Raji. The two monocytic cell lines, THP-1 (Tsuchiya et al., Int. J. Cancer 26:171-176, 1980) and U937 (Sundstrom et al., Int. J. Cancer 17:565-577, 1976), were negative.

As discussed herein, IL-21 may activate the immune system which is important in boosting immunity to infectious diseases, treating immunocompromised patients, such as HIV+ patients, or in improving vaccines. In particular, IL-21 stimulation or expansion of NK cells, or their progenitors, would provide therapeutic value in treatment of viral infection, and as an anti-neoplastic (anticancer) factor. NK cells are thought to play a major role in elimination of metastatic tumor cells and patients with both metastases and solid tumors have decreased levels of NK cell activity (Whiteside et. al., Curr. Top. Microbiol. Immunol. 230:221-244, 1998). Similarly, IL-21 stimulation of the immune response against viral and non-viral pathogenic agents (including bacteria, protozoa, and fungi) would provide therapeutic value in treatment of such infections by inhibiting the growth of such infections agents. Determining directly or indirectly the levels of a pathogen or antigen, such as a tumor cell, present in the body can be achieved by a number of methods known in the art and described herein. In general, a therapeutically effective amount of IL-21 includes an amount sufficient to produce a clinically significant change in virally infected mammals; such changes can include, but are not limited to, an amount of IL-21 sufficient to produce a clinically significant change in the level of CTLs or NK cells; an amount of IL-21 sufficient to produce a clinically significant change in viral load, and in anti-viral antibody titer. Determination of such clinically significant differences or changes is well within the skill of one in the art. The measurement of antiviral CTLs, NK cells, viral load and antiviral antibody titers have been studied after cytokine administration to both acute anc chronic models of lymphocytic choriomeningitis virus (LCMV) infection in the mouse. (Blattman et al. supra) CTL measurements including enumeration, cytolyic activity, cytokine production and proliferation have been measured along with viral load in chronically infected HCV patients (Wedemleyer et al. J. Immunol. 169:3447-3458, 2002).

Clinically, diagnostic tests for HCV include serologic assays for antibodies and molecular tests for viral particles. Enzyme immunoassays are available (Vrielink et al., Transfusion 37:845-849, 1997), but may require confirmation using additional tests such as an immunoblot assay (Pawlotsky et al., Hepatology 27:1700-1702, 1998). Qualitative and quantitative assays generally use polymerase chain reaction techniques, and are preferred for assessing viremia and treatment response (Poynard et al., Lancet 352:1426-1432, 1998; McHutchinson et al., N. Engl. J. Med. 339:1485-1492, 1998). Several commercial tests are available, such as, quantitative RT-PCR (Amplicor HCV Monitor™, Roche Molecular Systems, Branchburg, NJ) and a branched DNA (deoxyribonucleic acid) signal amplification assay (Quantiplex™ HCV RNA Assay [bDNA], Chiron Corp., Emeryville, CA). A non-specific laboratory test for HCV infection measures alanine aminotransferase level (ALT) and is inexpensive and readily available (National Institutes of Health Consensus Development Conference Panel, Hepatology 26 (Suppl. 1):2S-10S. 1997). Histologic evaluation of liver biopsy is generally considered the most accurate means for determining HCV progression (Yano et at., Hepatology 23:1334-1340, 1996.) For a review of clinical tests for HCV, see, Lauer et al., N. Engl. J. Med. 345:41-52, 2001.

There are several in vivo models for testing HBV and HCV that are known to those skilled in art. For example, the effects of IL-21 on mammals infected with HBV can accessed using a woodchuck model. Briefly, woodchucks chronically infected with woodchuck hepatitis virus (WHV) develop hepatitis and hepatocellular carcinoma that is similar to disease in humans chronically infected with HBV. The model has been used for the preclinical assessment of antiviral activity. A chronically infected WHV strain has been established and neonates are inoculated with serum to provide animals for studying the effects of certain compounds using this model. (For a review, see, Tannant et al., ILAR J. 42 (2):89-102,200). Chimpanzees may also be used to evaluate the effect of IL-21 on HBV infected mammals. Using chimpanzees, characterization of HBV was made and these studies demonstrated that the chimpanzee disease was remarkably similar to the disease in humans (Barker et al., J. Infect. Dis. 132:451-458, 1975 and Tabor et al., J. Infect. Dis. 147:531-534, 1983.) The chimpanzee model has been used in evaluating vaccines (Prince et al., In: Vaccines 97, Cold Spring Harbor Laboratory Press, 1997.) Therapies for HIV are routinely tested using non-human primates infected with simian immunodeficiency viruses (for a review, see, Hirsch et al., Adv. Pharmcol. 49:437-477, 2000 and Nathanson et al., AIDS 13 (suppl.A):SI 13-S120, 1999.) For a review of use of non-human primates in HIV, hepatitis, malaria, respiratory syncytial virus, and other diseases, see, Sibal et al., ILAR J. 42 (2):74-84, 2001.

For pharmaceutical use, the proteins of the present invention are formulated for parenteral, particularly intravenous or subcutaneous, delivery according to conventional methods. The bioactive polypeptide or antibody conjugates described herein can be delivered intravenously, intraarterially or intraductally, or can be introduced locally at the intended site of action. Intravenous administration will be by bolus injection or infusion over a typical period of one to several hours. In general, pharmaceutical formulations will include a IL-21 protein in combination with a pharmaceutically acceptable vehicle, such as saline, buffered saline, 5% dextrose in water or the like. Formulations may further include one or more excipients, preservatives, solubilizers, buffering agents, albumin to prevent protein loss on vial surfaces, etc. Methods of formulation are well known in the art and are disclosed, for example, in Remington: The Science and Practice of Pharmacy, Gennaro, ed., Mack Publishing Co., Easton, PA, 19th ed., 1995. Therapeutic doses will generally be in the range of 0.1 to 100 μg/kg of patient weight per day, preferably 0.5-20 μg/kg per day, with the exact dose determined by the clinician according to accepted standards, taking into account the nature and severity of the condition to be treated, patient traits, etc. Determination of dose is within the level of ordinary skill in the art. The proteins can be administered for acute treatment, over one week or less, often over a period of one to three days or can be used in chronic treatment, over several months or years.

The present invention also contemplates chemically modified IL-21 compositions, in which a IL-21 polypeptide is linked with a polymer. Illustrative IL-21 polypeptides are soluble polypeptides that lack a functional transmembrane domain, such as a mature IL-21 polypeptide. Typically, the polymer is water soluble so that the IL-21 conjugate does not precipitate in an aqueous environment, such as a physiological environment. An example of a suitable polymer is one that has been modified to have a single reactive group, such as an active ester for acylation, or an aldehyde for alkylation, In this way, the degree of polymerization can be controlled. An example of a reactive aldehyde is polyethylene glycol propionaldehyde, or mono-(C1-C10) alkoxy, or aryloxy derivatives thereof (see, for example, Harris, et al,, U.S. Patent No. 5,252,714). The polymer may be branched or unbranched. Moreover, a mixture of polymers can be used to produce IL-21 conjugates.

IL-21 conjugates used for therapy can comprise pharmaceutically acceptable water-soluble polymer moieties. Suitable water-soluble polymers include polyethylene glycol (PEG), monomethoxy-PEG, mono-(C1-C10)alkoxy-PEG, aryloxy-PEG, poly-(N-vinyl pyrrolidone)PEG, tresyl monomethoxy PEG, PEG propionaldehyde, *bis*-succinimidyl carbonate PEG, propylene glycol homopolymers, a polypropylene oxide/ethylene oxide co-polymer, polyoxyethylated polyols (*e.g*., glycerol), polyvinyl alcohol, dextran, cellulose, or other carbohydrate-based polymers. Suitable PEG may have a molecular weight from about 600 to about 60,000, including, for example, 5,000, 12,000, 20,000 and 25,000. A IL-21 conjugate can also comprise a mixture of such water-soluble polymers.

PEGylation of IL-21 can be carried out by any of the PEGylation reactions known in the art (see, for example, EP 0 154 316, Delgado et al., Critical Reviews in Therapeutic Drug Carrier Systems 9:249 (1992), Duncan and Spreafico, Clin. Pharmacokinet. 27:290 (1994), and Francis et al., Int J Hematol 68:1 (1998)).

The present invention contemplates methods for treating cancer using compositions comprising an IL-21 polypeptide or polypeptide as described herein. Such compositions can further comprise a carrier. The carrier can be a conventional organic or inorganic carrier. Examples of carriers include water, buffer solution, alcohol, propylene glycol, macrogol, sesame oil, corn oil, and the like.

The invention is further illustrated by the following non-limiting examples.

## EXAMPLES

### Example 1

### Mouse IL-21 is active in mouse bone marrow assay

#### A. Isolation of Non-adherent Low Density Marrow Cells:

Fresh mouse femur aspirate (marrow) was obtained from 6-10 week old male Balb/C or C57BL/6 mice. The marrow was then washed with RPMI+10% FBS (JRH, Lenexa KS; Hyclone, Logan UT) and suspended in RPMI+10% FBS as a whole marrow cell suspension. The whole marrow cell suspension was then subjected to a density gradient (Nycoprep, 1.077, Animal; Gibco BRL) to enrich for low density, mostly mononuclear, cells as follows: The whole marrow cell suspension (About 8 ml) was carefully pipetted on top of about 5 ml Nycoprep gradient solution in a 15 ml conical tube, and then centrifuged at 600X g for 20 minutes. The interface layer, containing the low density mononuclear cells, was then removed, washed with excess RPMI+10% FBS, and pelleted by centrifugation at 400X g for 5-10 minutes. This pellet was resuspended in RPMI +10% FBS and plated in a T-75 flask at approximately $10^6$ cells/ml, and incubated at 37˚C 5% $CO_2$ for approximately 2 hours. The resulting cells in suspension were Non-Adherent Low Density (NA LD) Marrow Cells.

#### B. 96-Well Assay

NA LD Mouse Marrow Cells were plated at 25,000 to 45,000 cells/well in 96 well tissue culture plates in RPMI +10% FBS + 1ng/mL mouse Stem Cell Factor (mSCF) (R&D Systems, Minneapolis, MN), plus 5% conditioned medium from one of the following: (1) BHK 570 cells expressing mouse IL-21 (U.S. Patent No. 6,307,024), (2) BHK 570 cells expressing human IL-21 (U.S. Patent No. 6,307,024), or (3) control BHK 570 cells containing vector and not expressing either Ligand. These cells were then subjected to a variety of cytokine treatments to test for expansion or differentiation of hematopoietic cells from the marrow. To test, the plated NA LD mouse marrow cells were subjected to human Interleukin-15 (hIL-15) (R&D Systems), or one of a panel of other cytokines (R&D Systems). Serial dilution of hIl-15, or the other cytokines, were tested, with 2-fold serial dilution from about 50 ng/ml down to about 6025 ng/ml concentration. After 8 to 12 days the 96-well assays were scored for cell proliferation by Alamar blue assay as described in U.S. Patent No. 6,307,024.

#### C. Results from the 96-well NA LD Mouse Marrow assay

Conditioned media from the BHK cells expressing both mouse and human IL-21 acted in synergy with hIL-15 to promote the expansion of a population of hematopoietic cells in the NA LD mouse marrow. This expansion of hematopoietic cells was not shown with control BHK conditioned medium plus IL-15. The population hematopoietic cells expanded by the mouse IL-21 with hIL-15, and those hematopoietic cells expanded by the human IL-21 with hIL-15, were further propagated in cell culture. These hematopoietic cells were stained with a Phycoerythrin labeled anti-Pan NK cell antibody (Pharmingen) and subjected to flow cytometry analysis, which demonstrated that the expanded cells stained positively for this natural killer (NK) cell marker.

The same 96-well assay was run, using fresh human marrow cells bought from Poietic Technologies, Gaithersburg, MD. Again, in conjunction with IL-15, the mouse and human IL-21 expanded a hematopoietic cell population that stained positively for the NK cell marker using the antibody disclosed above.

### Example 2

### IL-21 Transgenic Mice

#### A. Generation of transgenic mice expressing human and mouse IL-21

DNA fragments from transgenic vectors (U.S. Patent No. 6,307,024) containing 5' and 3' flanking sequences of the respective promoter (MT-1 liver-specific promoter (mouse IL-21 (U.S. Patent No. 6,307,024) or lymphoid specific LCK promoter (mouse and human IL-21 (U.S. Patent No. 6,307,024), the rat insulin II intron, IL-21 cDNA and the human growth hormone poly A sequence were prepared and used for microinjection into fertilized B6C3fl (Taconic, Germantown, NY) murine oocytes, using a standard microinjection protocol. See, Hogan, B. et al., Manipulating the Mouse Embryo. A Laboratory Manual. Cold Spring Harbor Laboratory Press, 1994.

Eight transgenic mice expressing human IL-21 from the lymphoid-specific EμLCK promoter were identified

among 44 pups. Four of these were pups that died and 4 grew to adulthood. Expression levels were fairly low in these animals. Twenty transgenic mice expressing mouse IL-21 from the lymphoid-specific EμLCK promoter were identified among 77 pups. All 20 grew to adulthood. Expression levels were fairly low in these animals. Three transgenic mice expressing mouse IL-21 from the liver-specific MT-1 promoter were identified among 60 pups. Two of these pups died and 1 grew to adulthood. Expression levels were fairly low in these animals. Tissues were prepared and histologically examined as describe below.

B. Microscopic evaluation of tissues from transgenic mice

Spleen, thymus, and mesenteric lymph nodes were collected and prepared for histologic examination from transgenic animals expressing human and mouse IL-21 (Example 2A). Other tissues which were routinely harvested included the following: Liver, heart, lung, kidney, skin, mammary gland, pancreas, stomach, small and large intestine, brain, salivary gland, trachea, espohogus, adrenal, pituitary, reproductive tract, accessory male sex glands, skeletal muscle including peripheral nerve, and femur with bone marrow. The tissues were harvested from a neonatal pup which died unexpectedly, and several adult transgenic mice, as described below. Samples were fixed in 10% buffered formalin, routinely processed, embedded in paraffin, sectioned at 5 microns, and stained with hematoxylin and eosin. The slides were examined and scored as to severity of tissue changes (0=none, 1=mild, 2--moderate, 3=severe) by a board certified veterinary pathologist blinded to treatment.

The pup and 2 female adult mice expressing the human IL-21 , and 3 of the 6 male adult mice expressing the mouse IL-21 showed inflammatory infiltrates in many of the tissues examined. The organs affected varied somewhat from mouse to mouse. The inflammatory infiltrate was composed primarily of neutrophils and macrophages in varying numbers and proportions and was generally mild to moderate degree in severity. Moreover, these animals showed changes in lymphoid organs, including moderate to severe lymphopenia in the spleen and thymus (human and mouse IL-21 transgenics); and severe lymphopenia (human IL-21 transgenics), or mild to severe suppurative to pyogranulomatous lymphadenitis (mouse IL-21 transgenics) in lymph nodes. In addition, increased extramedullary hematopoiesis was evident in the spleens. These changes were not observed in age-matched control mice.

C. Flow cytometric analysis of tissues from transgenic mice over expressing

Transgenic animals over expressing either human or mouse zalpha11ligand (Example 2A) were sacrificed for flow cytometric analysis of peripheral blood, thymus, lymph node, bone marrow, and spleen.

Cell suspensions were made from spleen, thymus and lymph nodes by teasing the organ apart with forceps in ice cold culture media (500 ml RPMI 1640 Medium (JRH Biosciences. Lenexa, KS); 5 ml 100x L-glutamine (Gibco BRL. Grand Island, NY); 5 ml 100x Na Pyruvate (Gibco BRL); 5 ml 100X Penicillin, Streptomycin, Neomycin (PSN) (Gibco BRL) and then gently pressing the cells through a cell strainer (Falcon, VWR Seattle, WA). Peripheral blood (200 ml) was collected in heparinized tubes and diluted to 10 ml with HBSS containing 10U Heparin/mL Erythrocytes were removed from spleen and peripheral blood preparations by hypotonic lysis. Bone marrow cell suspensions were made by flushing marrow from femurs with ice cold culture media. Cells were counted and tested for viability using Trypan Blue (GIBCO BRL, Gaithersburg, MD). Cells were resuspended in ice cold staining media (HBSS, 1% fetal bovine serum, 0.1% sodium azide) at a concentration of ten million per milliliter. Blocking of Fc receptor and non-specific binding of antibodies to the cells was achieved by adding 10% normal goat sera and Fc Block (Pharmingen, La Jolla, CA) to the cell suspension.

Cell suspensions were mixed with equal volumes of fluorochrome labeled monoclonal antibodies (PharMingen), incubated on ice for 60 minutes and then washed twice with ice cold wash buffer (PBS, 1% fetal bovine serum, 0.1% sodium azide) prior to resuspending in 400 ml wash buffer containing 1mg/ml 7-AAD (Molecular Probes, Eugene, OR) as a viability marker in some samples. Flow data was acquired on a FACSCalibur flow cytometer (BD Imnunocytometry Systems, San Jose, CA). Both acquisition and analysis were performed using CellQuest software (BD Immunocytometry Systems).

The transgenic animals that expressed either the human or mouse IL-21 at the highest levels had dramatically altered cell populations in all lymphoid organs analyzed. Changes seen included complete loss of thymic cellularity, complete absence of CD45R positive B cells and increased size and cellularity of spleens. Both spleen and bone marrow had increased numbers of myeloid sized cells, which was accounted for by increases in both monocytes and neutrophils. The pan NK cell marker (DX5) was increased in many populations. Moderate expressing founders had less dramatic but still significant changes consistent with the phenotype seen in the high expressers. Mice with the lowest level of expression had neither a significant increase in myeloid cells nor decrease in B cells numbers. They did show significant changes in thymocyte populations with decreases in CD4+CD8+ double positive cells and increases in both CD4 and CD8 single positive cells.

Example 3

IL-21 Purified Recombinant Human Protein

Dose-Response Study in Normal Mice

A. Summary

Normal six week old female C57Bl/6 (Harlan Sprague Dawley, Indianapolis, IN). mice were treated by intraperitoneal injection once daily for either four or eight days with one of four dose levels of purified recombinant human IL-21 (U.S. Patent No. 6,307,024) at 0.1, 0.5, 5 or 50μg/mouse/day or with vehicle as a control. Body weights and body temperatures were monitored daily. On either day four or day nine, four of the eight mice from each protein treatment group and five of the ten mice in the vehicle control group were sacrificed. Blood, bone marrow and tissues were harvested and analyzed. Potential perturbations in lymphoid tissues were examined, as well as general physiologic and toxicological parameters.

There was no evidence of toxicity of human IL-21 protein at any of the doses tested. Body weights and temperatures were unchanged. There were no apparent changes in clinical chemistry parameters. However, there were consistent findings relating to increased percentages of myeloid lineage cells in bone marrow, spleen and peripheral blood in mice treated with the highest dose of IL-21 compared to the vehicle control. There was a statistically significant increase in myeloid lineage sized cells identified by flow cytometric analysis of spleen homogenate in the high-dose group. The spleens of the two highest dose groups were statistically significantly larger than the other groups. On histopathologic examination, however, only a marginal increase in extramedullary hematopoiesis was seen in the highest dose group. There was a statistically significant increase in the myeloid to erythroid ratio of the bone marrow in the highest dose group compared to the other groups. Finally, there were increases seen in peripheral blood both in total white blood cell counts and in the percentage of monocytes in the same group.

B. Dosing solution preparation

Purified recombinant human IL-21 (U.S. Patent No. 6,307,024) was diluted into sterile phosphate buffered saline (GibcoBRL, Grand Island, NY) at concentrations to deliver 50, 5, 0.5 or 0.1 micrograms of protein in 0.1 ml of PBS vehicle. The doses for the first four days were made on day 0 and frozen in a frosty - 20˚C freezer prior to use. The doses for days five through eight were made on day five and frozen as above. Aliquots of the same PBS were similarly frozen for the vehicle treated control group. On the day of administration the appropriate aliquots were thawed and 0.1 ml of solution was injected intraperitoneally into the mice each day for either four or eight days.

C. Study design

The mice were six weeks old at the start of the study. Each treatment group consisted of eight mice, except for the vehicle control group that included ten mice. One half of the mice in each treatment group were sacrificed after four days of treatment and the other half after eight days.

Before treatment each day, each mouse was weighed and her body temperature recorded using the Portable Programmable Notebook System (BMDS, Inc, Maywood, NJ), by scanning the mouse for identification number and body temperature from transponders implanted subcutaneously (IPTT-100, BMDS, Maywood, NJ).

At sacrifice, tissues harvested to assess white blood cell populations by flow cytometric analysis included bone marrow, thymus and spleen. FACS analysis of the lymphoid organs and bone marrow was performed with the FACSCalibur, (Becton Dickinson, Mansfield, MA). The tissues harvested for histologic examination for signs of toxicity of the protein included: spleen, thymus, liver, kidney, adrenal gland, heart and lungs. All tissues fixed for histology were kept at 4˚C overnight in 10% Normal Buffered Saline (NBF) (Surgipath, Richmond, IL). The following day the NBF was replaced with 70% ethanol and the tissues returned to 4˚C until processing for histology.

The tissues were processed and stained for Hematoxylin and Eosin in house, then sent to a contract pathologist for histopathologic analysis. Blood was collected for complete blood cell counts (CBC) and serum chemistry profiles. The CBC's were analyzed in-house with the Cell Dyn 3500 Hematology Analyzer (Abbott Diagnostics Division, Abbott Park, IL) and manual differential white blood cell counts were analyzed at Phoenix Central Laboratory, (Everett, WA). The serum was kept frozen at -20˚C until submission to Phoenix Central Laboratory for complete serum chemistry panels. To assess myeloid:erythroid ratios, the bone marrow from one femur was applied to CytoSpin slides (CYTOSPIN 3 CYTOCENTRIFUGE and CYTO SLIDES, Shandon, Pittsburgh,PA) and sent to Phoenix Central Laboratories for analysis.

D. Study results

There were no apparent clinical indications of physiologic effects or of toxicity of human IL-21 at doses of 50ng/day or lower. Body weights and temperatures remained normal for the duration of the treatments. Serum chemistry parameters were in normal ranges. Red blood cell and platelet counts appeared normal. In the mice receiving 50pg/day for 8 days, manual differential white blood cell counts showed that the percentage of monocytes was elevated in the peripheral blood, and an apparent increase in the total white blood cell counts. In bone marrow flushed from a femur, myeloid to erythroid ratios were increased in the 50pg dose group, and to a lesser degree the 5ug dose group from the 8-day dose set. In a non-parametric multiple column comparison using InStat (InStat MAC; GraphPad Software, Inc., San Diego, CA), this difference was statistically significant (p=.0049). The difference between the highest dose group and vehicle was also significant, (p=.0286). The increased white blood cells in peripheral blood and the significant increase in myeloid precursors in the marrow may thus be related.

Histologic evaluation of the following tissues showed no apparent evidence of cytalogic or structural changes, mitotic events or necrosis: thymus, liver, kidney, adrenal gland, duodenum, pancreas, jejunum, caecum, colon, mesenteric lymph nodes, uterus, ovary, salivary gland, heart, trachea, lung, and brain. There were no apparent differences between the treatment groups in the weights of the thymus, kidney, liver or brain. Of all the tissues examined, only the spleen weights were , significantly affected.

Each mouse spleen weight was normalized to her brain weight. In the 50μg/day treatment group compared to the vehicle, 0.1 μg and 0.5 μg treatment groups, the average of the spleen weights was nearly 50% greater after four days of treatment and almost 100% greater after eight days than the average spleen weights of the other three groups. In the four-day set, the 5μglday group also tended to have larger spleens than the control and low dose groups. The difference in the spleen/brain weights with data from the four-day and the eight-day sets combined by treatment group was statistically significant (p = .0072) by Kruskall-Wallace non-parametric ANOVA; multiple column comparison test using the InStat program (GraphPad Software).

A marginal increase in extrameduallary hematopoiesis, especially in the red pulp was seen in spleens of mice from the highest dose group, even in the mice treated for four days. Flow cytometric analysis of the spleens showed a significant increase in the proportion of myeloid size cells in the highest dose group (p=0.01, Student's t test), representing increases in both monocytes and neutrophils. This effect may be related to the increased peripheral blood mononuclear cell percentage, as well as the apparent increase in myeloid precursors in the bone marrow, described above. Moreover, the transgenic mice derived from insertion of the human zalphall gene had increased extramedullary hematopoiesis in their spleens compared to non-transgenic litter mates.

Several changes were observed in the 50μg per day dose group compared to the control group that implicate IL-21 in production or development of cells of the myeloid lineage. Taken together, the observed changes suggest that zalphal may be useful as a therapeutic protein in such medical specialties as cancer and immunologic disorders described herein.

Example 4

Preliminary Elimination and Tissue Distribution Study Of

Purified Recombinant Human IL-21 Protein

A. Summary

In order to elucidate tissue distribution and elimination patterns of the purified rhIL-21 , a preliminary pharmacokinetic study was undertaken. Nine week old male C57Bl/6 mice were given purified recombinant human IL-21 protein labeled with $^{111}$Indium ($^{111}$In) (NEN, Boston, MA) by one of three routes. A single bolus injection was given to each mouse by either the intravenous (IV), intraperitoneal (IP), or subcutaneous route (SC). The mice injected by either the subcutaneous or intraperitoneal route were sacrificed at either one or three hours after injection. The mice injected intravenously were sacrificed after either ten minutes or one hour following injection. Blood, plasma and selected tissues were harvested at various timepoints and counted by a gamma counter to estimate the approximate half-life and tissue distribution of the exogenous labeled protein. The tissues that were harvested for counting as well as the intervals of sacrifice were selected based on reports of the distribution of other cytokines labeled with radionuclides.

At sacrifice, tissues harvested for counting of radioactivity included thymus, spleen, kidney, a lobe of liver, a lobe of lung, and urinary bladder. In the group receiving the injection intraperitoneally, gut was also counted to assess incidence of injection into the gut, and in the subcutaneously dosed mice, skin with underlying structures in the area of injection was counted. The cpm for whole liver and lung were calculated from a section that was counted and a percentage of the whole organ weight represented by the section.

After the end of the study the collected tissues, whole blood and plasma were counted on the COBRA II AUTO-GAMMA® gamma counter (Packard Instrument Company, Meriden, CT). An aliquot of the original labeled dosing solution was also counted at the end of the study with the tissues. This allowed calculation of percent total injected radioactivity for each mouse and simultaneous correction of all counts for radioactive decay. Approximations of remaining blood volume and organ weights indicated that the majority of the counts administered were accounted for, and therefore the percentage of counts per tissue were a reasonable representation of distribution of the counts following labeled IL-21 administration by each route.

B. $^{111}$Indium labeling of IL-21

Purified recombinant human IL-21 (U.S. Patent No. 6,307,024) was conjugated with a 10 fold molar excess of DTPA (Peirce, Rockford, I1) by incubating 30 minutes at room temperature in PBS. Unreacted DTPA and hydrolyzates were removed by buffer exchange on a Biomax-5k NMWL (Ultrafree-15, Millipore, Bedford, MA). The void volume protein peak was concentrated to 5 mg/ml and an aliquot taken for testing in a bioassay (anti-CD40 stimulation of murine B-cells (Example 10)). Upon confirming that the DTPA-conjugate still had full bioactivity the conjugate was diluted to 0.5 mg/ml with 1M Na Acetate pH 6.0. Two mCi of $^{111}$Indium was taken up in 0.5 ml 1M Na Acetate pH 6.0 and mixed with the DTPA-human IL-21 for 30 min. at room temperature. Unincorporated $^{111}$Indium was removed during buffer exchange to PBS on a PD-10 column (Pharmacia, Piscataway, NJ). The radio-labeled material was diluted with unlabeled human IL-21 to give a specific activity of 100 mCi/mg, sterile filtered and stored at 4˚C overnight. One hundred percent of the labeled protein was retained on a Biomax-5k NMWL membrane (Millipore). The labeled $^{111}$In-human IL-21 was administered to mice in the elimination and pharmacokinetic studies. Fifty μg human IL-21 protein labeled with 5 μCi of labeled human IL-21 in 0.1 ml of PBS vehicle was administered to each animal.

C. Results Of Preliminary Distribution Study

After one and three hours following administration by all three routes, the highest concentration of $^{111}$In-human IL-21, was found in kidney and the second highest was in urine and urinary bladder, as evinced by these tissues having the highest cpm. The average counts recovered from kidneys were from 3 to 8 times higher than the whole liver counts, depending on the route of injection and the sacrifice timepoint. For example, the average kidney cpm at 60 minutes following IV injection was 4.5 times greater than the average counts calculated for whole liver from the same group. In the group that was sacrificed ten minutes after intravenous administration, the highest cpm was again in kidney, and the second highest accumulation was equivalent in liver, urinary bladder and urine.

D. Preliminary Pharmacokinetic Study

Blood and plasma collections were done at 10, 30 and 60 minutes following injection by all three routes. Following injection by the IV route, a separate set of mice had blood and plasma samples taken at two, five and ten minutes. Another set of mice who received their injections by either the IP or SC route had blood sampled at one, two and three hours. For the treatment groups see Table 4. The short collection times bracket the reported half-life of IL-2 following intravenous injection. The reported T½ was in the range of 2.5 to 5.1 minutes. For reference to *in vivo* administration to IL-2, see Donohue JH and Rosenberg SA J Immunol. 130:2203, 1983. The long timepoints were chosen to outline the anticipated elimination phase.

Table 4

| Route of injection | Bleed Times (min.) | Sacrifice Time |
|---|---|---|
| Intravenous Group 1 | 2,5,10 | 10 min. |
| Intravenous Group 2 | 10, 30, 60 | 60 min. |
| Intraperitoneal Group 1 | 10,30,60 | 60 min. |
| Intraperitoneal Group 2 | 60,120,180 | 180 min. |
| Subcutaneous Group 1 | 10,30,60 | 60 min. |
| Subcutaneous Group 2 | 60,120,180 | 180 min. |

Un-labeled IL-2 has been shown to be eliminated from the serum with a half-life of approximately three minutes in mice after IV injection. For reference see Donahue, JH and Rosenburg supra.. Following IP and SC injection of similar amounts of IL-2, the duration of persistence of IL-2 activity in serum was prolonged from 2 units/ml for less than 30 minutes following IV injection to greater than 2 units/ml for 2 hours following IP and 6 hours following SC injections. The principle route of clearance of IL-2 appears to be the kidney. IL-21 has been shown to be structurally similar to IL-2, as discussed herein. Preliminary evaluation of the elimination of IL-21 appears to be consistent with the apparent clearance of IL-2 by the kidneys, based on the accumulation of cpm predominantly in the kidneys, followed by the urinary bladder and urine in the present study.

Estimations were made of pharmacokinetic parameters based on non compartmental analysis of the cpm data obtained from the plasma, using the PK analysis program WinNonLin, Version 1.1, (Scientific Consulting Inc., Cary, NC). Plasma half-lives of IL-21 were estimated using the predicted terminal elimination rate constants for intravenous, subcutaneous, and intraperitoneal administration of a 50 lAg dose. The pharmacokinetic results were estimations due to limited data points in the terminal elimination region of the plasma concentration vs. time profiles. Moreover, the fit of the terminal elimination phase for SC and IP dosing required use of data from timepoints during which absorption of the $^{111}$In-human IL-21 was apparently still occurring. However, estimations of half-lives following intravenous, subcutaneous, and intraperitoneal dosing were 13.6 min., 18.8 min., and 34.3 min., respectively. Since a dosing range was not evaluated it was not apparent whether saturable or active elimination (Michaelis Menten kinetics) was occurring. Therefore, these half-life calculations are estimations.

Estimates of the bioavailability of the labeled protein were made based on the area under the curve (AUC) following subcutaneous or intraperitoneal dosing compared to that of intravenous dosing. The estimated bioavailability following subcutaneous and intraperitoneal injection were 35.8% and 63.9% respectively. Because only one protein dose was studied, the bioavailability was not evaluated as a function of dose. The estimated clearance and volume of distribution (based on the data from the intravenous injection) were 0.48 ml/min. and 6.1 ml, respectively.

Although the data are preliminary, the fate of IL-21 administered IV was similar to that reported for IL-2, another 4-helix bundle cytokine (Donahue, JH and Rosenburg, SA supra.). Like IL-2, IV-administered IL-21 had a plasma half life of only minutes with the main clearance in the kidney. Three hours after injection, the majority of the labeled material extracted from kidney was still retained in a Biomax 5K NMLW membrane (Millipore). Since it has previously been reported that the indium remains associated with protein even during lysosomal degradation (Staud, F. et al., J. Pharm. Sciences 88:577-585, 1999) IL-21 is accumulating and may be degraded in the kidney. The current study also showed, as observed with many other proteins, including IL-2 (Donahue, JH and Rosenburg, SA, supra.), that IP and SC administration significantly prolonged the plasma levels of IL-21.

Example 5

Isolation and Expansion of Fresh Human Bone Marrow MNC CD34+ Fraction Using IL-21 for Assessment of NK Activity

A. Selection and Isolation of CD34+ cells from human Bone Marrow

Fresh human bone marrow mononuclear cells (MNC) were prepared to enrich for cells having NK cell activity. Fresh human MNCs were obtained from Poeitic Technologies (Gaithersburg, MD). 10 ml alpha MEM (JRH, Lenexa, KS) containing 10% HIA FBS (Hyclone, Logan, UT) and the antibiotic 1% PSN (Gibco, BRL, Grand Island, NY) was added to the cell suspension and the cells were passed through a 100 $\mu$m sieve. The cells were then counted, pelleted, washed with 10 ml PBS containing 2% FBS, then pelleted again and resuspended in 1 ml PBS containing 2% FBS. Cells having a CD34 cell surface marker (CD34+ cells) were magnetically separated using a Detachabead kit with Dynabeads M-450 CD34 ((Dynal, Oslo, Norway), as per manufacturer's instructions. Both the CD34+ cell and the CD34- cell fractions were further analyzed below.

B. Expansion of CD34+ cells using IL-21

A CD34+ cell fraction was plated into four wells in a 24-well plate. 50,000 positively selected cells suspended in 1 ml Alpha MEM (JRH) containing 10% HIA FBS (Hyclone) and 1% PSN (Gibco/BRL), plus the various cytokines described below were plated in each of the 4 wells (1-4). Various reagents were used to test for IL-21-induced expansion of the CD34+ selected bone marrow MNCs: Reagents included human flt3 (R&D, Minneapolis, MN); purified human IL-21 (U.S. Patent No. 6,307,024); human IL-15 (R&D). Reagents were combined as follows at day 0: In well #1, 2 ng/ml human flt3 was added. In well #2, 2ng/ml human flt3 and 15 ng/ml purified human IL-21 were added. In well #3, 2 ng/ml human flt3 and 20 ng/ml human IL15 were added. In well #4, 2 ng/ml human flt3, 15 ng/ml purified human IL-21, and 20 ng/ml human IL15 were added. After incubating for 18 days, the suspension cells from each well were pelleted, and then resuspended in 0.5 ml alpha MEM (JRH) containing 10% HIA FBS (Hyclone) and 1% PSN (Gibco/BRL), and counted to assess proliferation of the CD34+ cell fraction. A low level of proliferation was seen in the presence of flt3 alone (control well #1), but the presence of IL-15 or IL-21 in addition to flt3 had not significant effect on the expansion (wells, #2 and #3). However, expansion beyond the flt3 control was evident in well #4 which contained IL-15 and IL-21 in addition to flt3. This result suggested that IL-21 and IL-15 act in synergy to expand the human CD34+ cell population. Moreover, the results of this experiment supported the results seen with the mouse IL-21 in the mouse BM assay (Example 1).

All cell populations were then tested for NK activity and subjected to flow cytometry analysis, as shown below (Example 7).

C. Expansion of CD34+ or CD34- cells using IL-21 with delayed addition of IL-15

Both CD34 positive and negative (CD34-) fractions were plated separately into six 12 well plate wells (1-6). Each of six wells contained 100,000 positively or negatively selected cells in 2 ml alpha MEM containing 10%HIA FBS and PSN, described above. Reagents used were as described above. In well #1, 2 ng/ml human flt3 was added at day 0. In well #2, 2 ng/ml human flt3 was added at day 0, and after 5 days incubation 20 ng/ml human IL15 was added. In well #3, 2 ng/ml human flt3 and 15 ng/ml human IL-21 were added at day 0. In well #4, 2 ng/ml human flt3 and 15 ng/ml human IL-21 were added at day 0, and after 5 days incubation 20 ng/ml human IL15 was added. In well #5, 2 ng/ml human flt3 and 20 ng/ml human IL15 were added at day 0. In well #6, 2 ng/ml human flt3, 15 ng/ml human IL-21, and 20 ng/ml human IL15 were added at day 0. After incubating for a total of 15 days from the start of the experiment, the cells from each well were harvested and counted.

In the CD34+ population a low level of proliferation was seen in the presence of flt3 alone (control well #1), but the presence of IL,-15 or IL-21 added at day 0 in addition to flt3 had no significant effect on the expansion (wells, #3 and #5). Addition of IL-15 after 5 days had some proliferative effect in comparison to the flt3 control (well #2 compared to well #1) and a proliferative effect in the presence of zalpha11 (well #4 compared to well #3). However, the greatest expansion was evident in well #6 which contained IL-15 and IL-21 in addition to flt3 at day 0.

In the CD34- population, no proliferation was seen in the presence of flt3 alone (control well #1), and in fact a decrease in the cell population was evident. The presence of zalpha11 added at day 0 in addition to flt3 (well #3) was similar to the flt3 control. The presence of IL-15 added at day 5 increased proliferation effect of the cells in the presence (well #4) or absence (well #2) of IL-21. Again, the greatest expansion was evident in well #6 which contained IL-15 and IL-21 in addition to flt3 at day 0.

All cell populations were then tested for NK activity and subjected to FACS analysis, as shown below (Example 7).

Example 6

Isolation and Expansion of Fresh Mouse Cells Using Human and Mouse IL-21 for Assessment of NK Activity and NK Cell Markers

A. Isolation and Expansion of fresh mouse low density bone marrow cells musing human and mouse IL-21

Fresh mouse marrow cells were isolated by clipping both ends of mouse femurs, and flushing two to three milliliters of growth medium (see below) through the inside of the bone into a collection tube. The growth medium was 500 ml RPMI 1640 Medium (JRH Biosciences. Lenexa, KS); 5 ml 100x L-glutamine (Gibco BRL. Grand Island, NY); 5 ml 100x Na Pyruvate (Gibco BRL); 5 ml 100X Penicillin, Streptomycin, Neomycin (PSN) (Gibco BRL); and 50 ml heat-inactivated Fetal Bovine Serum (FBS) (Hyclone Laboratories. Logan, UT). The marrow cells were then broken-up by pipeting the media up and down several times. The cells were then pelleted and washed once with growth medium, and passed through a 70-micron sieve. The low-density mononuclear cells were then isolated by subjecting the marrow cells to a density gradient. Marrow cells in five to eight milliliters of growth medium were carefully pipetted on top of five to eight milliliters of NycoPrep 1.077 Animal (Nycomed. Oslo, Norway) in a centrifuge tube. This gradient was then centrifuged at 600 X g for 20 minutes. The low density mononuclear cells were harvested from the interface layer between the NycoPrep and the medium. These cells were then diluted to approximately 20 milliliters in growth medium, pelleted and washed. The cells were then plated at approximately 0.5-1.5x$10^6$ cells per milliliter in growth medium in a standard tissue culture flask and incubated at $37^0$ C, 5% $CO_2$ for two hours.

The non-adherent, low density (NA LD) marrow cells were then harvested and plated at 0.5-2.0x$10^5$ cells per milliliter in growth medium plus 2.5 nanograms per milliliter mouse flt3 (R and D Systems. Minneapolis, MN) plus 25 to 50 nanograms per milliliter human Interleukin 15 (IL-15) (R and D Systems) with or without 50 to 150 nanograms per milliliter human IL-21; or with or without 0.12 to 10 nanograms per milliliter mouse IL-21.

There was no significant expansion without the addition of the human or mouse IL-21. Non-adherent cells were expanded in the cultures containing mouse IL-21 as low as 0.12 ng/ml and in the cultures containing human IL-21 as low as 22 ng/ml. In cultures containing both the human and mouse IL-21, non-adherent cell expansion increased with increasing dose if IL-21, with the mouse ligand saturating response at about 5-10 ng/ml and the human not reaching a saturating response even at the highest dose of 200 ng/ml. Human IL-21 appeared to be approximately 20 to 100 fold less potent on mouse cells as the mouse IL-21. After approximately five to ten days the IL-21 expanded mouse cells were harvested and analyzed by flow cytometry (FACSCalibur; Becton Dickinson, Mansfield, MA) to determine what percentage of them were positive for NK cell antigens, where 46% were positive for the PanNK cell marker DX5(Pharmingen).

B. Isolation and Expansion of Fresh lineage Depleted Mouse Marrow Cells

Fresh mouse lineage depleted (lin-) marrow cells were isolated from fresh mouse marrow cells by first incubating the cells with the following antibodies: TER119, Gr-1, B220, MAC-1, CD3e and I-Ab (Pharmingen. San Diego, CA). The lin+ cells were then removed with Dynabeads M-450 sheep anti-rat IgG (Dynal, Lake Success, NY) as per manufacturer's instructions.

The negatively selected lin- marrow cells were then plated as above in growth medium plus either 2.5 ng/mL flt3 (R&D Systems) and 25 ng/mL IL-15 (R&D Systems); or flt3, IL-15 and mouse IL-21, 2 to 5% BHK mouse IL-21 conditioned medium. After six days of growth, the cultures were harvested, counted and submitted to an NK cell activity assay (Example 7). Cells grown with mouse IL-21 were approximately two to three times more effective at lysing NK cell target cells (YAC-1 cells) as the cells grown without IL-21.

C. Isolation and Expansion of CD4- CD8- (Double Negative or DN) Thymocytes

Fresh mouse thymocytes were isolated by chopping and sieving thymuses from three to eight week old mice. CD4- CD8- (DN) cells were then negatively selected by incubating the thymocytes with anti-CD4 and anti-CD8 antibodies (PharMingen), then removing the CD4+ CD8+ cells with Dynabeads M-450 sheep anti-rat IgG (Dynal) as per manufacturer's instructions.

The DN mouse thymocytes were then grown in growth medium plus 2.5 ng/mL flt3 (R&D Systems), 25 ng/mL IL-15 (R&D Systems) and 10 ng/mL IL-7 (R&D Systems) with or without mouse IL-21 as above. Six days later the cells were harvested, counted, analyzed by flow cytometry as described above, and also submitted to an NK cell activity assay (Example 7).

The culture grown with mouse IL-21 yielded approximately 480,000 cells while the culture without IL-21 yielded only approximately 160,000 cells. The culture grown with mouse IL-21 was found to be approximately 16.2% positive for the NK cell antigen Pan NK, DX5 (PharMingen). The culture grown without IL-21 was 14.6% positive for DX5. The cells grown with IL-21 lysed NK cell target cells, YAC-1, approximately two times better than the cells grown without IL-21. The expanded cells did not lyse significantly a negative control target cell line, EL4. These results suggested that IL-21 selectively expands lytic NK cells.

Example 7

Activity of Human and Mouse IL-21 Expanded Cells and Mature Murine NK Cells in NK Cell Cytotoxicity Assays

A. NK cell assay

NK cell-mediated target cytolysis was examined by a standard $^{51}$Cr-release assay. Target cells (K562 cells (ATCC No. CCL-243) in human assays, and YAC-1 cells (ATCC No. TIB-160) in mouse assays) lack expression of major histocompatability complex (MHC) molecules, rendering them susceptible to NK cell-mediated lysis. A negative control target cell line in mouse assays is the MHC$^+$ thymoma EL4 (ATCC No. TIB-39). We grew K562, EL4, and YAC-1 cells in RP10 medium (standard RPMI 1640 (Gibco/BRL, Grand Island, NY) supplemented with 10% FBS (Hyclone, Logan, UT), as well as 4 mM glutamine (Gibco/BRL), 100 I.U./ml penicillin+100 MCG/ml streptomycin (Gibco/BRL), 50 $\mu$M $\beta$-mercaptoethanol (Gibco/BRL) and 10m HEPES buffer (Gibco/BRL). On the day of assay, 1-2x10$^6$ target cells were harvested and resuspended at 2.5-5x10$^6$ cells/ml in RP10 medium. We added 50-100 $\mu$l of 5 mCi/ml $^{51}$Cr-sodium chromate (NEN, Boston, MA) directly to the cells and incubated them for 1 hour at 37°C, then washed them twice with 12 ml of PBS and resuspended them in 2 ml of RP10 medium. After counting the cells on a hemacytometer, the target cells were diluted to 0.5-1x10$^5$ cells/ml and 100 $\mu$l (0.5-1x10$^4$ cells) were mixed with effector cells as described below.

In human assays, effector cells were prepared from selected and expanded human CD34$^+$ BM cells (Example 5B) which were harvested, washed, counted, mixed at various concentrations with $^{51}$Cr-labeled target cells in 96-well round bottomed plates, and incubated for 4 hours at 37°C. After co-incubation of effector cells and the labeled target cells, half of the supernatant from each well was collected and counted in a gamma counter for 1 min/sample. The percentage of specific $^{51}$Cr release was calculated from the formula 100 x (X-Y)/(Z-Y), where X is $^{51}$Cr release in the presence of effector cells, Y is the spontaneous release in the absence of effectors, and Z is the total $^{51}$Cr release from

target cells incubated with 0.5% Triton X-100. Data were plotted as the % specific lysis versus the effector-to-target ratio in each well.

B. Activity of human IL-21 expanded cells

Isolated $CD34^+$ human HPCs cultured with flt3 +/- IL-21 and flt3 +IL-15 +/- IL-21 (Example 5), were harvested the cells on day 15 to assess their capacity to lyse $MHC^-$ K562 cells in a standard $^{51}$Cr-release assay as described above, and to analyze their surface phenotype by flow cytometry. As expected from previous reports (Mrozek, E et al., Blood 87:2632-2640, 1996; and Yu, H et al., Blood 92:3647-3657, 1998), simultaneous addition of IL-15 and flt3L did induce the outgrowth of a small population of $CD56^+$ cells. Interestingly, although BM cells cultured simultaneously with IL-21 and flt3L did not expand significantly, there was a significant increase in total cell numbers in cultures containing a combination of flt3L, IL-21 and IL-15 (see, Example 5).

For an assessment of the surface phenotype of these human BM cultures, we stained small aliquots of the cells for 3-color flow cytometric analysis with anti-CD3-FITC, anti-CD56-PE and anti-CD16-CyChrome mAbs (all from PharMingen, San Diego, CA) and analyzed them on a FACSCalibur using CellQuest software (Becton Dickinson, Mountain View, CA). This flow cytometric analysis confirmed that the cells growing out of these cultures were differentiated NK cells, as they were large and granular and expressed both CD56 and CD16, and were $CD3^-$ (Lanier, LL Annu. Rev. Immunol. 16:359-393, 1998). Furthermore, these cells exhibited significantly higher effector function than those cells grown with IL-15 and flt3. More specifically, cells grown in all three cytokines lysed more than 40% of the K562 targets at an effector-to-target ratio (E:T) of 1.5, whereas cells grown in IL-15+flt3L lysed fewer than 5% of the targets at an E: T of 2. These data demonstrate that, in combination with IL-15, IL-21 stimulates the differentiation of NK cells from $CD34^+$ BM cells.

C. Activity of mouse IL-21 expanded cells

To test the effects of IL-21 on murine hematopoietic progenitor cells, purified Lineage-negative (Lin-) bone marrow cells from C57B1/6 mice were expanded in flt3+IL-15+/- IL-21, as described in Example 6B. On day 6 of culture, the cells ("effectors") were harvested and counted, then resuspended in 0.4 ml of RP10 medium (Example 7A). Two aliquots (0.15 ml each) of each sample expanded with or without IL-21 (Example 7A) were diluted serially 3-fold in duplicate in 96-well round bottomed plates, for a total of 6 wells of 100 μl each. The remaining 100 μl of cells were stained for NK cell surface markers with FITC-anti-2B4 and PE-anti-DX5 mAbs (PharMingen) and analyzed by flow cytometry. Each group of cells exposed to flt3+IL-15 with or without the presence of IL-21 had similar fractions of 2B4+DX5+ cells, ranging from 65-75% positive for both NK markers.

For the NK lysis assay, target cells (YAC-1 and EL4) were labeled with $^{51}$Cr as described above. After counting the target cells on a hemacytometer, the target cells were diluted to 0.5-1x$10^5$ cells/ml and 100 μl of YAC-1 or EL4 (0.5-1x$10^4$ cells) were mixed with 100 μl effector cells and incubated for 4 hours at 37°C. Specific lysis was determined for each well as described above.

We found that cells grown in the presence of flt3+IL-15+IL-21 exhibited enhanced lytic activity (roughly 2-fold) against the YAC-1 targets (but did not kill the $MHC^+$ control cell line EL4). At an effector-to-target ratio (E :T) of 5, NK cells generated in the presence of all 3 cytokines (IL-21 +flt3+IL-15) lysed 12% of the YAC-1 cells, whereas those NK cells expanded with flt3+IL-15 lysed 6% of the YAC-1 targets. Subsequent experiments confirmed this trend.

In a second approach to determine the biological activity of IL-21 on murine NK cells, we isolated immature $CD4^-CD8^-$ ("double negative", DN) mouse thymocytes as described in Example 6C and cultured them with IL-15+flt3+IL-7 or IL-15+flt3+IL-2, with or without IL-21. On day 6 of culture, the cells were harvested and assayed for NK lytic activity on YAC-1 and EL4 cells as described above. We found that cells cultured in the presence of IL-21 had the greatest lytic activity in this assay, with enhanced lytic activity over those cells cultured in the presence of the other cytokines. Specifically, DN thymocytes grown with IL-15+flt3+IL-7 killed 18% of the YAC-1 cells at E:T of 24 while cells grown in the presence of IL-15+flt3+IL-7 plus IL-21 killed 48% of the targets at the same E:T. DN thymocytes grown in IL-15+flt3+IL-2 killed 15% of the YAC-1 targets at an E:T of 6, whereas cells grown with these 3 cytokines and IL-21 killed 35% of the YAC-1 cells at an E:T of 9. Flow cytometry was performed on the cultured cells one day before the NK lysis assay. As was true for the bone marrow cultures, despite the proliferative effect of IL-21 (cell numbers increase approximately 2-fold when IL-21 is added), it did not significantly enhance the fraction of $DX5^+$ cells (17-20% of total cells in the cultures with IL-7, and 35-46% of total in cultures with IL-2). These data imply that IL-21, in combination with IL-15 and flt3, enhances the lytic activity of NK cells generated from murine bone marrow or thymus.

D. Activity of mouse IL-21 on Mature murine NK cells

In order to test the effects of mouse IL-21 on mature NK cells, we isolated spleens from four 5-week old C57B1/6 mice (Jackson Laboratories, Bar Harbor, ME) and mashed them with frosted-end glass slides to create a cell suspension. Red blood cells were removed by hypotonic lysis as follows: cells were pelleted and the supernatant removed by aspiration. We disrupted the pellet with gentle vortexing, then added 900 μl of sterile water while shaking, followed quickly (less than 5 sec later) by 100 μl of 10X HBSS (Gibco/BRL). The cells were then resuspended in 10 ml of 1X HBSS and debris was removed by passing the cells over a nylon mesh-lined cell strainer (Falcon). These RBC-depleted spleen cells were then pelleted and resuspended in MACS buffer (PBS+1%BSA+2mM EDTA) and counted. We stained $300x10^6$ of the cells with anti-DX5-coated magnetic beads (Miltenyi Biotec) and positively selected $DX5^+$ NK cells over a MACS VS+ separation column, according to the manufacturer's instructions, leading to the recovery of $8.4x10^6$ $DX5^+$ cells and $251x10^6$ $DX5^-$ cells. Each of these groups of cells were cultured in 24-well plates ($0.67x10^6$ cells/well, 2 wells per treatment condition) in RP10 medium (Example 7A) alone or with 1) 30 ng/ml mouse IL-21, 2) 30 ng/ml recombinant mouse IL-2 (R&D Systems, Inc., Minneapolis, MN), 3) 30 ng/ml recombinant human IL-15 (R&D), 4) 30 ng/ml each of mouse IL-21 and hIL-15, or 5) 30 ng/ml each of mIL-2 and ML-15. The cells were harvested after 21 hours, washed, and resuspended in RP10 medium and counted. The cells were then assayed for their ability to lyse $^{51}Cr$-labeled YAC-1 or EL4 targets cells, as described in Example 7A.

In general, there was little NK activity from the $DX5^-$ (non-NK cells) groups, but the $DX5^-$ cells cultured with IL-21 and hIL-15 did lyse 25% of the YAC-1 target cells at an E:T of 82. By comparison, $DX5^-$ cells cultured with hIL-15 alone lysed 14% of the YAC-1 targets at an E:T of 110. This suggests that IL-21 and IL-15 are acting together on the residual $NK1.1^+$ NK cells in this cell preparation. As for the $DX5^+$ cell preparation, treatment with mouse IL-21 alone did not significantly increase their effector function (their lysis of YAC-1 cells was similar to the untreated group). As expected, both IL-2 and IL-15 significantly improved NK activity. The highest level of lysis, however, was detected in the group treated with IL-21 and hIL-15 (65% lysis of YAC-1 cells at an E:T of 3.3, vs. 45% lysis at an E:T of 4 for the hIL-15 treatment group). Taken together, these results suggest that although IL-21 alone may not increase NK cell lysis activity, it does enhance NK lysis activity of mature NK cells, when administered with IL-15.

## Example 8

### TL-21 Proliferation of Human and Mouse T-cells in a T-cell

### Proliferation Assay

### A. Murine IL-21 Proliferation of Mouse T-cells

T cells from C57B1/6 mice (Jackson Laboratories, Bar Harbor, ME) were isolated from pooled splenocytes and lymphocytes from axillary, brachial, inguinal, cervical, and mesenteric lymph nodes (LNs). Spleens were mashed with frosted-end glass slides to create a cell suspension. LNs were teased apart with forceps and passed through a cell strainer to remove debris. Pooled splenocytes and LN cells were separated into $CD8^+$ and $CD4^+$ subsets using two successive MACS magnetic separation columns, according to the manufacturer's instructions (Miltenyi Biotec, Auburn, CA). Whole thymocytes were collected from the same mice.

Cells were cultured at $3x10^5$ cells/well (thymocytes) or $10^5$ cells/well (mature T cells) with increasing concentrations of purified murine IL-21 (0-30 ng/ml) (U.S. Patent No. 6,307,024) in 96-well flat bottomed plates pre-coated overnight at 4°C with various concentrations of anti-CD3 mAb 2C11 (PharMingen) for 3 days at 37°C. The anti-CD3 antibody served to activate the murine T-cells through the T-cell receptor. Each well was pulsed with 1μCi $^3H$-thymidine on day 2 and plates were harvested and counted 16 hours later to assess proliferation.

When we tested IL-21 in T cell proliferation assays, we found that it co-stimulated anti-CD3-activated murine thymocytes, leading to an accelerated outgrowth of $CD8^+CD4^-$ cells (the majority of the thymocytes cultured with anti-CD3+IL-21 were $CD8^+CD4^-$ by day 3 of culture, while cells cultured with anti-CD3 alone did not significantly skew to this phenotype until day 5). We did not observe significant levels of proliferation of thymocytes to IL-21 in the absence of anti-CD3.

Interestingly, when we assayed mature peripheral murine T cells for their ability to respond to IL-21+anti-CD3, we found that only the $CD8^+$, but not the $CD4^+$ subset, responded in a dose-dependent manner to IL-21. We also observed weak but reproducible proliferation of $CD8^+$ cells (but not $CD4^+$ cells) in response to IL-21 alone. Interestingly, this was not observed for human T cells (see Example 8B, below).

### B. Human IL-21 Proliferation of Human T-cells

Human CD4+ and CD8+ T cells were isolated from PBMC as described in Example 9 (below) Cells were cultured at about $10^5$ cells/well with increasing concentrations of purified human IL-21 (0-50 ng/ml) (U.S. Patent No. 6,307,024) in 96-well flat bottomed plates pre-coated overnight at 4°C with various concentrations of anti-human CD3 mAb UCHT1 (PharMingen) for 3 days at 37°C. Each well was pulsed with luCi $^3$H-thymidine on day 2 and plates were harvested and counted 16 hours later. Unlike our results with mouse T cells, our preliminary data suggests that human IL-21 co-stimulates CD4+, but not CD8+, human T cells in a dose-dependent fashion.

In other experiments, mature murine CD4+ and CD8+ T cells were enriched from pooled C57B1/6 spleen and LN cells by depletion of $CD19^+$ B cells using a magnetic bead column. The resulting cell populations were assayed for proliferation to plate-bound anti-mouse CD3ε mAb in the absence or presence of increasing concentrations of murine IL-21, as indicated. Data shown are representative of results from 4 experiments.

T cells from C57B1/6 mice were isolated from pooled splenocytes and lymphocytes from auxiliary, brachial, inguinal, cervical, and mesenteric LNs. Spleens were mashed with frosted-end glass slides to create a cell suspension. LNs were teased apart with forceps and passed through a cell strainer to remove debris. Pooled splenocytes and LN cells were separated into $CD8^+$ and $CD4^+$ subsets using two successive MACS magnetic separation columns, according to the manufacturer's instructions (Miltenyi Biotec, Sunnyvale, CA). Cells were cultured at $10^5$/well with increasing concentrations of murine IL-21 (0-30 ng/ml) in 96-well flat bottomed plates pre-coated overnight at 4°C with various concentrations of anti-CD3ε mAb 2C11 (PharMingen) for 3 days at 37°C. Each well was pulsed with 1μCi $^3$H-thymidine on day 2 and plates were harvested and counted 16 hours later.

Table 5 illustrates that mIL-21 co-stimulates the proliferation of murine CD8+ T cells. Values represent the CPM incorporated of $^3$H-thymidine (average +/standard deviation of triplicate wells).

Table 5

| | | **Anti-CD3 mAb (ug/ml)** | | | | |
|---|---|---|---|---|---|---|
| | **ng/ml mIL-21** | **0** | **0.11** | **0.33** | **1.0** | **3.0** |
| CD4+ | **0** | 405 +/-101 | 67895 +/-18752 | 141175 +/- 6733 | 202251 +/- 35571 | 246626+/-45106 |
| | **1.2** | 247+/-86 | 80872+/-23598 | 126487+/-7472 | 178863 +/- 33583 | 205861+/-14675 |
| | **6** | 302 +/-106 | 75192 +/-5323 | 102005 +/- 20059 | 191598 +/- 15881 | 218718 +/-12142 |
| | **30** | 364+/-126 | 86164 +/-8065 | 141065 +/- 4921 | 186089 +/- 17585 | 266650 +/-39839 |
| | **0** | 168+/-47 | 40198+/-4557 | 70272+/-4141 | 84771 +/-9450 | 97869 +/-3368 |
| CD8+ | **1.2** | 268+/-117 | 50095+/-3959 | 84319+/-6373 | 105176 +/-10828 | 113394 +/-3657 |
| | **6** | 323+/-159 | 78113 +/-6967 | 108461 +/-2175 | 132301 +/-13386 | 178551 +/-16373 |
| | **30** | 2007 +/-470 | 132238 +/-1915 | 182485 +/-4991 | 272229 +/-9325 | 330434+/-47185 |

Example 9

Real Time PCR Shows IL-21 Expression in Human CD4+ cells

A. Purified Human T cells as a Primary Source used to assess human IL-21 Expression

Whole blood (150 ml) was collected from a healthy human donor and mixed 1:1 with PBS in 50 ml conical tubes. Thirty ml of diluted blood was then underlayed with 15 ml of Ficoll Paque Plus (Amersham Pharmacia Biotech, Uppsala, Sweden). These gradients were centrifuged 30 min at 500 g and allowed to stop without braking. The RBC-depleted cells at the interface (PBMC) were collected and washed 3 times with PBS. The isolated human PBMC yield was $200x10^6$ prior to selection described below.

The PBMCs were suspended in 1.5 ml MACS buffer (PBS, 0.5% EDTA, 2mM EDTA) and $3x10^6$ cells were set aside for control RNA and for flow cytometric analysis. We next added 0.25 ml anti-human CD8 microbeads (Miltenyi Biotec) and the mixture was incubated for 15 min at 4°C. These cells labeled with CD8 beads were washed with 30 ml MACS buffer, and then resuspended in 2 ml MACS buffer.

A VS+ column (Miltenyi) was prepared according to the manufacturer's instructions. The VS+ column was then placed in a VarioMACS magnetic field (Miltenyi). The column was equilibrated with 5 ml MACS buffer. The isolated primary mouse cells were then applied to the column. The CD8 negative cells were allowed to pass through. The column was rinsed with 9 ml (3 X 3 ml) MACS buffer. The column was then removed from the magnet and placed over a 15 ml falcon tube. CD8+ cells were eluted by adding 5 ml MACS buffer to the column and bound cells flushed out using the plunger provided by the manufacturer. The yield of CD8+ selected human peripheral T cells was about $51x10^6$ total cells. The CD8-negative flow through cells were collected, counted, stained with anti-human CD4 coated beads, then incubated and passed over a new VS+ column at the same concentrations as described above. The yield of CD4+ selected human peripheral T cells was $42x10^6$ total cells.

A sample of each of the CD8+ and CD4+ selected human T cells was removed for staining and sorting on a fluorescence activated cell sorter (FACS) to assess their purity. A PE-conjugated anti-human CD4 antibody, an anti-human CD8-FITC Ab, and an anti-human CD19-CyChrome Ab (all from PharMingen) were used for staining the CD8+ and CD4+ selected cells. The CD8-selected cells in this first experiment were 80% CD8+, and the CD4-selected cells were 85% CD4+. In 2 subsequent experiments (Example 9B), the CD8+ purified cells were 84% and 81% pure, and the CD4+ cells were 85% and 97% pure, respectively. In one experiment, we stained the non-binding (flow-through) cells with anti-human CD19-coated beads (Miltenyi) and ran them over a third magnetic bead column to isolate CD19+ B cells (these were 92% pure).

The human CD8+, CD4+ and CD19+ selected cells were activated by incubating $0.5X10^6$ cells/ml in RPMI + 5% human ultraserum (Gemini Bioproducts, Calabasas, CA) + PMA 10 ng/ml and Ionomycin 0.5 μg/ml (Calbiochem) for about 4, 16, or 24 hours at 37˚C. The T-cells ($2.5x10^6$/well) were alternately stimulated in 24-well plates pre-coated overnight with 0.5 μg/ml plate-bound anti-CD3 mAb UCHT1 (PharMingen) with or without soluble anti-CD28 mAb (PharMingen) at 5 μg/ml. At each timepoint, the cells were harvested, pelleted, washed once with PBS, and pelleted again. The supernatant was removed and the pellets were snap-frozen in a dry ice/ethanol bath, then stored at -80˚C for RNA preparation at a later date.

Real Time-PCR was performed on these human CD8+, CD4+ and CD19+ selected cells as described in Example 9B and Example 9C below for assessing human IL-21 and receptor expression.

B. Primers and Probes for Quantitative RT-PCR for human IL-21 expression

Real-time quantitative RT-PCR using the ABI PRISM 7700 Sequence Detection System (PE Applied Biosystems, Inc., Foster City, CA) has been previously described (see, Heid, CA et al., Genome Research 6:986-994, 1996; Gibson, UEM et al., Genome Research 6: 995-1001, 1996; and Sundaresan, S et al., Endocrinology 139:4756-4764, 1998). This method incorporates use of a gene specific probe containing both reporter and quencher dyes. When the probe is intact the reporter dye emission is negated due to the proximity of the quencher dye. During PCR extension using additional gene-specific forward and reverse primers, the probe is cleaved by 5' nuclease activity of Taq polymerase which releases the reporter dye resulting in an increase in fluorescent emission.

The primers and probes used for real-time quantitative RT-PCR analyses were designed using the primer design software Primer ExpressTM (PE Applied Biosystems). Primers for human IL-21 were designed spanning an intron-exon junction to eliminate amplification of genomic DNA. The forward primer, ZC22,281 (SEQ ID NO:11) and the reverse primer, ZC22,279 (SEQ ID NO:12) were both used at 300 nM concentration to synthesize an 80 bp product. The corresponding IL-21 TaqMan probe, ZG32 (SEQ ID NO:13) was synthesized by PE Applied Biosystems. The probe was labeled with a reporter fluorescent dye (6-carboxyfluorescein) (FAM) (PE Applied Biosystems) at the 5' end and a quencher fluorescent dye (6-carboxy-tetramethyl-rhodamine) (TAMRA) (PE Applied Biosystems) at the 3' end. In order to test the integrity or quality of all the RNA samples, they were screened for rRNA using the primer and probe set ordered from PE Applied Biosystems (cat No. 4304483). The reporter fluorescent dye for this probe is VIC (PE Applied Biosystems). The rRNA results will allow for the normalization of the IL-21 results.

RNA was prepared from pellets provided in Example 9A, using RNeasy Miniprep™ Kit (Qiagen, Valencia, CA) per the manufacturer's instructions. Control RNA was prepared from about 10 million BHK cells expressing human IL-21

C. Primers and Probes for Quantitative RT-PCR for human zalphall Receptor expression

Real time PCR was performed to assess the expression of IL-21 receptor as per Example 9B and Example 9D, using the cells prepared under the conditions detailed in 43A, and probes specific for the IL-21 receptor. The forward primer, ZC22,277 (SEQ ID NO:14) and the reverse primer, ZC22,276 (SEQ ID NO:15) were used in a PCR reaction (above) at about 300 nM concentration to synthesize a 143 bp product. The corresponding IL-21 TaqMan® probe, designated ZG31 (SEQ ID NO:16) was synthesized and labeled by PE Applied Biosystems. RNA from BaF3 cells expressing human IL-21 receptor was used to generate appropriate control for standard curves for the real-time PCR described in Example 9D below.

D. Real-time quantitative RT-PCR

Relative levels of IL-21 RNA were determined by analysis of total RNA samples using the One-Step RT-PCR method (PE Applied Biosystems). RNA from BHK cells expressing human IL-21 was used to generate a standard curve. The curve consisted of serial dilutions ranging from $2.5\text{-}2.5x10^{-4}$ng for the rRNA screen and 25-0.0025 ng for the IL-21 screen with each point analyzed in triplicate. The total RNA samples were also analyzed in triplicate for human IL-21 transcript levels and for levels of rRNA as an endogenous control. Each One-step RT-PCR reaction consisted of 25 ng of total RNA in buffer A (50 mM KCL, 10 mM Tris-HCL, and the internal standard dye, ROX (PE Applied Biosystems)), appropriate primers (50 nM for rRNA samples, 300 nM for IL-21 samples) and probe (50 nM for rRNA, 100 nM for IL-21 ), 5.5 mM $MgCl_2$, 300 μM each d-CTP, d-ATP, and d-GTP and 600 μM of d-UTP, reverse transcriptase (0.25 U/μl), AmpliTaq DNA polymerase (0.025 U/μl) and RNase Inhibitor (0.4 U/μl) in a total volume of 25 μl. Thermal cycling conditions consisted of an initial RT step at 48°C for 30 minutes, an AmpliTaq Gold activation step of 95°C for 10 minutes, followed by 40 cycles of amplification for 15 seconds at 95°C and 1 minute at 60°C. Relative IL-21 RNA levels were determined by the Standard Curve Method as described in User Bulletin No. 2 (PE Biosystems; User Bulletin #2: ABI Prism 7700 Sequence Detection System, Relative Quantitation of Gene Expression, December 11, 1997) using the rRNA measurements to normalize the IL-21 levels. Samples were compared relative to the calibrator within each experiment. The calibrator was arbitrarily chosen based on good quality RNA and an expression level to which other samples could significantly be compared. Results of the experiments analyzing the expression of the IL-21 and IL-21 receptor in stimulated and unstimulated cells (Example 9A) are as described in Example 9E below.

E. Expression of human IL-21 1 Receptor and Ligand in CD4+, CD8+ and CD19+ cells

The first experiment used RT-PCR, described above, to assess zalphall receptor expression in unstimulated and ariti-CD3 stimulated CD4+ and CD8+ samples at timepoints of 0h (unstimulated ("resting") cells), and at 4h, 15.5h and 24h, after stimulatoin. The resting CD4+ sample was arbitrarily chosen as the calibrator and given a value of 1.00. There was approximately a 4-fold increase in receptor expression in unstimulated CD4+ cells from 4h to 24h of culture and about an 8-fold increase over the same time period in anti-CD3 stimulated CD4+ cells. The CD8+ cells showed a 7-fold increase in IL-21 receptor expression that peaked at 4hrs and decreased over time. With anti-CD3 stimulation, the CD8+ cells had a constant 8-fold increase in receptor expression.

This first experiment also used RT-PCR to assess IL-21 expression in the same anti-CD3 stimulated and unstimulated CD4+ and CD8+ samples. The 4 hr anti-CD3 stimulated CD8+ sample was arbitrarily chosen as the calibrator and given a value of 1.00. The results showed that unstimulated CD4+ and CD8+ cells do not express IL-21 . We observed a significant elevation of expression in the anti-CD3 stimulated CD4+ cells at 4 h, with about a 300-fold increase in signal observed at 15.5 h. The CD8+ cells expressed a small amount of ligand upon anti-CD3 stimulation, however this is probably due to contamination of the CD8+ population with a small number of CD4+ cells.

The second experiment used RT-PCR to assess IL-21 receptor expression in anti-CD3-stirnulated, PMA + Ionomycin-stimulated and unstimulated CD4+ and CD8+ samples at timepoints of 0 h, and at 3.5 h, 16 h and 24 h after activation. The resting CD8+ sample was arbitrarily chosen as the calibrator and given a value of 1.00. The resting CD4+ and CD8+ cells did not have significant amounts of receptor expression. The expression was about 3 fold higher in the PMA + Ionamycin-stimulated CD4+ samples at 3.5 h, 16 h and 24 h after stimulation. The expression in anti-CD3 activated CD4+ cells peaked at 10-fold above background levels at 3.5 h after stimulation, then fell back to levels 4-fold above background at 16 h after stimulation. The CD8+ cells showed a 4-fold expression increase at 3.5 h after PMA + Ionomycin stimulation, with expression decreasing at subsequent timepoints. As in the first experiment, the anti-CD3 stimulated CD8+ cells again exhibited an 8-fold above background induction of receptor expression.

These samples from the second experiment were also used to assess IL-21 expression. The 24hr PMA + Ionomycin stimulated CD4+ sample was arbitrarily chosen as the calibrator and given a value of 1.00. The results showed that again none of the unstimulated cells expressed IL-21. There was about a 30-fold induction of ligand expression in the CD4+ cells stimulated with anti-CD3 at 3.5h, as seen in the previous experiment (at 4h). However, there was only about a 5-fold induction with PMA + Ionomycin stimulation at 3.5h that went down at subsequent timepoints. Again, the CD8+ cells expressed a very small amount of IL-21 that was probably attributed to contaminating CD4+ cells.

The final experiment used RT-PCR to assess IL-21 receptor expression in anti-CD3- and anti-CD3/anti-CD28-stimulated and unstimulated CD4+ and CD8+ samples at timepoints of 0 h, and at 2 h, 4 h, and 16 h after stimulation. CD19+ cells activated with PMA + Ionomycin were also screened for receptor expression at the same time intervals. The resting CD4+ sample was arbitrarily chosen as the calibrator and given a value of 1.00. The 2h anti-CD3 stimulated CD4+ cells only had a 4-fold induction of receptor, compared to the 10-fold induction seen at 3.5h in the previous experiment. The combination of anti-CD3 and anti-CD28 increased IL-21 receptor expression to 8-fold above background. The 16 h anti-CD3/anti-CD28 stimulated CD8+ cells had very low IL-21 receptor expression levels, as seen in the CD8+ cells in previous experiments (above). The CD19+ cells stimulated with PMA + Ionomycin had the most significant IL-21 receptor expression with a 19-fold increase at 2h, but the expression levels decreased back to those of resting cells by 16h.

These samples from the final experiment were also used to assess IL-21 by RT-PCR. The 16h anti-CD3/anti-CD28 stimulated CD8+ sample was arbitrarily chosen as the calibrator and given a value of 1.00. The results showed that at 2 h the CD4+ cells had about a 2-fold induction of IL-21 expression with anti-CD3 stimulation and a 5-fold induction with anti-CD3 plus anti-CD28 stimulation. These stimulation conditions induced Ligand expression over time, with the 16 h stimulated CD4+ cells exhibiting Ligand expression levels 70-fold above background. CD8+ and CD19+ cells showed no IL-21 expression.

A certain amount of variation was expected between blood draws (i.e. multiple samples at different times from the same patient and between multiple patients). Therefore, data trends were analyzed within each study or from a single blood sample and the three experiments above were compared for an overall conclusion. The trend from the Real Time PCR experiments described above is that of all the cell types tested, CD19+ B cells activated with PMA + ionomycin expressed the highest levels of IL-21 receptor RNA. CD4+ and CD8+ cells can also be stimulated to express receptor, but at lower levels than in B cells. IL-21 was expressed almost exclusively in stimulated CD4+ T cells (and not by CD8+ T cells or CD19+ B cells). Although stimulation with PMA + Ionomycin induced a good IL-21 signal in this assay, a significantly higher signal was obtained from CD4+ T cells stimulated with anti-CD3 mAb or a combination of anti-CD3 and anti-CD28 mAbs, conditions that better mimic an antigen encounter *in vivo.*

Example 10

IL-21-dependent Proliferation of B-cell Cells Sstimulated Anti-CD40 or Anti-Ig-M

A. Purification of Human B cells

A vial containing 1 x $10^8$ frozen, apheresed human peripheral blood mononuclear cells (PBMCs) was quickly thawed in a 37˚C water bath and resuspended in 25 ml B cell medium (RPMI Medium 1640 (JRH Biosciences. Lenexa, KS), 10% Heat inactivated fetal bovine serum, 5% L-glutamine, 5% Pen/Strep) (Gibco BRL)) in a 50 ml tube (Falcon VWR, Seattle, WA). Cells were tested for viability using Trypan Blue (Gibco BRL). Ten milliliters of Ficoll/Hypaque Plus (Pharmacia LKB Biotechnology Inc., Piscataway, NJ) was layered under the cell suspension and spun for 30 minutes at 1800 rpm and allowed to stop with the brake off. The interface was then removed and transferred to a fresh 50 ml Falcon tube, brought up to a final volume of 40 ml with PBS and spun for 10 minutes at 1200 rpm with the brake on. The viability of the isolated cells was again tested using Trypan Blue. Alternately fresh drawn human blood was diluted 1:1 with PBS (Gibco BRL) and layered over Ficoll/Hypaque Plus (Pharmacia), spun and washed as above. Cells isolated from either fresh or frozen sources gave equivalent results.

B cells were purified from the Ficoll floated peripheral blood cells of normal human donors (above) with anti-CD19 magnetic beads (Miltenyi Biotec, Auburn, CA) following the manufacturer's instructions. The purity of the resulting preparations was monitored by flow cytometric analysis with anti-CD22 FITC Ab (Pharmingen, SanDiego, CA). B cell preparations were typically >90% pure.

B. Purification of Murine B cells

A suspension of murine splenocytes was prepared by teasing adult C57B1/6 mouse (Charles River Laboratories, Wilmington, MA) spleens apart with bent needles in B cell medium. RBCs were removed by hypotonic lysis. CD43 positive cells were removed with CD43 magnetic beads (Miltenyi Biotec) following the manufacturer's instructions. The purity of the resulting preparations was monitored by flow cytometric analysis with anti-CD45R FITC Ab (Pharmingen). B cell preparations were typically >90% pure.

C. Proliferation of anti-CD40-stimulated B-Cells in the presence of human or murine IL-21

The B cells from either the human or mouse source were resuspended at a final concentration of 1 x $10^6$ cells/ml in B cell medium and plated at 100 μl/well in a 96 well U bottom plate (Falcon, VWR) containing various stimulation conditions to bring the final volume to 200 μl/well. For anti-CD40 stimulation human cultures were supplemented with 1μg/ml anti-human CD40 (Genzyme, Cambridge, MA) and mouse cultures were supplemented with 1μg/ml anti-murine CD40 (Serotec, UK). Human or murine IL-21 was added at dilutions ranging from 1 pg/ml-100 ng/ml. The specificity of the effect of IL-21 was confirmed by inhibition of IL-21 with 25mg/ml soluble human zalpha11CEE (Example 10A). All treatments were performed in triplicate. The cells were then incubated at 37˚C in a humidified incubator for 120 hours (human) or 72 hours (mouse). Sixteen hours prior to harvesting, 1 μCi $^3$H-thymidine (Amersham, Piscataway, NJ) was added to all wells to assess whether the B-cells had proliferated. The cells were harvested into a 96 well filter plate (UniFilter GF/C, Packard, Meriden, CT) using a cell harvester (Packard) and collected according to manufacturer's instructions. The plates were dried at 55˚C for 20-30 minutes and the bottom of the wells were sealed with an opaque plate sealer. To each well was added 0.25 ml of scintillation fluid (Microscint-O, Packard) and the plate was read using a TopCount Microplate Scintillation Counter (Packard).

Incubation with IL-21 at concentrations of 3 ng/ml or more enhanced the proliferation induced by soluble anti-CD40 in a dose dependent manner in both murine and human B cells by as much as 30 fold. The murine and human B cells responded equally as well to their respective IL-21. In both species, the stimulation was specific to IL-21, as it was reversed by the presence of soluble IL-21 receptor in the culture.

D. Proliferation of anti-IgM-stimulated B-Cells in the presence of human, or murine IL-21

The B cells from either human or mouse source as described above (Example 10A and Example 10B) were plated as described above (Example 10C). For anti-IgM stimulation of human cells the plates were pre-coated overnight with 10mg/ml $F(ab')_2$ anti-human IgM Abs (Southern Biotech Associates, Birmingham, Alabama) and washed with sterile media just prior to use. The cultures were supplemented with 0-10 ng/ml hu rIL-4 (R&D Systems, Minneapolis, MN). For anti-IgM stimulation of murine cells soluble anti-IgM (Biosource, Camarillo, CA) was added to the cultures at 10 mg/ml. To each of the preceding anti-IgM/IL-4 conditions, human or murine IL-21 was added at dilutions ranging from 1 μg/ml-100 ng/ml as described above. The specificity of the effect of IL-21 was confirmed by inhibition with soluble human zalphall receptor as described above (Example 10C). All treatments were performed in triplicate. The cells were incubated, labeled with $^3$H-thymidine, harvested, and analyzed as described in Example 10C.

Incubation with IL-21 at concentrations of 0.3 ng/ml or more inhibited the proliferation induced by insoluble anti-IgM (mouse) or anti-IgM and IL-4 (human) in a dose-dependent manner. This inhibition was specific to IL-21 as it was reversed by the presence of soluble IL-21 receptor in the culture.

Example 11

Human IL-21 Effect on B-cells and IL-21 Toxic Saporin Fusion

The effects of human IL-21 were tested on the following human B-cell lines: and human Burkitt's lymphoma cell lines Raji (ATCC No.CCL-86), and Ramos (ATCC No. CRL-1596); human EBV B-cell lymphoma cell line RPMI 1788 (ATCC No. CRL-156); human myeloma/plasmaeytoma cell line IM-9 (ATCC No. CRL159); and human EBV transformed B-cell line DAKIKI (ATCC No. TIB-206), and HS Sultan cells (ATCC No. CRL-1484 ). Following about 2-5 days treatment with IL-21 , changes in surface marker expression were found in IM-9, Raji, Ramos, and RPMI1788 cell lines, showing that these cells can respond to IL-21. Human B-cell lines treated with IL-21 grew much more slowly than untreated cells when re-plated in cell culture dishes. These cells also had an increased expression of FAS ligand, as assessed by flow cytometry (Example 11D and Example 11E), and moderately increased sensitivity to an activating FAS antibody (Example 11A). This results indicate that IL-21 could control some types of B-cell neoplasms by inducing them to differentiate to a less proliferative and or more FAS ligand sensitive state. Moreover, zalpha11 receptor is expressed on the surface of several of these cell lines (U.S. Patent No. 6,307,024). Thus, IL-21 and the human IL-21-saporin immunotoxin conjugate (Example 11B, below), or other IL-21-toxin fusion could be therapeutically used in B-cell leukemias and lymphomas.

A. The effect of human IL-21 on B-cell lines.

IM-9 cells were seeded at about 50,000 cells per ml +/- 50 μg/ml purified human IL-21 (U.S. Patent No. 6,307,024). After 3 days growth the cells were harvested, washed and counted then re-plated at about 2500 cells/ml in 96 well plates in to wells with 0, 0.033, 0.1 or 0.33 μg/ml anti-FAS antibody (R&D Systems, Minneapolis). After 2 days an Alamar blue fluorescence assay was performed (U.S. Patent No. 6,307,024) to assess proliferation of the cells.

IL-21-treated IM-9 cells grew to only 27% the density of the untreated cells in the absence of anti-FAS antibody. In the presence of 0.33 μg/ml anti-FAS antibody, the IL-21-treated cells were inhibited an additional 52% while the untreated cells were inhibited by only 30%. The overall inhibition of cell growth with both IL-21 and 0.33 μg/ml anti-FAS antibody treatment was 86%.

When the IM-9 cells were pretreated for three days with or without IL-21 and then re-plated at 100 cells per well and grown with or without anti-FAS antibody for 6 days, the growth of untreated cells assessed by Alamar Blue assay (U.S. Patent No. 6,307,024) was inhibited only 25% by anti-FAS antibody while the growth of IL-21-treated cells was inhibited 95% relative to the growth of untreated cells in zero anti-FAS antibody.

B. The effect of human IL-21-saporin immunotoxin on B-cell lines.

The human IL-21-saporin immunotoxin conjugate (zalphallL-sap) construction and purification is described in Example 12. The human zaipha11L-sap was far more potent than the saporin alone in inhibiting cell growth. When the treated cell are re-plated after a three or four day treatment the human zalpha11L-sap treated cells grew very poorly.

IM-9, Ramos and K562 (ATCC No. CCL-243) cells were seeded at about 2500 cells/well in 96 well plates with zero to 250 ng/ml human zalphallL-sap conjugate or 0-250 ng/ml saporin (Stirpe et al., Biotechnology 10,:405-412, 1992) only as a control. The plates were incubated 4 days then an Alamar Blue proliferation assay was performed (U.S. Patent No. 6,307,024). At the maximal concentration of human zaipha11-sap conjugate, the growth of IM-9 cells and RAMOS cells was inhibited by 79% and 65% respectively. K562 cells which are low/negative by flow for expression of the IL-21 receptor were not affected by the zalpha11-sap, thus showing the specificity of the conjugate's effect.

IM-9 cells were seeded a 50,000 cells/ml into 6 well plates at zero and 50 ng/ml human zaipha11L-sap conjugate. After 3 days the cells were harvested and counted then re-plated from 100 to 0.8 cells per well in 2 fold serial dilutions, and 12 wells per cell dilution without the human IL-21-saporin immunotoxin. After 6 days the number of wells with growth at each cell dilution was scored according to the results of an Alamar blue proliferation assay (U.S. Patent No. 6,307,024).

When cell number was assessed, by Alamar blue assay (U.S. Patent No. 6,307,024), after 6 days of growth control cells seeded at about 12.5 and 6.25 cells per well had equivalent growth to zalpha11-sap treated cells seeded at 100 and 50 cells/well respectively. Thus, the growth of the surviving treated IM-9 cells was markedly impaired even after the removal, by re-plating, of the zalpha11-sap immunotoxin.

The limited tissue distribution of the human IL-21 receptor (U.S. Patent No. 6,307,024 and WIPO Publication No.s WO 0/17235 and WO 01/7717), and the specificity of action of the zalpha11-sap to receptor-expressing cell lines suggest that this conjugate may be tolerated *in vivo.*

C. The effect of human IL-21-saporin immunotoxin on B-cell line viabilily.

HS Sultan cells (ATCC No. CRL-1484 ) were seeded at about 40,000 cells per ml into 12 well plates and grown for five days with either no added cytokines or 4 0 ng/ml purified human IL-21 (U.S. Patent No. 6,307,024) or 25 ng/ml human zalpha11L-sap conjugate (Example 12, below) or with 20 ng/ml IFN-alpha (RDI) or IL-21 and IFN-alpha. IL-21 inhibited the outgrowth of Hs Sultan cells by 63%. IFN-alpha inhibited the growth by 38%. IL-21 plus IFN-alpha inhibited growth 78%, indicating that the growth inhibitory effects of human IL-21 and IFN-alpha may be additive. The human zalpha11L-sap inhibited growth of the HS Sultans by 92%.

The results above support the possible use of IL-21 or human zalpha11L-sap in the treatment of malignancies or other diseases that express the zalpha11 preceptor, particularly those of B-cell origin. The combination of IL-21 with IFN-alpha is specifically suggested by their additive effect in the inhibition of HS Sultan cells. Some other types of lymphoid malignancies and diseases may also express the IL-21 receptor, as activated T-cells also express the receptor mRNA (U.S. Patent No. 6,307,024 and WIPO Publication No.s WO 0/17235 and WO 01/7717) and some of these diseases may also be responsive to IL-21 of IL-21-toxic fusion therapy.

D. FAS (CD95) Expression on Human B-cell Lines is Increased by human IL-21 Stimulation

Human B-cell lines HS Sultan (ATCC No. CRL-1484), IM-9 (ATCC No. CRL159), RPMI 8226 (ATCC No. CCL-155), RAMOS (ATCC No. CRL-1596), DAKIKI (ATCC No. TIB-206), and RPMI 1788 (ATCC No. CRL-156), were all treated with or without purified 10 to 50 ng/ml human IL-21 (U.S. Patent No. 6,307,024) for 2 to 8 days. The cells were then stained with anti-CD95 PE-conjugated antibody (PharMingen, San Diego, CA), per manufacturer's protocol, and analyzed on a FACScalibur (Becton Dickinson, San Jose, CA). In all cell lines, anti-CD95 (FAS or APO-1) staining was increased, in some cases more than two fold, upon treatment with human IL-21.

E. FAS (CD95) Expression on Primary Mouse Spleen B-cells is Increased by Human IL-21 Stimulation

Primary mouse splenocytes were obtained by chopping up spleens from 8 to 12 week old C57/BL6 mice. Erythrocytes were lysed by treating the preparation for 5 seconds with water then put through a 70 micron sieve. The remaining splenocytes were washed and plated in RPMI (JRH Bioscience) plus 10% HIA-FBS (Hyclone, Logan, UT). IL-2 (R & D Systems) with or without human IL-21, as described above. They were then incubated at 37˚C, in 5% $CO_2$ for 5 days. The splenocytes were harvested and stained with anti-CD95 PE conjugated antibody (PharMingen) and anti-CD19 FITC conjugated antibody (PharMingen) per manufacturer's protocol. The cells were analyzed by flow cytometry on a FACScalibur (Becton Dickinson). Upon gating on the CD19+ mouse B-cells, it was found that anti-CD95 staining was increased on B-cells treated with IL-2 plus human IL-21 compared to those in IL-2 alone. The anti-CD95 staining was 37 relative fluorescent units (RFU) on the B-cells in IL-2 alone and 55 RFU on the B-cells cultured in IL-2 and human IL-21

Example 12

Construction and Purification of IL-21 Toxic Fusion

Under a supply contract, 10 mg human IL-21 (U.S. Patent No. 6,307,024) was sent to Advanced Targeting Systems (ATS, SanDiego, CA) for conjugation to the plant toxin saporin (Stirpe et al., Biotechnology 10,:405-412, 1992). ZymoGenetics received from ATS 1.3 mg of a protein conjugate comprised of 1.1 molecules saporin per molecule of human IL-21, formulated at a concentration of 1.14 mg/ml in 20 nM Sodium phosphate, 300 nM sodium cloride, pH 7.2.

Example 13

IL-21 Toxic Fusion *in vivo*

A. Testing IL-21-saporin conjugate in mice

IL-21-saporin conjugate (Example 11) was administered to C57BL6 mice (female, 12 weeks of age, purchased from Taconic) at two different dosages: 0.5 and 0.05 mg/kg. Injections were given i.v. in vehicle consisting of 0.1% BSA (ICN, Costa Mesa, CA). Three injections were given over a period of one week (day 0, 2, and 7). Blood samples were taken from the mice on day 0 (pre-injection) and on days 2 and 8 (post-injection). Blood was collected into heparinized tubes (Bectin Dickenson, Franklin Lakes, NJ), and cell counts were determined using an automated hematology analyzer (Abbot Cell-Dyn model No. CD-3500CS, Abbot Park, IL). Animals were euthanized and necropsied on day 8 following blood collection. Spleen, thymus, liver, kidney and bone marrow were collected for histopathology. Spleen and thymus were weighed, and and additional blood sample was collected in serum separator tubes. Serum was sent to Pheonix Central Labs, Everett, WA, for testing in a standard chemistry panel. Samples were also collected for flow cytometric analysis as described herein.

Circulating blood cell counts and serum chemistry measurements did not differ significantly between IL-21 conjugate treated mice and mice treated with an equivalent dose of unconjugated toxin (saporin). Histological analysis of tissues in IL-21-saporin treated mice showed no significant changes relative to mice treated with an equivalent dose of unconjugated toxin. These results indicated that the saporin conjugate was not toxic *in vivo.*

B. Testing IL-21 toxic saporin fusion on B-cell derived tumors *in vivo*

The effects of human IL-21 and the human IL-21 toxic saporin fusion (Example 12) on human tumor cells are tested *in vivo* using a mouse tumor xenograft model described herein. The xenograft models are initially tested using cell lines selected on the basis of *in vitro* experiments, such as those described in Example 11. These cell lines include, but are not limited to: human Burkitt's lymphoma cell lines Raji (ATCC No.CCL-86), and Ramos (ATCC No. CRL-1596); human cell line RPMI 1788 (ATCC No. CRL-156); human myeloma/plasmacytoma cell line IM-9 (ATCC No. CRL159); human cell line DAKIKI (ATCC No. TIB-206), and HS Sultan cells (ATCC No. CRL-1484). Cells derived directly from human tumors can also be used in this type of model. In this way, screening of patient samples for sensitivity to treatment with IL-21 or with a IL-21 toxic saporin fusion can be used to select optimal indications for use of zalphal 1 in anti-cancer therapy.

After selection of the appropriate zenograft *in vivo* model, described above, IL-21-induced activity of natural killer cells and/or IL-21 effects on B-cell derived tumors are assessed *in vivo.* Human IL-21 is tested for its ability to generate cytotoxic effector cells (e.g. NK cells) with activity against B-cell derived tumors using mouse tumor xenograft models described herein. Moreover, direct affects of human IL-21 on tumors can be assessed. The xenograft models to be carried out are selected as described above. A protocol using IL-21 stimulated human cells is developed and tested for efficacy in depleting tumor cells and promoting survival in mice innoculated with cell lines or primary tumors.

Example 14

Preliminary Evaluation of the Aqueous Stability of Human IL-21

Preliminary studies were conducted to evaluate the aqueous stability characteristics of human IL-21 in support of bioprocessing, formulation, and *in vivo* administration. The objectives were to: 1) verify the stability and recovery from Alzet Minipumps & general storage and handling, 2) determine the stability-indicating nature of several analytical methods including cation-exchange HPLC (CX-HPLC), reverse-phase HPLC (RP-HPLC), size exclusion HPLC (SEC-HPLC), & bioassay (BaF3/zalpha11R proliferation (e.g., U.S. Patent No. 6,307,024), and 3) determine the stability-limiting degradation pathways and their kinetic dependencies.

Aliquots of purified human IL-21 (U.S. Patent No. 6,307,024) were prepared by dilution to 2 mg/mL in PBS (pH 7.4) and stored in low density polyethylene (LDPE) cryovials (Nalgene, 1.8 mL) at -80˚C (control), 5˚C, 30˚C, and 37˚C. Samples were assayed intermittently over 29 days by CX-, RP-, SEC-HPLC, and bioassay. Aliquots were also stored at -80˚C and subjected to freeze-thaw (f/t) cycling (-80˚C/RT; 5X f/t, 10X f/t). Recovery of human IL-21 was determined relative to the -80˚C control (1 f/t) in all assays.

The remaining human IL-21 solution from the -80˚C control samples were refrozen (-80˚C) after analysis. This aliquot (2 f/t) was used to evaluate the thermal and conformational stability of human IL-21 as a function of pH using circular dichroism (CD). The 2 mg/mL solution was diluted to 100 μg/mL in PBS buffers ranging from pH 3.3-8.8. The far-UV CD spectra was monitored over the temperature range 5-90°C in 5°C intervals (n=3/pH). The CD spectropolarimeter used was a Jasco 715 (Jasco, Easton, MD). The thermal unfolding was monitored by changes in ellipticity at 222 nm as a function of temperature. Estimates of the $T_m$ were estimated assuming a two-state unfolding model. The data was fit (sigmoidal) using SlideWrite Plus for Windows v4.1 (Advanced Graphics Software; Encinitas, CA).

Recovery and stability from Alzet Minipumps (Model No. 1007D; ALZA Corporation, Mountain View, CA) was assessed by filling pumps with 100 μL of the 2 mg/mL human IL-21 solution, placing the pumps in 1.8 mL LDPE containing 1 mL of PBS (pH 7.4), and storing them at 37°C. The release/recovery of human IL-21 from the minipumps was assessed by CX-, RP-, and SEC-HPLC on days 2, 4, and 7. The activity was assessed by bioassay on day 7. The study was designed to evaluate the release from 3 pumps per sampling time.

The chromatographic data suggested that the CX- & SEC-HPLC methods were stability-indicating, whereas the RP-HPLC method was not. At least 3 additional peaks indicating apparent degradation products were observed by CX-HPLC. The SEC-HPLC method resolved an apparent human IL-21 aggregate, eluting prior to human IL-21. However, no significant additional peaks were observed eluting after the human IL-21 peak. This suggests that the degradation products observed by CX-HPLC most probably result from amino acid modifications such as deamidation, rather than hydrolysis/proteolysis processes leading to clipped variants. A small degree of fronting/tailing was observed by RP-HPLC (relative to control) in samples which had been shown to have undergone significant degradation by SEC- & CX-HPLC. However, apparent degradation products were not resolved by RP-HPLC. The degradation observed by CX-HPLC increased as a function of time-temperature, and followed apparent first-order kinetics. The % human IL-21 recovered by CX-HPLC after 29 days at 37°C, 30°C, and 5°C was 39%, 63%, and 98%, respectively. Aggregation also increased in a time-temperature dependent fashion. The % aggregate found in preparations stored for 29 days at 37°C, 30°C, and 5°C was 7.4, 3.4, and below detectable limits (BDL), respectively. No significant differences were observed by bioassay in any sample, suggesting the degradation products have equivalent activity to intact human IL-21. No degradation was observed by any assay in samples subjected to up to 10 f/t cycles.

The release of human IL-21 from Alzet Minipumps was consistent with the theoretical expected volume release. This suggests that significant surface adsorption would not impair the delivery of human IL-21 using the Alzet Minipumps with a 2 mg/mL fill concentration. The degradation consistent with that previously noted was observed. The % purity determined by CX-HPLC of human IL-21 released after 2, 4, and 7 days was 96%, 90%, and 79%, repectively. It should be recognized that degradation also occurs after human IL-21 is released into or diluted with release medium. Therefore, the % purity within the minipump may be somewhat different than that determined to be in the release medium. The bioactivity of each sample was consistent with the expected amount of human IL-21 released from the minipumps.

The human IL-21 far-UV CD spectra, as expected, was consistent with interleukins, such as IL-3 (J. Biochem., 23:352-360, 1991), IL-4 (Biochemistry, 30:1259-1264, 1991), and IL-6 mutants (Biochemistry, 35:11503-11511, 1996). Gross changes in the far-uv CD spectra as a function of pH were not observed. Results showed that the pH of maximum thermal/conformational stability was ≈ pH 7.4. Analysis of the unfolding curves were based on a two-state unfolding mechanism to allow comparison of the thermal/conformational stability as a function of pH/composition. However, one or more intermediates may exist during the unfolding process since the cooperativity was relatively low, based on the shallowness of the unfolding curve. Although studies were not specifically designed to determine whether human IL-21 refolds following thermal unfolding to 90°C, preliminary data suggests that at least partial refolding occurs after the temperature of the sample is cooled back to 20°C.

These studies allow an analytical paradigm to be identified to evaluate the purity and verify the stability of human IL-21. For instance, SEC-HPLC can be used to characterize the extent and rate of aggregation in aqueous solution. Likewise, CX-HPLC can be used to characterize the extent and rate of degradation of human IL-21 by mechanisms other than aggregation. The bioassay can be used to verify activity of human IL-21 and it's aqueous degradation products. For instance, the human IL-21 variants generated in aqueous solution & resolved by CX-HPLC may themselves be useful as therapeutic agents, since they have equivalent bioactivity. Also, the fact that human IL-21 degrades by several different processes (aggregation, amino acid modifications) suggests a preferred or unique formulation which minimizes the rate of each degradation process may be necessary for long-term stability of a solution product.

Identification of the nature of the aqueous degradation products and determination of their kinetic dependencies (pH, concentration, excipients) is underway. Human IL-21 stability in serum/plasma is determined to support the design and interpretation of *in vivo* studies.

Example 15

IL-21 Effect on B-cell Derived Ttumors *in vivo*

A. Infusion of IL-21 using mini-osmotic pumps

Administration of IL-21 by constant infusion via mini-osmotic pumps resulted in steady state serum concentrations proportional to the concentration of the IL-21 contained in the pump. 0.22 ml of human IL-21 (U.S. Patent No. 6,307,024) contained in phosphate buffered saline (pH 6.0) at a concentration of 2 mg/ml or 0.2 mg/ml was loaded under sterile conditions into Alzet mini-osmotic pumps (model 2004; Alza corporation Palo Alto, CA). Pumps were implanted subcutaneously in mice through a 1 cm incision in the dorsal skin, and the skin was closed with sterile wound closures. These pumps are designed to deliver their contents at a rate of 0.25 μl per hour over a period of 28 days. This method of administration resulted in significant increase in survival in mice injected with tumor cells (below).

B. IL-21 effect on B-cell derived tumors *in vivo*

The effects of human IL-21 (U.S. Patent No. 6,307,024) were tested *in vivo* using a mouse tumor xenograft model described herein. The xenograft models tested were human lymphoblastoid cell line IM-9 (ATCC No. CRL159). C.B-17 SCID mice (female C.B-17/IcrHsd-scid; Harlan, Indianapolis, Indiana) were divided into 4 groups. On day 0, IM-9 cells (ATCC No. CRL159) were harvested from culture and injected intravenously, via the tail vein, to all mice (about 1,000,000 cells per mouse). On day 1, mini-osmotic pumps containing test article or control article were implanted subcutaneously in the mice. Mice in groups 1-3 (n=9 per group) were treated with increasing concentrations of IL-21: group 1 contained 2.0 mg/mL of human IL-21 and delivered 12 μg per day; group 2 contained 0.20 mg/mL of human IL-21 and delivered 1.2 μg per day; group 3 contained 0.02 mg/mL of human IL-21 and delivered .12 μg per day. Mice in group 4 (n = 9) were a control and were treated with vehicle (PBS pH 6.0).

Mice treated with either 12 μg/day or 1.2 μg/day IL-21 infusion had increased survival compared to vehicle treated mice (p<.0001 and p<.005 for 12 μg/day or 1.2 μg/day vs. vehicle, respectively, using log rank tests of the survival function). Mice in the .12 μg/day dose group had survival no different than the mice in the vehicle treated group. These results showed that IL-21 significantly reduced the effects of the B-cell tumor cells *in vivo,* significantly resulting in increased survival.

Example 16

*In vivo* Anti-tumor Effects of IL-21 in B16-F10 Melanoma and EG.7 Thymoma Models

A. Murine IL-21 effect on B16-F10 melanoma metastasis growth *in* vivo

Mice (female, C57B16, 9 weeks old; Charles River Labs, Kingston, NY) were divided into three groups. On day 0, B16-F10 melanoma cells (ATCC No. CRL-6475) were harvested from culture and injected intravenously, via the tail vein, to all mice (about 100,000 cells per mouse). Mice were then treated with the test article or associated vehicle by intraperitoneal injection of 0.1 ml of the indicated solution. Mice in the first group (n = 24) were treated with vehicle (PBS pH 6.0), which was injected on day 0, 2, 4, 6, and 8. Mice in the second group (n = 24) were treated with murine IL-21 (U.S. Patent No. 6,307,024), which was injected at a dose of 75 μg on day 0, 2, 4, 6, and 8. Mice in the third group (n = 12) were treated with murine IL-21, which was injected at a dose of 75 μg daily from day 0 through day 9. All of the mice were sacrificed on day 18, and lungs were collected for quantitation of tumor. Foci of tumor growth greater than 0.5 mm in diameter were counted on all surfaces of each lung lobe. In both groups of mice treated with murine IL-21, the average number of tumor foci present on lungs was significantly reduced, compared to mice treated with vehicle. Mice treated more frequently (i.e. daily) had fewer tumor foci than mice treated on alternate days, although this was not a statistically significant finding between these two groups.

These results indicated that treatment with murine IL-21 either slowed the growth of the B16 melanoma tumors or enhanced the ability of the immune system to destroy the tumor cells. The effects of the treatment on tumor cells were likely mediated through cells of the immune system (i.e. lymphocytes, NK cells), which do possess receptors for IL-21, such as, for example, IL-21 receptor and zalphal 1/IL-2Rγ (WIPO Publication No.s WO 0/17235 and WO 01/7717) and are known to be associated with anti-tumor activity.

B. Murine IL-21 effect on EG.7 thymoma growth in vivo

Mice (female, C57B16, 9 weeks old; Charles River Labs, Kingston, NY) were divided into three groups. On day 0, EG.7 cells (ATCC No. CRL-2113) were harvested from culture and 1, 000, 000 cells were injected intraperitoneal in all mice. Mice were then treated with the test article or associated vehicle by intraperitoneal injection of 0.1 mL of the indicated solution. Mice in the first group (n = 6) were treated with vehicle (PBS pH 6.0), which was injected on day 0, 2, 4, and 6. Mice in the second group (n = 6) were treated with murine IL-21 (U.S. Patent No. 6,307,024), which was injected at a dose of 10 μg on day 0, 2, 4, and 6. Mice in the third group (n = 6) were treated with murine IL-21, which was injected at a dose of 75 μg on day 0, 2, 4, and 6. In both groups of mice treated with murine IL-21, time of survival was significantly increased, compared to mice treated with vehicle. The group treated with 75 μg doses of IL-21 had significantly greater survival than the group treated with 10 μg doses, and 33% (2/6 mice) of this group survived longer than 70 days. An additional portion of this study tested the effect of the same dosages carried out through day 12. The results were very similar to the shorter dosing schedule, with both doses having significantly increased survival over vehicle treatment, and the highest dose gave the best response (50% survival past 70 days).

In some experiments about 4,000,000 OT-I T cells were injected intraperitoneally in the mice on the day prior to day 0. The mice were then treated with IL-21 or vehicle as above. The presence of the OT-I T cells had no effect on the survival time of the vehicle treated mice. In mice treated with IL-21 the presence of the OT-I T cells enhanced survival time compared to the mice treated with IL-21 alone.

These results indicated that treatment with murine IL-21 either slowed the growth of the EG.7 tumors or enhanced the ability of the immune system to destroy the tumor cells. The increase in survival conferred by the OT-I T cells in the presence of IL-21 treatment suggests that IL-21 is activating effector cells of the immune system.

## Example 17

## IL-21 Effects on Serum Cytokines and Vascular Leak

### A. Analysis of IL-21 on Serum Cytokines

IL-2 therapy is effective in the treatment of certain cancers. However, the use of IL-2 as a therapeutic agent has been limited by its toxic effects, namely vascular leak syndrome (VLS). IL-2 induced VLS is characterized by infiltration of lymphocytes, monocytes and neutrophils into the lung causing endothelial damage in the lung eventually leading to vascular leak (reviewed in Lentsch AB et al, Cancer Immunol. Immunother., 47:243, 1999). VLS in mice can be induced with administration of repeated high doses of IL-2 and measuring vascular leak by Evan's Blue uptake by the lung. Other parameters that have been shown to be characteristic of VLS in mice include increased serum levels of TNFα and IFNγ (Anderson JA et al, J. Clin. Invest. 97:1952, 1996) as well as increased numbers of activated T, NK and monocytes in various organs. Blocking of TNFα with a soluble TNFR-Fc molecule inhibited lung infiltration by lymphocytes and therefore lung injury (Dubinett SM et al, Cell. Immunol. 157:170,1994). The aim was to compare the ability of IL-2 and IL-21 to induce VLS in mice and to measure the different parameters indicative of VLS (Evan's Blue uptake, serum cytokine analysis, spleen cellular phenotype).

Mice (female, C57B16, 11 week old; Charles River Labs, Kingston, NY) were divided into five groups. All groups contained 10 mice per group. Groups are as follows: Group I or Vehicle group received Phosphate Buffered Saline (PBS); Group II and III received IL-2 0.6 or 1.8 million IU/injection respectively; Group IV and V received mouse IL-21 (U.S. Patent No. 6,307,024) or 100 μg/injection respectively. The study consisted of 4 days, body weight was measured daily and animals received 7 intraperitoneal injection of test substance over the 4-day period. Animals received two daily injections on day 1-3 and on the fourth day received a single morning injection. Two hours post final injection animals received a tail vein injection of 1% Evan's blue (0.2 ml). Two hours post Evan's blue injection mice where anesthetized with Isoflurane and blood was drawn was serum cytokine analysis. Following blood draw animals where transcardial perfused with heparinized saline (25 U hep/ml saline). Following perfusion spleen was removed and weighed, liver and lung where removed and placed into 10 mls of formamide for 24 hr incubation at room temperature. Following 24 hr incubation vascular leakage was quantitated by Evan's blue extravasation via measurement of the absorbance of the supernatant at 650 nm using a spectrophotometer.

Mice were bled and serum separated using a standard serum separator tube. 25μl of sera from each animal was used in a Becton Dickenson (BD) Cytokine Bead Array (Mouse Th1/Th2 CBA Kit) assay. The assay was done as per the manufacturer's protocol. Briefly, 25 μl of serum was incubated with 25 μl bead mix (IL-2, IL-4, IL-5, TNFα and IFNγ) and 25 μl PE-detection reagent for two hours at room temperature in the dark. A set of cytokine standards at dilutions ranging from 0-5000 pg/ml was also set up with beads as per the manufacturer's instructions. The incubated beads were washed once in wash buffer and data acquired using a BD FACScan as per instructions outlined in the Kit. The data was analyzed using the BD Cytometric Bead Array Software (BD Biosciences, San Diego, CA).

Serum cytokine analysis using the CBA cytokine kit (Becton Dickenson, San Diego, CA) showed no increases in levels of IL-2, IL-4, IL-5, IFNγ or TNFα in the PBS control treated groups. There was a dose dependent increase in the levels of IL-5, IFNγ and TNFα in sera from IL-2 treated mice. There was no increase in the levels of the 5 measured cytokines in the serum of mice treated with IL-21. The cytokine levels in the highest dose of IL-21 mirrored that of the PBS treated animals. This shows that unlike IL-2 treatment that leads to increase in serum levels of the inflammatory cytokines IL-5, TNFα and IFNγ, treatment with IL-21 does not have any effect on these inflammatory cytokines.

The results from a representative experiment is given in Table 6. All concentrations are expressed in pg/ml were an average of 4 animals/group.

Table 6

| | TNFα | IFNγ | IL5 | IL4 | IL2 |
|---|---|---|---|---|---|
| PBS | 2.4 | 0.0 | 2.9 | 2.2 | 1.3 |
| IL2 0.6 millU | 22.9 | 21.6 | 1095.0 | 2.5 | 2.0 |
| IL2 1.8 millU | 69.1 | 185.1 | 1132.9 | 2.0 | 1.9 |
| IL2 3.6 millU | 78.9 | 195.6 | 651.3 | 1.8 | 2.1 |
| IL-21 3μg | 2.1 | 1.6 | 2.7 | 1.7 | 1.6 |
| IL-21 100μg | 3.1 | 0.0 | 4.0 | 0.0 | 1.1 |
| IL-21200μg | 7.3 | 1.9 | 4.0 | 2.6 | 1.9 |

As shown in Table 5 above, treatment of mice with IL-2 resulted in a dramatic increase in serum inflammatory cytokines, namely IL-5, IFNγ and TNα. Treatment of mice with IL-21 did not show any increase in cytokine levels above PBS treated mice. These results show that even at the highest doses, IL-21 does not upregulate inflammatory cytokines and it's effect on cells in vivo is different from IL-2.

Treatment of mice with repeated high dose IL-2 resulted in increased serum levels of IL-5, IFNγ and TNFα. These pro-inflammatory cytokines have been shown to play a role in VLS associated with IL-2 toxicity. Blocking TNFa resulted in decreased lymphocyte infiltration into the lungs and decreased lung injury associated with IL-2 toxicity (Dubinett SM et al, 1994, Cell. Immunol. 157:170). IL-21 treatment did not have any effects on serum IL-5, TNFα or IFNγ levels. This suggests that IL-21 acts different from IL-2 in vivo and that the lack of pro-inflammatory cytokines in sera of IL-21 treated mice might indicate lesser toxicity of IL-21 compared to IL-2.

B. Analysis of IL-21 on vascular leak - immunophenotyping of splenic cells

IL-2 induced vascular leak syndrome (VLS) involves organ damage that occurs at the level of postcapillary endothelium. However, this damage occurs secondary to two distinct pathological processes: the development of VLS, and transendothelial migration of lymphocytes. Acute organ injury is mediated by infiltrating neutrophils while chronic organ injury is mediated by infiltration monocytes and lymphocytes (reviewed in Lentsch AB et al, supra.). In mice, depletion of cells with surface phenotypes characteristic of LAK or NK cells ameliorates organ damage (Anderson TD et al, Lab. Invest. 59:598, 1988; Gately, MK et al. J. Immunol., 141:189, 1988). Increased numbers of NK cells and monocytes is therefore a marker for IL-2 mediated cellular effects of VLS. In addition, IL-2 directly upregulates the expression of adhesion molecules (i.e LFA-1, VLA-4 and ICAM-1) on lymphocytes and monocytes (Anderson JA et al, supra.). This increase is thought to enable cells to bind activated endothelial cells and help in transmigration of cells to the tissue. Increased expression of these molecules is considered another marker of IL-2 induced cellular activation during VLS. The aim of this study was to study splenic cells from IL-2 and IL-21 treated mice under a VLS protocol and compare the effects of the two cytokines to mediate cellular effects associated with VLS.

Groups of age and sex matched C57BL/6 mice treated and described above (Example 17A) were analysed. On d4, mice were sacrificed and phenotype of splenic cell populations studied by standard flow cytometry. Splenic weight and cellularity were dramatically increased in IL-2 treated mice compared to PBS treated mice. IL-21 treated mice had a slight increase in splenic weights (at the higher doses) but no significant increase in splenic cellularity compared to the PBS treated groups. Cell population analysis showed a significant increase in the percentage and numbers of NK, NKT and monocytes in IL-2 treated mice but not in the IL-21 treated mice. Furthermore, there was a dose dependent dramatic increase in LFA-1 expressing cells in the IL-2 treated groups compared to PBS controls. IL-21 treatment had no effect on LFA-1 expression on splenic cells.

Spleens were isolated from mice from the various groups. Red blood cells were lysed by incubating cells for 4 minutes in ACK lysis buffer (0.15M NH4Cl, 1mM KHCO3, 0.1mM EDTA) followed by neutralization in RPMI-10 media (RPMI with 10% FBS). The expression of cell surface markers was analyzed by standard three color flow cytometry. All antibodies were obtained from BD Pharmingen (San Diego, CA). Fluorescin- isothiocyanate (FITC) conjugated CD11a (LFA-1), CD49d (VLA-4, a chain), Gr-I FITC, phycoerythrin (PE) conjugated CD4, NK1.1, CD11b and CyC-conjugated CD8, CD3 and B220 were used to stain cells. 1-3 x $10^6$ cells were used for individual stains. Non-specific binding was blocked by incubating cells in blocking buffer (PBS, 10% FBS, 20ug/ml 2.4G2). After blocking, cells were incubated with primary antibodies for 20 minutes. Unless specified otherwise, all mAbs were used at lug/stain in a volume of 100ul. Cells were washed once in 1X PBS and resuspended in PBS before being acquired using the FACScan or FACSCalibur instruments (BD Biosciences, San Diego, CA). Data was analyzed using the Cellquest Software (BD Biosciences).

IL-2 treated mice had significantly increased spleen weights compared to PBS treated groups (Table 7, below) IL-21 treated mice had significant increase in spleen weight over controls. However, the increases in IL-21 treated groups were significantly less than in the IL-2 treated groups (p=0.0002). The increase in spleen weights in both groups was dose dependent. Table 7 below shows the treatment groups; the average splenic weights were shown in mg, and n=4.

Table 7

| | Total spleen weight (mg) | Stdev | p value (vs PBS) |
|---|---|---|---|
| PBS | 63.5 | 9.7 | 0 |
| IL-2 (0.6) | 177.5 | 17.8 | <0.0001 |
| IL-2(1.8) | 204.25 | 10 | <0.0001 |
| IL-2(3.6) | 231.2 | 9.6 | <0.0001 |
| IL-21 (33) | 92.8 | 6 | 0.0022 |
| IL-21(100) | 117.6 | 19.3 | 0.0024 |
| IL-21 (200) | 125.85 | 33 | 0.0111 |

Average Splenic cellularity data is shown in Table 8, below (n=4). Higher dose IL-2 treatment increased splenic cellularity significantly over control PBS treated groups. IL-21 treated groups did not show significant increase in splenic cellularity compared to PBS groups.

Table 8

| | Total cells (x$10^6$) | Stdev | p value (vs PBS) |
|---|---|---|---|
| PBS | 48 | 16.4 | o |
| IL-2(0.6) | 57.1 | 11.8 | 0.4014 |
| IL-2 (1.8) | 100.4 | 21.6 | <.0083 |
| IL-2 (3.6) | 101.8 | 4.25 | <.0007 |
| IL-21 (33) | 58.8 | 13.5 | 0.3463 |
| IL-21 (100) | 48 | 7.83 | 0.9769 |
| IL-21 (200) | 53.8 | 22.5 | 0.6917 |

IL-2 induced VLS is characterized by increased numbers of NK cells, monocytes and cells expressing the adhesion marker LFA-1 (reviewed in Lentsch AB et al, supra.). The above data using IL-2 reproduces published reports on the increase of NK cells, monocytes and LFA-1+ cells. IL-2 treated mice show all signs of VLS compared to controls. In contrast, IL-21 treated mice, although having an increased uptake of Evan's Blue, show no increase in serum pro-inflammatory cytokines, or increase in LFA-1+ cells or NK cells. Furthermore, although IL-21 treated mice do show increased numbers of monocytes, the increase is less than what is seen with IL-2 treated animals, suggesting that IL-2 mediated effects are more severe than IL-21 mediated effects. Taking together the splenic cellularity data and the serum cytokine data, IL-21 does not induce a comparable inflammatory response as IL-2. All parameters analyzed would indicate that IL-21 induces minor if any inflammatory response when administered in a VLS protocol in similar doses to IL-2 (weight/weight).

In addition, as shown in Table 9 and Table 10, below, flow cytometry analysis of spleen cells from mice revealed that IL-2 treated mice had a dose dependent increase in the % and numbers of splenic NK/T cells (NK1.1+CD3+), NK cells (NK1.1+CD3-), macrophages (CD11b+) and LFA-1+ cells (TABLE III and IV). IL-21 treated mice had no increase in NK/T cells, NK cells or CFA-1+ cells. There was an increase in the % and numbers of macrophages and granulocytes (data not shown) in IL-21 treated group compared to the control PBS treated groups. This increase was similar or less than the increase in IL-2 treated mice.

Table 9: Average % of lineage cells in spleen (n=4)

| | % NK/T | % NK | % macrophages | % B | % CD4 T | % CD8 T | % LFA-1+ |
|---|---|---|---|---|---|---|---|
| PBS | 0.7225 | 3.1925 | 6.625 | 50.025 | 21.975 | 14.275 | 11.1775 |
| IL-2 (0.6) | 4.34 | 9.3375 | 12.525 | 43.9 | 17.65 | 10.15 | 29.26 |
| IL-2(1.8) | 3.2 | 13.9 | 14.525 | 43.75 | 15.55 | 11.55 | 34.825 |
| IL-2 (3.6) | 3.075 | 14.3 | 11.875 | 42.8 | 14.875 | 17.325 | 44.05 |
| IL-21 (33) | 0.615 | 2.9875 | 7.825 | 53.65 | 17.925 | 10.95 | 8.4375 |
| IL-21 (100) | 0.63 | 2.76 | 11.375 | 48.325 | 17.825 | 11.275 | 13.35 |
| IL-21 (200) | 1.025 | 3.4325 | 16.175 | 45.125 | 17.225 | 12.15 | 15.7 |

Table 10: Splenic cell numbers (x $10^6$ cells, n=4)

| | NKT | NK | CD11b | B220 | Cd4 | Cd8 | LFA-1 | Gr-1 |
|---|---|---|---|---|---|---|---|---|
| PBS | 0.33 | 1.54 | 3.19 | 24.34 | 10.19 | 6.58 | 5.41 | 1.11 |
| IL-2 (0.6) | 2.34 | 5.33 | 7.04 | 25.23 | 10.16 | 5.77 | 16.60 | 2.82 |
| IL-2 (1.8) | 3.08 | 14.29 | 14.16 | 43.63 | 15.57 | 11.62 | 35.11 | 7.65 |
| IL-2 (3.6) | 3.15 | 14.59 | 12.08 | 43.53 | 15.13 | 17.64 | 44.83 | 4.76 |
| IL-21 (33) | 0.37 | 1.77 | 4.67 | 31.50 | 10.47 | 6.32 | 5.05 | 1.05 |
| IL-21 (100) | 0.30 | 1.33 | 5.35 | 23.16 | 8.55 | 5.40 | 6.35 | 1.54 |
| IL-21(200) | 0.56 | 1.86 | 8.46 | 23.85 | 9.20 | 6.39 | 8.43 | 3.20 |

In addition, additional endpoints were measured between groups. The following endpoints where compared: Body weight, spleen weight, vascular leakage in lung and liver, and serum cytokines. No significant difference in body weights was observed between groups. As discussed above, animals treated with both doses of IL-2, Group II and III, had significantly heavier spleen weights as compared to IL-21 and PBS control treated animals (p<.0001). Animals treated with both doses of IL-21 treated animals, Group IV and V, had significantly heavier spleen weights as compared to PBS control animals (p<.007 Group IV and p<.0001 Group V).

Vascular leakage was also measured in both lung and liver. In lung, both groups of IL-2 treated animals, Group II and III, had a significant increase in vascular leakage (p<.0001) as compared to PBS control animals. Only Group III, the high dose of IL-2 had a significant increase in vascular leakage as compared to both low dose and high dose IL-21 (p<.0001 and p<.0065) respectively. Only the highest dose of IL-21, Group V, had a significant increase in vascular leakage as compared to PBS treated animals (p<.0001). However, the amount of vascular leak was significantly lower than all of the IL-2 treated animals. In liver, both the low and high dose of IL-2 treated animals had a significant increase in vascular leakage (p<.0016 and p<.0001 respectively) as compared to PBS treated animals. Animals treated with the high dose of IL-2 had a significant increase in vascular leakage as compared to both low and high dose IL-21 treated animals (p<.0002 and p<.0001 respectively). Only the low dose IL-21 treated animals had a significant increase in vascular leakage as compared to PBS treated animals (p<.0397).

## Example 18

## Flow Cytometric Analysis IL-21 Receptor Expression.

The expression of IL-21 receptors on neoplastic B cells derived from non-Hodgkin's lymphoma (NHL) specimens was assessed. Multiple MAbs were used to identify neoplastic B cells and to co-localize IL-21 receptors (WIPO Publication No.s WO 0/17235 and WO 01/77171). The immunofluorescent staining by anti-IL-21R MAb or by biotin-IL-21 was recorded as mean peak fluorescence. The qualitative scores were assessed based on the shift in mean peak fluorescence relative to an isotype matched control MAb.

Using either anti-IL-21 receptor MAb or biotin-IL-21 (U.S. Patent No. 6,307,024) we consistently detected IL-21 receptor on follicular lymphoma (FL) specimens derived from lymph node. However, nearly all specimens derived from chronic lymphocytic leukemia (CLL) patients did not show significant staining for IL-21 receptors, or staining at very low intensity relative to negative control MAbs. The staining by anti-IL-21 receptor MAbs and biotin-IL-21 correlated well and detected moderate staining of follicular lymphoma. These data suggested that IL-21 receptors represent a therapeutic target for follicular lymphoma.

Example 19

Activity of Mouse IL-21-Treated Alloreactive Murine CTL (cytotoxicity assays)

A. CTL assay

CTL (cytotoxic T lymphocyte)-mediated target cytolysis was examined by a standard $^{51}Cr$-release assay. Alloreactive (H-$2^b$ anti-H-$2^d$) CTL were generated in a mixed lymphocyte culture with C57B1/6 splenocytes (H-$2^b$) with 3000 rad-irradiated Balb/c splenocytes (H-$2^d$). After 7 days, the CTL were re-stimulated with irradiated Balb/c splenocytes (and no additional cytokines). After an additional 7 days, CTL were re-stimulated for 5 days in the presence of supernatants collected from conA-activated rat splenocytes (a crude source of cytokines known to support CTL growth), 10 ng/ml recombinant mouse IL-2 (R&D Systems, Inc, Minneapolis, MN), recombinant human IL-15 (R&D Systems), IL-21, or a combination of IL-15 and IL-21 (5 ng/ml each). After 5 days, the CTL were assayed for their capacity to lyse $^{51}Cr$-labeled target cells: H-$2^d$ P815 mastocytoma cells (ATCC No.TIB-64) and the H-$2^b$ thymoma EL4 (ATCC No.TIB-39) as a negative control.

We grew P815 and EL4 cells in RP10 medium (standard RPMI 1640 (Gibco/BRL, Grand Island, NY) supplemented with 10% FBS (Hyclone) as well as 4 mM glutamine (Gibco/BRL), 100 I.U./ml penicillin+100 MCG/ml streptomycin (Gibco/BRL), 50 $\mu$M $\beta$-mercaptoethanol (Gibco/BRL) and 10mM HEPES buffer (Gibco/BRL). On the day of assay, 1-2x$10^6$ target cells were harvested and resuspended at 2.5-5x$10^6$ cells/ml in RP10 medium. We added 50-100 $\mu$l of 5 mCi/ml $^{51}Cr$-sodium chromate (NEN, Boston, MA) directly to the cells and incubated them for 1 hour at 37˚C, then washed them twice with 12 ml of PBS and resuspended them in 2 ml of RP10 medium. After counting the cells on a hemacytometer, the target cells were diluted to 0.5-1x$10^5$ cells/ml and 100 $\mu$l (0.5-1x$10^4$ cells) were mixed with effector cells at various effector:target ratios. After a 4-hour co-incubation of effector cells and the labeled target cells at 37˚C, half of the supernatant from each well was collected and counted in a gamma counter for 1 min/sample. The percentage of specific $^{51}Cr$ release was calculated from the formula 100 x (X-Y)/(Z-Y), where X is $^{51}Cr$ release in the presence of effector cells, Y is the spontaneous release in the absence of effectors, and Z is the total $^{51}Cr$ release from target cells incubated with 0.5% Triton X-100. Data were plotted as the % specific lysis versus the effector-to-target ratio in each well.

CTL re-stimulated in the presence of rmIL-2 exhibited the highest lytic activity on the P815 target cells, achieving >70% specific lysis at an effector-to-target ratio of 33:1. The next most active CTL were those re-stimulated in the presence of IL-21+rhIL-15 (62% specific lysis), followed by CTL cultured with rhIL-15 (~50% lysis), CTL cultured with IL-21 alone (30% lysis), and CTL re-stimulated with rat conA supernatant (~10% lysis). None of the CTL lysed the H-$2^b$ EL4 cells (all CTL lysed fewer than 2% of the EL4 targets, even at the highest effector-to-target ratio of 33:1). This pattern of cytokine enhancement of cytolysis (IL-2>IL-21+IL-15>IL-15>IL-21>conA SN) held true in 2 replicate experiments. These data demonstrate that IL-21, particularly in combination with IL-15, can enhance CTL effector function.

Example 20

Delayed Type Hypersensitivity in IL-21 Knockout (KO) mice

IL-21 is a cytokine that is produced by T cells and has been shown to play a role in T cell proliferation and function. Delayed Type Hypersensitivity (DTH) is a measure of helper CD4 T cell responses to specific antigen. In this, mice are immunized with a specific protein (E.g., chicken ovalbumin, OVA) and then later challenged with the same antigen in the ear. Increase in ear thickness after the challenge is a measure of specific immune response to the antigen, mediated mainly by CD4 T cells. To understand the *in vivo* function of IL-21, mice deficient in IL-21 protein were engineered (IL-21 KO mice). If IL-21 is important for T cell responses, IL-21 KO mice should be expected to have a defect in T cell responses. One method to test this is to induce a DTH response in IL-21 KO mice. IL-21 KO mice and control littermates were immunized with OVA mixed with an adjuvant CFA (Complete Freund's Adjuvant). Groups of mice were then rechallenged in the ear with either PBS (control) or OVA. Control mice developed good DTH when injected with OVA as shown by increase in the ear thickness at 24 hours post challenge. In contrast, IL-21 KO mice had a lesser degree of ear thickness compared to controls. This difference was statistically significant (p-0.0164). However, at 48 hour post challenge, there was no difference in the response of wild type or IL-21 KO mice. As expected, both control and IL-21 KO mice did not respond to PBS (no change in ear thickness).

IL-21 KO mice (n=8) and control wild type littermates (n=8) were immunized in the back with 100 $\mu$g chicken

ovalbumin (OVA) emulsified in CFA in a total volume of 200 μl. Seven days after the immunization, half the mice in each group (n=4/gp) were injected with 10 μl PBS in the ear and the other half injected with 10 μg OVA in PBS in a volume of 10 μl. Ear thickness of all mice was measured before injecting mice in the ear (0 measurement). Ear thickness was measured 24 hours and 48 hours after challenge. The difference in ear thickness between the 0 measurement and the 24 hour or 48 hour measurement was calculated.

At 24 hour post challenge, control mice or IL-21 KO mice rechallenged with PBS showed minimal or no change in ear thickness. In response to the OVA rechallenge, control mice ears showed significant inflammation ($7.3 \pm 1.1 \times 10^{-3}$ in). In contrast" IL-21 KO mice showed a decrease in ear thickness compared to controls ($5 \pm 0.42 \times 10^{-3}$ in). This difference was statistically significant (p=0.0164). This suggests that IL-21 does play an important role in CD4 T cell responses. However, at 48 hour post challenge, responses in IL-21 KO mice were no different from controls suggesting that IL-21 does not influence the response at this stage. Further experiments are underway to assess the role of IL-21 in DTH responses and in T cell responses.

These results suggest that IL-21 plays an important role in CD4 T cell responses. CD4 T cell responses contribute significantly to immunity, both in a positive manner to boost immunity towards microbes and tumors and in a negative manner in cases of autoimmunity and inflammation. Use of IL-21 may be considered to boost CD4 T cell responses based on the above results.

Example 21

IL-21 Modifies the Response of OT-I T cells to OVA Peptide as Presented by Murine Dendritic Cells.

A. Isolation and labeling of OT-I T cells

Mice bearing a transgenic T cell receptor specific for OVA257-264 in H-$2K^b$ are available (OT-I transgenics, Jackson Laboratories). Cells from lymph nodes from these animals were adherence depleted and the CD8 T cells (OT-I T cells) were enriched by negative selection using CD8 Cellect columns (Cedarlane Laboratories, Hornby, Ontario, Canada). Purity of CD8 T cells was assessed by flow cytometry and was typically 90-95% with <1% CD4 T cells..

OT-I T cells were labeled with carboxyfluorescein diacetate succinimidyl ester (CFSE; Molecular Probes, Eugene, OR) by placing them in growth media comprising RPMI-1640 medium supplemented with 10% FCS (JRH, Lenexa KS; Hyclone, Logan UT), 2 mM glutamine (Gibco BRL), 50 U/ml penicillin (Gibco BRL), 50 μg/ml streptomycin (Gibco BRL, Grand Island, NY) and 50 μM 2-mercaptoethanol (Sigma, St Louis, MO) containing 5 μM CFSE for 5 min at room temperature. The cells were then washed three times, each time by resuspending in PBS containing 5% FBS, centrifuging 5 min at 300' g, 20°C, and removing the supernatant. Cells were resuspended in growth media prior to use.

B. Preparation of murine dendritic cells

Bone marrow derived dendritic cells (DCs) from mouse bone marrow were cultured in growth media the presence of GM-CSF using well-known methods (e.g., Inaba, K. et al., J. Exp. Med. 176:1693-1702, 1992). After six days in culture they were stimulated with 1ug/ml LPS (Sigma, St Louis, MO) overnight and then washed in growth media prior to use.

C. *In vitro* stimulation of T cells

DCs prepared as above were pulsed with 10 nm OVA257-264 peptide (SEQ ID NO:17) for 2 hours. The pulsed DCs are washed in growth media to remove any unbound peptide and then cultured with purified OT-I T cells prepared as described above in the presence of either media alone or 20 ng/ml mouse rIL-2 (R&D Systems, Minneapolis, MN) or 50 ng/ml murine IL-21 (U.S. Patent No. 6,307,024). After either 48 or 72 hours of incubation the cells were harvested and analyzed by flow cytometry for levels of CFSE fluorescence and Annexin V binding (Pharmingen, San Diego, CA) per manufacturers instruction.

Results showed that when OT-I T cells are presented specific antigen on DC's they undergo 3-5 rounds of cell division by day 2 and 5-7 rounds of cell division by day 3 as evidenced with CFSE labeling. In the presence of IL-2 their proliferation is increased such that by day 2 they have gone 5-6 rounds and by day 3, 7-9 rounds. When the T cells are treated with IL-2 they undergoing apoptosis at day 3 as demonstrated by Annexin V binding. In contrast to IL-2, IL-21 induces increased T cell proliferation and prevents Annexin V labeling up to day 3. IL-21 continues to enhance proliferation and prevent apoptosis even in the presence of added IL-2.

IL-21 both enhances proliferation and reduces apoptosis of the murine CTL cells. This activity implies a positive immunostimulatory role for IL-21 in clinical settings, such as cancer or viral disease, where CTL's can play a role.

Example 22

Murine IL-21 Effect on EG.7 Thymoma Growth *in vivo:* IL-21 Modifies the Response of OT-I T Cells in the EG-7 Model of CTL Mediated Anti-tumor Activity

Cytotoxic T lymphocytes (CTL) recognize infected and transformed cells by virtue of the display of viral and tumor antigens on the cell surface. Effective anti-tumor responses require the stimulation and expansion of antigen specific CTL clones. This process requires the interaction of several cell types in addition to CTL and usually results in the establishment of immunologic memory. The EG-7 tumor cell line is transfected with chicken ovalbumin and thereby expresses a well characterized T cell antigen, an ova peptide (SEQ ID NO:17) presented in H-$2K^b$. OT-I T cells (Example 21) kill EG7 tumor cells *in vitro* and *in vivo.* (Shrikant, P and Mescher, M,. J. Immunology 162:2858-2866,1999).

Mice (female, C57B16, 9 weeks old; Charles River Labs, Kingston, NY) were divided into three groups. On day 0, EG.7 cells (ATCC No. CRL-2113) were harvested from culture and 1, 000, 000 cells were injected intraperitoneal in all mice. Mice were then treated with the test article or associated vehicle by intraperitoneal injection of 0.1 ml of the indicated solution. Mice in the first group (n = 6) were treated with vehicle (PBS pH 6.0), which was injected on day 0, 2, 4, and 6. Mice in the second group (n = 6) were treated with murine IL-21, which was injected at a dose of 10 μg on day 0, 2, 4, and 6. Mice in the third group (n = 6) were treated with murine IL-21, which was injected at a dose of 75 μg on day 0, 2, 4, and 6. In both groups of mice treated with murine IL-21, time of survival was significantly increased, compared to mice treated with vehicle. The group treated with 75 μg doses of IL-21 had significantly greater survival than the group treated with 10 μg doses, and 33% (2/6 mice) of this group survived longer than 70 days. An additional portion of this study tested the effect of the same dosages carried out through day 12. The results were very similar to the shorter dosing schedule, with both doses having significantly increased survival over vehicle treatment, and the highest dose gave the best response (50% survival past 70 days).

In some experiments 4,000,000 OT-I T cells were injected Intraperitoneal in the mice on the day prior to day 0. The mice were then treated with IL-21 or vehicle as above. At various times after treatment OT-I T cells were recovered from the peritoneal cavity and counted. The presence of the OT-I T cells had no effect on the survival time of the vehicle treated mice. IL-21 treatment resulted in a ten-fold increase in the number of OT-I T cells that could be recovered from the peritoneal cavity. In mice treated with IL-21 the presence of the OT-I T cells enhanced survival time compared to the mice treated with IL-21 alone.

The increase in survival conferred by IL-21 treatment with or with out exogenously added tumor specific T cells shows that IL-21 is activating endogenous effector cells of the immune system. The increased recovery of OT-I T cells from the peritoneal cavity shows that IL-21 is increasing the number of tumor specific T cells at the site of the tumor.

As predicted by the ability of IL-21 to enhance T cell survival *in vitro* these results indicate that treatment with IL-21 has enhanced the ability of the immune system to destroy tumor cells *in vivo.* These results *in vivo* demonstrate a positive immunostimulatory role for IL-21 in relevant clinical settings, such as in human cancer or viral disease, where CTL's can play a role in combating disease.

Example 23

IL-21 Reduces Tumor Load in the RMA-RAE1 Model of NK Mediated Anti-tumor Activity

Natural killer cells serve as a first line of defense against certain viral infections and tumors. Effective NK cell activity does not require prior exposure to the target nor are they thought to maintain immunologic memory of the target. Thus, NK cells "sense" if cells are transformed, infected, or otherwise "stressed" by virtue of a range of molecules on the surface of the target cell. RAE-1 is a protein expressed on the surface of "stressed" cells which specifically engages an activating a receptor on the surface of NK cells thereby leading to lysis of the "stressed" cell. Transfection of the RMA tumor cell line with RAE-1 renders it sensitive to lysis by NK cells both in *vitro* and *in vivo.*

The RMA lymphoma cell line (provided by Dr. L. Lanier and Dr. Jay Ryan, UCSF, San Francisco, CA) was grown in RPMI-1640 medium supplemented with 10% FCS (JRH, Lenexa KS; Hyclone, Logan UT), 2 mM glutamine (Gibco BRL), 50 U/ml penicillin (Gibco BRL), 50 μ/ml streptomycin (Gibco BRL, Grand Island, NY) and 50 μM 2-mercaptoethanol (Sigma, St Louis, MO). Stable transfectants of RMA-RAE-1delta or mock-transfected RMA cells were established by electroporation: 30 μg of RAE-1de1ta-pCDEF3 plasmid (RAE-1delta transfectant), or pCDEF3 plasmid (mock-transfectant) was added to about 1 x $10^7$ cells in RPMI-1640 medium in a 4-mm cuvette (BioRad, Richmond, CA), respectively. The pCDEF3 vector was kindly provided by Dr. Art Weiss (UCSF, San Francisco California). Electroporation was performed by using a BioRad gene pulser (250 V, 960 μF). 48 h after electroporation, RMA-RAE-ldelta and mock-transfected cells were cultured in complete RPMI-1640 medium supplemented with 1 mg/ml G418 (GIBCO BRL).

Groups of six or more animals per experiment were injected intraperitoneally with cells which were mock-transfected or transfected with RMA-RAE-1delta. Preliminary experiments titrating the number of tumor cells injected indicated that 1 x $10^4$ RMA cells and 1x$10^5$ RMA-RAE-1delta cells resulted in tumor formation and subsequent morbidity in 100% of animals. Intraperitoneal (IP) inoculation of mice with RMA-RAE1delta cells at numbers comparable to lethal doses (about 1 x $10^4$) of the parental RMA cells results in the tumors being completely rejected without the involvement of T cells or the establishment of immunologic memory. IP inoculation of mice with a 10 fold excess of RMA-RAE1delta cells results in death of the mouse presumably by 'swamping' the capacity of the NK cells to reject the tumor (Cerwenka et al., Proc. Nat. Acad. Sci. 98:11521-11526, 2001). For cytokine efficacy experiments six IP injections of 10ug of murine IL-21 or vehicle control were administered to the mice every other day on days -4, -2, 0, 2, 4 and 6. All mice were monitored daily for tumor ascites development, indicated by swelling of the abdomen, and were sacrificed when tumor burden became excessive to avoid pain and suffering. Animals were regarded tumor free when surviving longer than 8. weeks. For the re-challenge experiments, surviving animals were inoculated with1 x $10^4$ RMA-mock cells after 8 weeks.

The administration of small amounts of murine IL-21 resulted in enhanced survival of mice receiving the 10 fold excess number of RAE1 bearing tumor cells. Some IL-21 treated mice became completely tumor-free. IL-21 treatment had no effect on the survival of mice given the parental RMA cell line showing that the effect was specifically mediated by NK cells. It appeared that RAE1, and thereby NK cells, were required for the IL-21 effect. Moreover, mice that survived the lethal RMA-RAE1 tumor challenge by virtue of IL-21 treatment were able to reject a subsequent challenge with the parental RMA cell line. This showed that IL-21 had also induced immunological memory in these mice. This ability of IL-21 to enhance the activity of NK cells shows that IL-21 can have therapeutic benefit in the treatment of patients who have tumors or viral diseases.

Example 24

Immunohistochemistry of IL-21 in Various Human Tissues and Cell Lines

The purpose of this experiment was to determine if IL-21 could be detected in select tissues by means of immunohistochemistry. Tissues were processed by standard immunohistochemical methods using a Techmate 500 (BioTek Solutions, Tucson, AZ). Briefly, deparaffinized sections of paraffin-embedded tissues were treated with 5% normal goat serum in PBS and a blocking agent (Zymed Laboratories, Inc., South San Francisco, CA, Reagent A and B (ready to use)) to minimize nonspecific background staining. One of two primary anti-IL-21 antibodies was applied (E3149 (mouse>human IL-21-CHO, HH4.9.1C2.1A6.1C8, PAS) or E2865 (mouse>human IL-21-CHO, HH4.3.1.2D1.1C12, PAS), both made in house) followed by a biotinylated goat anti-mouse antibody (Vector Laboratories, Burlingame, CA). A colored reaction product was generated via a peroxidase-3'3'-diaminobenzidine reaction (ChemMate peroxidase/DAB staining kit including methyl green counter stain; CMS/Fisher, Houston, TX). The slides coverslipped and then examined under a light microscope (Nikon Eclipse E600, Nikon Corporation, Tokyo, Japan).

The following cells and tissues were tested: BHK cells transfected with human IL-21 (positive control), BHK-570 cells, wild type (negative control), and human normal lung, human lung with chronic perivascular inflammation, human normal lymph node, human lymph node with B cell lymphoma, human spleen with myelofibrosis, and human duodenum. These tissues were obtained on a contract basis either from CHTN (Nashville, TN) or NDRI (Philadelphia, PA). Also tested were normal human tissues on a multi-tissue slide (Biomeda, Hayward, CA) and human abnormal/ tumor tissues on a multi-tissue slide (Biomeda, Hayward, CA).

The E3149 antibody produced positive staining only in the transfected BHK cells. With the E2865 antibody, intense staining was observed in the positive control cells as well as occasional mononuclear cells of unknown identity in the epithelium of the small intestine in the normal multi-tissue block. The location in the small intestine from which this sample was obtained is unknown. This positive cell type was rare (1 cell in 3 sections) in a separate section of human duodenum.

Moreover, a positive population of mononuclear cells was diffusely distributed throughout the human spleen from a patient with myelofibrosis. A similar staining pattern was not found in the spleens of the normal multi-human tissue block. Although there was some staining in the spleens of the multi-tissue block, the staining was not clearly cell associated. In addition, a section of inflamed lung contained stained spindle-shaped cells and mononuclear cells in what looks like the subpleural space (the size and quality of section make determination of location difficult). Positive staining was observed in scattered pituicytes in pituitaries in the multitissue block. Isotype antibody stained pituitaries were negative. Colloid staining was observed in thyroid sections of the multi-tissue block and in a section of thyroid adenocarcinoma in the multi-tumor block. The significance of this is unknown-the colloid in the isotype section occasionally was stained (but much less intensely than in the corresponding anti-IL-21 stained tissues). Light staining was also seen in the thyroid follicular epithelium, but its intensity was near background levels.

Staining in the undifferentiated carcinoma in the multi-tumor block is associated with a central area of necrotic debris mixed with inflammatory cells-the specificity of this staining is questionable. Likewise, staining in the pancreatic adenocarcinoma may be associated with necrotic debris or associated inflammatory cells.

Because of the location of staining in the above tissues it is possible that IL-21 plays a role in intestinal mucosal immunity (occasional cells in gut epithelium), inflammation (associated with inflammatory cells in lung, undifferentiated carcinoma, pancreatic adenocarcinoma) and myelofibrosis; fibrosis in the bone marrow resulting in extramedullary hematopoiesis in the spleen-could this also involve proliferation of the lineage of cells that produces IL-21 with IL-21 attempting to regulate the process. A caveat with these results is that we get different staining patterns with different antibodies. This may be due to the recognition of different epitopes by the two antibodies.

Example 25

IL-21 Promotes IL-2 Stimulated NK-cell Expansion in PBMNC Cultures in the Presence of IL-4

IL-4 inhibits the expansion of NK-cells stimulated with IL-2. In two experiments human peripheral blood mononuclear cells (PBMNCs) were seeded at 200,000 cells/well in alpha-MEM+ 10% autologous serum with 10 ng/ml IL-2 (R&D Systems, Minneapolis, MN) with or without 0.5 ng/ml IL-4 (R&D Systems, Minneapolis, MN), and with or without 10 ng/ml IL-21 (U.S. Patent No. 6,307,024) and grown for 8 days. The number of viable cells per well was determined using standard methods and the cells analyzed by flow cytometry for expression of CD3, CD 16, and CD56. NK-cells were defined as the CD56 positive CD3 negative population.

The cultures from the two donors cultured with IL-2 alone contained about 151,000 and 326,000 NK-cells respectively on day 8. The cultures from the two donors cultured with IL-2 and IL-21 contained about 446,000 and 588,000 NK-cells respectively. The cultures from the two donors cultured with IL-2 and IL-4 contained about 26,000 and 29,000 NK-cells on day 8. However, cultures from the two donors cultured with IL-2, IL-4 and IL-21 contained about 229,000 and 361,000 NK-cells representing an 8.8 and 12.5 fold increase in NK-cell yield over the culture with IL-2 and IL-4 only.

These results demonstrate that IL-21 promotes NK-cell expansion, and that IL-21 can largely overcome the inhibitory effects of IL-4 on NK-cell growth. In some diseases IL-4 expression can play a role in the pathology. For example mice bearing the B16F10 melanoma generate a large population of IL-4 producing CD4+ T-cells which appear to limit the host anti-tumor response. Furthermore, STAT 6 (required for IL-4 signaling) gene deficient mice exhibit an enhanced ability to reject tumors. The ability of IL-21 to antagonize the action of IL-4, and induce the expression of IFN-γ (described herein), in addition to the *in vivo* anti-tumor activity and data described herein, suggest that IL-21 can be useful in treating malignacies, infections or autoimmune disease where there is a Th2 response limiting the hosts ability to control the disease.

Example 26

IL-21 Synergizes With IL-2 to Promote the Growth of NK Cells From Peripheral Blood.

Peripheral blood lymphocytes from a healthy human donor were prepared by a standard Ficoll centrifugation method. Lymphocytes were magnetically negatively enriched as described herein using the human NK cell negative enrichment system from Stem Cell Technologies. NK's were cultured at a starting concentration of about 75,000/ml in 2 ml/well alpha MEM with 10% donor serum, 50 μM BME, 2 ng/ml flt3L, and 0, 0.5, 10, or 50 ng/ml of IL-2 +/- 0, 5, or 50 ng/ml IL-21. After 15 days of culture cells were harvested, counted, and analyzed by flow cytometry for CD3, CD56, and CD161. All cells analyzed after 15 days of culture were CD3-/CD56+, which are defined as NK cells. At day 0, cells were analyzed by flow cytometry and found to be >98% CD3-/CD56+.

The "fold increase" in cell number is defined as the final cell number divided by the starting cell number. Any "fold increase" below 1 is therefore a decrease in cell number. In general, the results at 10 ng/ml of IL-2 were similar to the results obtained with 50 ng/ml of IL-2, and the results at 5 ng/ml IL-21 were similar to those obtained with 50 ng/ml IL-21. With no IL-2 present, the fold increase in total cells was 0.064. When 5 ng/ml IL-21 was included in the culture, the fold increases was 0.11. This indicates that IL-21 has very little proliferative activity on NK's by itself. At a low concentration of 0.5 ng/ml IL-2, we saw a fold increase of .25. When 5 ng/ml IL-21 was included, we saw a fold increase of 2.9. At higher concentrations of IL-2 the fold increases were overall higher, but the effect of IL-21 was in general decreased, although still positive. At 10 ng/ml IL-2 we saw a fold increase of 2.9. When 5 ng/ml IL-21 was included, we saw a fold increase of 7.

IL-21's effect in these cultures was dependent on the presence of at least low dose IL-2. Without IL-2, the effect of IL-21 was minimal. When IL-2 is present, especially at the lower, perhaps physiological, concentration, IL-21's effect is the most profound. The lack of effect of IL-21 alone, coupled with its ability to synergize with low concentrations of other cytokines may allow it to act therapeutically at sites of infection or malignancy without causing systemic toxicity.

Example 27

IL-21 Stimulates Outgrowth of NK and NKT From Peripheral Blood Lymphocyte Cultures That Contain IL-2 or IL-15

Peripheral blood lymphocytes from 3 healthy human donors were prepared by the standard Ficoll centrifugation method. Lymphocytes were then cultured at a starting concentration of 200,000/ml in alpha MEM with 10% donor serum, 50 μM BME (Sigma), 2 ng/ml flt3L (R&D Systems), and 0, 0.5, 10, or 50 ng/ml of IL-2 (R&D Systems) or IL-15 (R&D Systems) +/- 0, 5, or 50 ng/ml IL-21 (U.S. Patent No. 6,307,024). After 12 days of culture cells were harvested, counted, and analyzed by flow cytometry for CD3, CD56, and CD8. NK cells were defined as CD56+/CD3- and NKT cells were defined as CD56+/CD3+.

The "fold increase" in cell number (defined as final cell number/starting cell number) was widely variable between the three donors, but the trends were fairly consistent. In general, the results at 10 ng/ml of IL-2 or IL-15 were similar to the results obtained with 50 ng/ml of IL-2 or IL-15, and the results at 5 ng/ml IL-21 were similar to those obtained with 50 ng/ml IL-21. With no IL-2 or IL-15 present, the fold increase in total cells was 0.33, 0.23, and 0.19 among the three donors. When 5 ng/ml IL-21 was included in the culture, the fold increases were 0.47, 0.31, and 0.35. At a low concentration of 0.5 ng/ml IL-2, we saw total cell fold increases of 2.2, 1.1, and 1.0 among the three donors. When 5 ng/ml IL-21 was included, we saw total cells increase by 5.5, 2.3, 3.1. We saw fold increases in NK numbers without IL-21 of 16, 4.2, and 3.5. When IL-21 was present (at 5 ng/ml) these increases were 24, 15, and 21 respectively. NKT's were also positively effected under these conditions. NKT fold increases were 4.4, 5.7, and 1.8 without IL-21, and 10, 9, and 15 with 5 ng/ml IL-21.

These results are mirrored with IL-15. At 0.5 ng/ml IL-15, a total cell fold increases of 0.98, 0.43, and 0.88 was seen among the three donors. When 5 ng/ml IL-21 was included, we saw total cells increase by 1.4, 0.9, 1.7 fold. Fold increases of NK numbers of 8.0, 0.85, and 3.7 were seen without IL-21. When 5 ng/ml IL-21 was present, these fold increases were 13, 5.5, and 11. NKT fold increases at 0.5 ng/ml IL-15 were 3.3, 2.3, and 1.6 for the three donors, but they were 3.9, 5.2, and 4.7 when 5 ng/ml IL-21 was included.

At higher concentrations of IL-2 the fold increases were overall higher, but the effect of IL-21 was in general decreased, although still positive. At 10 ng/ml IL-2 we saw total cell fold increases of 18, 2.5, and 2.8 among the three donors. When 5 ng/ml IL-21 was included, we saw total cells increase by 21, 3.6, 9.8 fold. We saw fold increases in NK numbers of 114, 13, and 13 without IL-21. When IL-21 was also present (at 5 ng/ml) these increases were 100, 19, and 56 respectively. NKT's were also positively effected under these conditions. NKT fold increases were 33, 15, and 12 without IL-21, and 52, 20, and 38 with 5 ng/ml IL-21.

At 10 ng/ml IL-15, we saw total cell fold increases of 18, 0.8, and 1.7 among the three donors. When 5 ng/ml IL-21 was included, we saw total cells increase by 23, 1.4, 6.9 fold. We saw fold increases of NK numbers of 128, .58, and 2.0 without IL-21. When 5 ng/ml IL-21 was present, these fold increases were 107, 1.1, and 9.4. NKT fold increases at 10 ng/ml IL-15 were 60, 6.5, and 5.7 for the three donors, but they were 66, 12, and 33 when 5 ng/ml IL-21 was included.

IL-21's effects in these cultures were dependent on the presence of at least low dose IL-2 or IL-15. Without those cytokines, the effect of IL-21 was minimal. When IL-2 or IL-15 is present, especially at the lower, perhaps physiological, concentrations, IL-21's effect is the most profound. The lack of effect of IL-21 alone, coupled with its ability to synergize with low concentrations of other cytokines may allow it to act therapeutically at sites of infection or malignancy without causing systemic toxicity.

Example 28

IL-21 Inhibits the Production of IL-13 in NK-Cell Cultures.

IL-13 shares receptor subunits and many of the biological activities of IL-4, but unlike IL-4, IL-13 is produced by NK-cells. Since NK-cells also produce IFN-γ, and these two cytokines have in large part opposing activities, experiments were conducted to examine the effects of IL-21 on IL-13 and IFN-γ expression in PBMNC and NK-cell cultures.

Negatively selected human peripheral blood NK-cells were seeded at about 3.75x10e5 cells/ml and stimulated 2days with 10 ng/ml IL-2, IL-4 (R&D Systems) or IL-21 (U.S. Patent No. 6,307,024) or without any cytokine in alpha-MEM+10% autologous serum. After two days in culture IL-2 was added to all wells to 10 ng/ml, and the cell were cultured for an additional 3 days, then the supernatants were collected and analyzed by ELISA for IL-13 and IFN-γ. NK-cells grown for two days without any cytokine produced about 2130 pg/ml IFN-γ and 175 pg/ml IL-13. Cells stimulated with IL-21 produced abut 10,300 pg/ml IFN-γ and 90 pg/ml IL-13. Cells stimulated with IL-2 produced 12,700 pg/ml IFN-γ and 1000 pg/ml IL-13. Cells stimulated with IL-4 produced no detectable IFN-γ nor IL-13.

Of note, cells stimulated for the first two days with IL-21 produced 5 times more IFN-γ, but only one half the IL-13 of unstimulated cells. When compared to cell stimulated with IL-2 for the first two days of culture cells stimulated with IL-21 produced 80% as much IFN-γ, but only 9% as much IL-13. Thus IL-21 selectively promotes the expression of IFN-γ and depresses IL-13 expression.

Example 29

IL-21 Synergizes With IL-2 to Promote IFN-γ Production in Mouse Splenic NK Cells

C57BL/6 mouse splenic NK cells were prepared by water lysing a cell suspension from the spleens, then utilizing the Stem Cell Technologies murine NK negative enrichment magnetic cell sorting protocol. The cells prepared using this method were 65% Pan NK positive based on flow analysis using the DX5 Pan NK antibody from PharMingen.

The negatively enriched murine NK cells were cultured for 8 days at 500,000 cells/ml in RPMI 1640 with 10% heat inactivated fetal bovine serum and 2 mM L-glutamine, 50 μM BME, and PSN antibiotic with 20 ng/ml mIL-2 (R&D Systems) or 10 ng/ml mIL-21 (U.S. Patent No. 6,307,024) or both. The cell supernatants were harvested and cells were counted at the end of the culture period. Cell supernatants were assayed for mIFN-γ using a commercially available ELISA kit from PharMingen.

Cell numbers at the end of the eight day period were about 1,300,000 for the IL-2 containing culture, 220,000 for the IL-2/IL-21 culture, and 10,000 for the IL-21 culture. The mIFN-γ levels were 2.2 ng/ml, 30 ng/ml, and .28 ng/ml respectively. When expressed as pg/500,000 cells, the results are 238, 14,000, and 12,000. IL-21 enhances IFN-γ expression in these cultures, which when combined with the cell survival/proliferation effects of IL-2, results in high levels of IFN-γ being secreted into the medium. IFN-γ is an important initiator of the immune response, and is considered a TH1 biased cytokine. This data supports that IL-21 plays a role in the anti-cancer, antiviral activity of the immune system, and hence can be used as a therapeutic in anti-cancer, anti-viral and other applications.

Example 30

Effects IL-21 -Saporin Toxin Conjugate on T Cells and Human T Cell Lines

The ability of the murine IL-21-saporin toxin conjugate to bind normal murine T Cells was determined by FACS competition assays and compared to binding on the same cells by the murine IL-21. It was shown that the murine IL-21-saporin toxin conjugate binds to these cells with the same affinity as IL-21 (Example 30A).

The presence of Human IL-21 Receptor (WIPO Publication No.s WO 0/17235 and WO 01/77171) on the following T Cell lines was determined by FACS analysis: human T Cell leukemia MOLT-13 (DSMZ No. ACC_436), human cutaneous T Cell lymphoma HUT-78 (ATCC No. TIB_161), human cutaneous T Cell lymphoma HUT-102 (ATCC No. TIB_162); human ALCL line DEL (DSMZ No. ACC_338), and human T/NK cell leukemia YT (DSMZ No. ACC_434; Example 30B).

The effects of human IL-21-saporin toxin conjugate described herein were tested on both normal human T Cells (Example 30C) and the Human T cell lines shown to express the Human IL-21 Receptor (i.e., MOLT-13, HUT-78, HUT-102, DEL and YT; example 30D). The results showed that normal Human T cells and T cell lines treated with IL-21-saporin toxin conjugate proliferated much less or not at all as compared with cells left untreated or cells cultured with unconjugated IL-21 or with saporin alone.

The results indicate that IL-21-toxin conjugate (saporin or other) can control some types of T-cell neoplasms with little or no effect also on normal Human T cell proliferation *in vitro* at 30 pM or less. A proposed mechanism of the observed inhibition of cell line proliferation *in vitro* is as follows: the Human IL-21-toxin conjugate binds with high affinity to IL-21 receptor expressed on the surface of these cells. The Human IL-21-toxin conjugate is then taken up by the cells and, in the case with the saporin-toxin conjugate, the cells' ability to produce protein and their proliferation is subsequently blocked. Thus, IL-21-saporin immunotoxin conjugate, or other IL-21-toxin fusion could be used therapeutically in prevention and treatment T-cell leukemias and lymphomas, and other cancers wherein IL-21 receptors are expressed.

A. The binding of Murine IL-21 -saporin toxin conjugate on normal Murine T Cells by flow cytometry analysis.

Total murine splenocytes were isolated from normal 4-month old female C57/BL6 mice (Jackson Laboratories, Bar Harbor, ME). Spleens were harvested and gently mashed between frosted slides to create a cell suspension. Red blood cells were removed by hypotonic lysis as follows: cells were pelleted and the supernatant removed by aspiration. We disrupted the pellet with gentle vortexing, then added 900 ul of sterile water while shaking, followed quickly (less than 5 sec later) by 100 ul of 10X HBSS (Gibco/BRL; Rockville Maryland). The cells were then resuspended in 10 ml of 1X HBSS and debris was removed by passing the cells over a nylon mesh-lined cell strainer (Falcon/BD; Franklin NJ). These RBC-depleted spleen cells were then pelleted and resuspended in FACS stain buffer: HBSS (GibcoBRL; Rockville Maryland) containing 3% Human serum, 1% BSA, and 10mM HEPES.

Aliquots containing about $1X10^6$ white blood cells from spleen were stained for 3-color flow cytometric analysis with anti-murine CD3-FITC, anti-murine B220-CyChrome mAbs (PharMingen, San Diego, CA) and biotinylated murine IL-21 (2 ug/ml) followed by Streptavidin-PE (Caltag; Burlingame CA). Staining of the biotinylated murine IL-21 was competed by equivalent titered molar amounts (0.0175nM to 3.5nM) of both unbiotinylated IL-21 and IL-21-saporin conjugate. Cells were analyzed on a FACSScan using CellQuest software (Becton Dickinson, Mountain View, CA). The results demonstrated that the murine IL-21 -saporin toxin conjugate binds to these cells with the same affinity as IL-21.

B. The binding of biotinylated Murine IL-21 on Human T Cell lines by flow cytometry analysis.

Aliquots containing 0.4X10exp6 to 1X10exp6 MOLT-13 cells, HuT-78 cells, HuT-102 cells, DEL cells or YT cells were stained for 1-color flow cytometric analysis with titered biotinylated murine IL-21 (40ng/ml to 1000ng/ml) followed by Streptavidin-PE (Caltag; Burlingame CA). Cells were analyzed on a FACSScan using CellQuest software (Becton Dickinson, Mountain View, CA). The results demonstrated that biotinylated murine IL-21 binds to these cell lines with strong affinity. The results also demonstrated that the murine IL-21 is cross-species reactive as it recognizes and binds the Human IL-21 Receptor molecule on the surface of the Human T cell lines.

C. The effect of Human IL-21-saporin immunotoxin on normal Human T-cell proliferation.

Whole blood was collected from a healthy human donor, aliquoted into 50 ml tubes and passed over Ficoll density gradients. RBC-depleted cells at the interface (PBMC) were collected and washed extensively with PBS followed by RPMI 1640 supplemented with 10% human ultraserum and 2mM L glutamine. The PBMC were suspended to 111X10exp6/ml in MACS buffer (PBS, 1% BSA, 0.8mg/L EDTA). Cells were combined with anti-human CD14 microbeads, anti-human CD19 microbeads, and anti-human CD56 microbeads (Miltenyi Biotech; Auburn CA) as per manufacturer specifications. The mixture was incubated for 20 min. at 4˚C. These cells labeled with CD14, CD19 and CD56 beads were washed with 5X volume MACS buffer, and then resuspended in 1.5 ml MACS buffer.

A VS+ column (Miltenyi Biotech; Auburn CA) was prepared according to the manufacturer's instructions. The VS+ column was then placed in a VarioMACS™ magnetic field (Miltenyi Biotech; Auburn CA). The column was equilibrated with 3ml MACS buffer. The CD14/CD19/CD56 bead-coated PBMC were then applied to the column. The CD14-CD19-CD56- PBMC fraction containing Human T cells were allowed to pass through the column and were collected in a 15ml tube. The column was washed with 10ml (2 X 5 ml) MACS buffer to wash out residual Human T Cells. The column with bound CD14+CD19+CD56+ cells was discarded. The Human T cells and wash eluant were pooled together and counted.

A sample of the negatively-selected human T cells was removed for staining to assess the fraction's purity. A cychrome-conjugated mouse anti-human CD3 antibody (PharMingen) was used for staining the selected cells. The negatively-selected T cells were shown to be 67% CD3+.

The isolated primary T cells were cultured at $0.5X10^6$/ml with equivalent titered molar amounts (0.03pM to 30pM) of both IL-21 and IL-21-saporin conjugate on a plate coated with titered (0 to 2$\mu$g/ml) anti-human CD3 (clone UCHT-1; Southern Biotech; Birmingham Alabama) as a co-activator for the Human T cells. After 3 days growth, the cells were pulsed with 3H-thymidine (5 $\mu$Ci/ml; AmershamBiosciences, Piscataway NJ) for 18 hours. Cells were lysed and DNA was captured onto glass filter mats (Packard, Meriden CT) and counted for 3H-thymidine incorporation to assess proliferation of the cells.

The results showed that IL-21 at 0.3 pM and higher had a stimulatory effect in combination with 2 $\mu$g/ml coated anti-CD3. The IL-21-saporin conjugate had no such stimulatory effect. It also had no inhibitory effect as compared with the 2 $\mu$g/ml anti-CD3 coat stimulus alone. In sum, these data can be interpreted to mean that while a IL-21 stimulus was present in the wells containing the IL-21-saporin conjugate, the expected increase in a proliferation due to the IL-21 part of the molecule was ablated by the presence of the saporin portion of the conjugated molecule. However, the saporin portion of the molecule did not ablate the stimulatory effect due to the anti-CD3 coated antibody.

D. The effect of Human IL-21-saporin immunotoxin conjugate on Human T cell lines.

Cells were seeded at 5,000 cells/ml to 50,000 cells/ml with equivalent titered molar amounts (0.2pM to 400pM) of both IL-21 and IL-21-saporin conjugate. After 2 days growth the cells were, pulsed with 5 $\mu$Ci/ml $^3$H-thymidine (AmershamBioscierices, Piscataway NJ) for 18 hours. Cells were either lysed and DNA was captured onto glass filter mats (Packard, Meriden CT) and counted for $^3$H-thymidine incorporation to assess proliferation of the cells.

IL-21 -saporin treated MOLT-13 cells had incorporated $^3$H-thymidine to only 70% of the $^3$H-thymidine incorporation by the IL-21 treated MOLT-13 cells. IL-21-saporin treated HuT-78 cells grew to only 33% the density of the untreated HuT-78 cells. IL-21-saporin treated HuT-102 cells grew to only 20% the density of the untreated Hut-102 cells. IL-21-saporin treated DEL cells grew to only 25% the density of the untreated DEL cells. IL-21 -saporin treated YT cells grew to only 33% the density of the untreated YT cells. The results indicate that IL-21-toxin conjugate (saporin or other) can be effective in controlling some types of T-cell neoplasms. Moreover, IL-21-saporin immunotoxin conjugate, or other IL-21-toxin fusion could be used therapeutically in prevention and treatment T-cell leukemias and lymphomas, and other cancers wherein IL-21 receptors are expressed.

Example 31

*In vivo* Effects of IL-21 on B-cell lymphomas

Human B-lymphoma cell lines are maintained *in vitro* by passage in growth medium. The cells are washed thoroughly in PBS to remove culture components.

SCID Mice are injected with (typically) one million human lymphoma cells via the tail vein in a 100 microliter volume. ( The optimal number of cell injected is determined empirically in a pilot study to yield tumor take consistently with desired kinetics.) IL-21 treatment is begun the next day by either subcutaneous. implantation of an ALZET® osmotic mini-pump (ALZET, Cupertino, CA) or by daily i.p injection of IL-21 or vehicle. Mice are monitored for survival and significant morbidity. Mice that lose greater than 20% of their initial body weight are sacrificed, as well as mice that exhibit substantial morbidity such as hind limb paralysis. Depending on the lymphoma cell line employed, the untreated mice typically die in 3 to 6 weeks. For B cell lymphomas that secrete IgG or IgM, the disease progression can also be monitored by weekly blood sampling and measuring serum human Immunoglobulin levels by ELISA.

A. IL-21 Do seresponse/IM-9 model

Mice were injected with 1 x $10^6$ IM-9 cells, and 28 day osmotic mini pumps implanted the following day. The pumps were loaded with the following concentrations of IL-21 to deliver: 0, 0.12, 1.2 or 12 micrograms per day with 8 mice per dose group. IL-21 exhibited a clear dose dependent effect in protecting mice from the tumor cell line. The effects of IL-21 were dose dependent. Surviving mice at the end of the experiment had no signs of disease and no detectable human IgG in their serum.

B. IL-21 NK depletion/ IM-9 model

Mice were depleted of NK-cells by administering 5 doses of anti-asialo-GM-1 antibody every third day beginning 15 days prior to injection of tumor cells or left undepleted as controls. Half of the depleted and undepleted mice were treated with 12 μg/day IL-21 and the other half were treated with vehicle only. Depletion of NK-cells did not significantly diminish the activity of IL-21. These data demonstrated that NK-cells are not necessary for the effect of IL-21 in the IM-9 model in SCID mice.

C. Other cell lines tested

The following additional cell lines were tested using the model shown for IM-9 cells. IL-21 delivered at 12 μg/day by minipump is effective against CESS cells in SCID mice. IL-21 administered to mice with RAJI cell implanted tumors had no efficacy. IL-21 administered to mice with RAMOS cell implanted tumors had no efficacy. IL-21 administered to mice with HS SULTAN cell implanted tumors had significant efficacy, but did not prevent disease in most mice, only slows its onset. IL-21 DoHH2 had no efficacy.

These data demonstrate that the efficacy of IL-21 in SCID mouse lymphoma models correlates with the ability to inhibit the growth of the lymphoma cell lines *in vivo.* Furthermore, NK-cell depletion of SCID mice for both T-cells and B-cells does not diminish the effectiveness of IL-21 in the IM-9 model. It is likely that the efficacy of IL-21 in SCID mouse lymphoma models is dependent on it direct effects on the tumor cells because no efficacy was seen in three of three cell lines tested in the model that were not inhibited by IL-21 *in vitro,* and NK depletion had no effect on the efficacy of IL-21 in the IM-9 model. In a patient with an intact immune system, IL-21 dependent effector cell mediated antitumor effects are predicted from experiments with immunocompetent mice in syngeneic tumor models. The demonstration of direct antitumor effects in SCID mice suggests that IL-21 therapy could have combined direct and effector mediated anti-tumor effects in selected B-cell malignancies in humans.

Example 32

The Effects of IL-21 in a Mouse Syngeneic Ovarian Carcinoma Model

The effect of IL-21 is tested for efficacy in ovarian carcinoma using a mouse syngeneic model as described in Zhang et al., Am. J. of Pathol. 161:2295-2309, 2002. Briefly, using retroviral transfection and fluorescence-activated cell sorting a C57BL6 murine ID8 ovarian carcinoma cell line is generated that stably overexpresses the murine VEGF164 isoform and the enhanced green fluorescence protein (GFP). The retroviral construct containing VEGF 164 and GFP cDNAs was transfected into BOSC23 cells. The cells are analyzed by FACS cell sorting and GFP high positive cells are identified.

The ID8 VEGF 164/GFP transfected cells are cultured to subconfluence and prepared in a single-cell suspension in phosphate buffer saline (PBS) and cold MATRIGEL (BD Biosciences, Bedford, MA). Six to eight week old femal C57BL6 mice are injected subcutaneously in the flank at 5 x $10^6$ cells or untransfected control cells. Alternatively, the mice can be injected intraperitoneally at 7 x $10^6$ cells or control cells. Animals are either followed for survival or sacrificed eight weeks after inoculation and evaluated for tumor growth. Mice are treated with recombinant. murine IL-21 beginning 3-14 days following tumor implantation, or when tumor engraftment and growth rate is established. Treatment levels of 0.5 - 5 mg/kg will be administered on a daily basis for 5-14 days, and may be continued thereafter if no evidence of neutralizing antibody formation is seen.

Example 33

The Effects of IL-21 in a Mouse RENCA Model

The efficacy of IL-21 in a renal cell carcinoma model is evaluated using BALB/c mice that have been injected with RENCA cells, a mouse renal adenocarcinoma of spontaneous origin, essentially as described in Wigginton et al., J. Nat. Cancer Instit. 88:38-43, 1996.

Briefly, BALB/c mice between eight and ten weeks are injected with RENCA cells R 1X $10^5$ cells into the kidney capsule of the mice. Twelve days after tumor cell implantation, the mice are nepharectomized to remove primary tumors. The mice are allowed to recover from surgery, prior to administration of IL-21. Mice are treated with recombinant. murine IL-21 beginning 3-14 days following tumor implantation, or when tumor engraftment and growth rate is established. Treatment levels of 0.5 - 5 mg/kg will be administered on a daily basis for 5-14 days, and maybe continued thereafter if no evidence of neutralizing antibody formation is seen. Alternatively, RENCA cells may be introduced by subcutaneous (5 x 10e5 cells) or intravenous (1 x 10e5 cells) injection.

The mice are evaluated for tumor response as compared to untreated mice. Survival is compared using a Kaplan-Meier method, as well as tumor volume being evaluated.

Example 34

The Effects of IL-21 in a Mouse Colorectal Tumor Model

The effects of IL-21 in a colorectal mouse model are tested as described in Yao et al., Cancer Res. 63:586-592, 2003. In this model, MC-26 mouse colon tumor cells are implanted into the splenic subcapsul of BALB/c mice. After 14 days, the treated mice are administered IL-21. Mice are treated with recombinant. murine IL-21 beginning 3-14 days following tumor implantation, or when tumor engraftment and growth rate is established. Treatment levels of 0.5 - 5 mg/kg will be administered on a daily basis for 5-14 days, and may be continued thereafter if no evidence of neutralizing antibody formation is seen.

The efficacy of IL-21 in prolonging survival or promoting a tumor response is evaluated using standard techniques described herein.

Example 35

The Effect of IL-21 in a Mouse Pancreatic Cancer Model

The efficacy of IL-21 in a mouse pancreatic cancer model is evaluated using the protocol developed by Mukherjee et al., J. Immunol. 165:3451-3460, 2000. Briefly, MUC1 transgenic (MUC1.Tg) mice are bred with oncogene-expressing mice that spontaneously develop tumors of the pancreas (ET mice) designated as MET. MUC1.Tg mice. ET mice express the first 127 aa of SV40 large T Ag under the control of the rat elastase promoter. Fifty percent of the animals develop life-threatening pancreatic tumors by about 21 wk of age. Cells are routinely tested by flow cytometry for the presence of MUC1. All mice are on the C57BL/6 background. Animals are sacrificed and characterized at 3-wk intervals from 3 to 24 wk. Mice are carefully observed for signs of ill-health, including lethargy, abdominal distention, failure to eat or drink, marked weight loss, pale feces, and hunched posture.

The entire pancreas is dissected free of fat and lymph nodes, weighed, and spread on bibulus paper for photography. Nodules are counted, and the pancreas is fixed in methacarn, processed for microscopy by conventional methods, step sectioned at 5 μm (about 10 sections per mouse pancreas), stained with hematoxylin and eosin, and examined by light microscopy, Tumors are obtained from MET mice at various time points during tumor progression, fixed in methacarn (60% methanol, 30% chloroform, 10% glacial acetic acid), embedded in paraffin, and sectioned for immunohistochemical analysis. MUC1 antibodies used are CT1, a rabbit polyclonal Ab that recognizes mouse and human cytoplasmic tail region of MUC1, HMFG-2, BC2, and SM-3, which have epitopes in the TR domain of MUC1.

Determination of CTL activity is performed using a standard $^{51}Cr$ release method after a 6-day in vitro peptide stimulation without additional added cytokines. Splenocytes from individual MET mice are harvested by passing through a nylon mesh followed by lysis of RBC.

Single cells from spleens of MET mice are analyzed by two-color immunofluorescence for alterations in lymphocyte subpopulations: CD3, CD4, CD8, Fas, FasL, CD11c, and MHC class I and II. Intracellular cytokine levels were determined after cells are stimulated with MUC1 peptide (10 μg/ml for 6 days) and treated with brefeldin-A (also called Golgi-Stop; PharMingen) as directed by the manufacturer's recommendation (4 μl/1.2 x $10^7$ cells/6 ml for 3 h at 37°C before staining). Cells are permeabilized using the PharMingen permeabilization kit and stained for intracellular IFN-γ, IL-2, IL-4, and IL-5 as described by PharMingen. All fluorescently labeled Abs were purchased from PharMingen. Flow cytometric analysis was done on Becton Dickinson FACscan using the CellQuest program (Becton Dickinson, Mountain View, CA).

Mice are treated with recombinant. murine IL-21 beginning 3-14 days following tumor implantation, or when tumor engraftment and growth rate is established. Treatment levels of 0.5 - 5 mg/kg will be administered on a daily basis for 5-14 days, and may be continued thereafter if no evidence of neutralizing antibody formation is seen.

Example 36

The Effects of IL-21 in a Murine Breast Cancer Model

The efficacy of IL-21 in a murine model for breast cancer is made using a syngeneic model as described in Colombo et al., Cancer Research 62:941-946, 2002. Briefly, TS/A cells which are a spontaneous mammary carcinoma for BALB/C mice. The cells are cultured for approximately one week to select for clones. The selected TS/A cells are grown and used to challenge CD-1 *nu*/*nu* BR mice (Charles River Laboratories) by injected 2 x $10^2$ TS/A cells subcutaneously into the flank of the mouse.

Mice are treated with recombinant. murine IL-21 beginning 3-14 days following tumor implantation, or when tumor engraftment and growth rate is established. Treatment levels of 0.5 - 5 mg/kg will be administered on a daily basis for 5-14 days, and may be continued thereafter if no evidence of neutralizing antibody formation is seen. The tumors are excised after sacrificing the animals and analyzed for volume and using histochemistry and immunohistochemistry.

Example 37

The Effects of IL-21 in a Murine Prostate Cancer Model

The effects of IL-21 on tumor response are evaluated in murine prostate cancer model, using a model similar to that described in Kwon et al., PNAS 96:15074-15079, 1999. In this model, there is a metastatic outgrowth of transgenic adenocarcinoma of mouse prostate (TRAMP) derived prostate cancer cell line TRAMP-C2, which are implanted in C57BL/6 mice. Metastatic relapse is reliable, occurring primarily in the draining lymph nodes in close proximity to the primary tumor.

Briefly, the C2 cell line used is an early passage line derived from the TRAMP mouse that spontaneously develops autochthonous tumors attributable to prostate-restricted SV40 antigen expression. The cells are cultured and injected subcutaneously into the C57BL/6 mice at 2.5-5 x $10^6$ cells/0.1 ml media. Mice are treated with recombinant. murine IL-21 beginning 3-14 days following tumor implantation, or when tumor engraftment and growth rate is established. Treatment levels of 0.5 - 5 mg/kg will be administered on a daily basis for 5-14 days, and may be continued thereafter if no evidence of neutralizing antibody formation is seen. The tumors are excised after sacrificing the animals and analyzed for volume and using histochemistry and immunohistochemistry.

Example 38

The Effects of IL-21 and Chemotherapeutics on Growth of Human B-Cell Lines In *Vitro*

The effects of IL-21 and zeocin alone and in combination were tested on the growth of IM-9 and HS Sultan human B-cell lines *in vitro* to ascertain if the growth inhibitory/cytotoxic effects of IL-21 and a chemo-therapeutic agent will be additive or synergistic on IL-21 sensitive cells lines. ZEOCIN ( Invitrogen, Carlsbad, CA) is an antibiotic with a mechanism of action similar to the related chemo-therapeutic bleomycin was used.

IM-9 and HS Sultan cell lines were plated at 50,000 cells/ml in RPMI1640 medium suppplemented with 2 mM L-glutamine and 10% heat inactivated FBS with and without IL-21 (at 20 ng/ml) and or ZEOCIN (at 15.6 μg/ml for IM-9s and 31 μg/ml for HS Sultans) for two days then the cells were harvested and washed once to remove the zeocin and replated with or without IL-21 in a serial cell dilution series, with 6 wells per dilution, in 96 well round bottom plates to determine their relative growth ability. The plates were scored in 6 days using Alamar blue, as a measure of viable cells per well, and reflecting their survival and outgrowth from the prior treatment. Cells populations treated with ZEOCIN had less than one tenth the growth capacity of untreated cells, and the combination of IL-21 with zeocin further reduced the growth capacity by approximately an order of magnitude. These data suggest that IL-21 could be successfully combined with chemotherapy to augment response rates in the treatment of lymphoma.

Example 39

LCMV Models

LCMV models are *in vitro* models to test a compound's effect on cells infected with a member of the *Flaviviridae* family, to which HCV is a member. These models are used to evaluate the effect IL-21 has on CTLs and the effect IL-21 has on viral load. There are two models that are used: LCMV Armstrong (acute) infection and LCMV Clone 13 (chronic) infection. (See, e.g., Wherry et al., J. Virol. 77:4911-4927, 2003; Blattman et al., Nature Med. 9(5):540-547, 2003; Hoffman et al., J. Immunol. 170:1339-1353, 2003.) There are three stages of CD8 T cell development in response to virus: 1) expansion, 2) contraction, and 3) memory (acute model). IL-21 is injected during each stage for both acute and chronic models. In the chronic model, IL-21 is injected 60 days after infection to assess the effect of IL-21. For both acute and chronic models, IL-21 is injected, and the following parameters are examined: tetramer staining by flow to count the number of LCMV-specific CD8+ T cells; the ability of tetramer+ cells to produce cytokines when stimulated with their cognate LCMV antigen; and the ability of LCMV-specific CD8+ T cells to proliferate in response to their cognate LCMV antigen. LCMV-specific T cells are phenotyped by flow cytometry to assess the cells activation and differentiation state. Also, the ability of LCMV-specific CTL to lyse target cells bearing their cognate LCMV antigen is examined. The number and function of LCMV-specific CD4+ T cells is accessed, except for in the cytolysis assay.

Improvement in the quality and quantity of LCMV specific CD8+ T cells after IL-21 administration is determined. Specifically, results in increased cytokine production, especially IFN-γ increased percentages of tetramer+ cells that proliferate and an increase in the cytolytic activity are shown. The phenotype of CD8+ T cells from IL-21 treated mice reflects differentiation to an effector cell, that is, loss of CD27 expression and loss of CCR7 expression as well as increased perforin and granzyme B expression. Also, a reduction in viral load after treatment with IL-21 is shown. For IL-21 treated mice a 20% increase in the percentage of tetramer positive T cells that proliferate, make cytokine, or display a mature phenotype relative to untreated mice, is considered significant. A 20% increase in cytolytic activity is considered significant.

IL-21 injection leading to a reduction in viral load is due to more effective control of viral infection especially in the chronic model where untreated the viral titers remain elevated for an extended period of time. A 5 fold reduction in viral titer relative to untreated mice is considered significant.

Example 40

*Ex vivo* studies of human CTL from HCV patients

Blood obtained from chronically infected HCV patients and HCV-specific CTL is examined *in vitro* after culture with IL-21. The HCV-specif T cells are enumerated by staining with tetramers containing HCV peptides and soluble HLA Class I proteins. Using flow cytometry the ability of CD8+ HCV-specific T cells to proliferate and produce cytokines (especially interferon-γ and IL-2) in response to HCV antigens incubated in the presence or absence of IL21 is accessed. HCV-specific CTLs are phenotyped with respect to activation state and effector function (specifically for CD27 and CCR7 expression). Cytolytic activity for HLA-matched target cells bearing HCV peptides is also evaluated. (See, e.g., Wedemeyer et al., J. Immol. 169:3447-58, 2002; Gruener et al., J. Virol. 75:5550-58, 2001; Cramp et al., Gastroenterology 118:346-55, 2000.)

*In vitro* culture of HCV-specific T cells with their cognate antigen with IL-21 is measured to demonstrate increased survival, proliferation, and cytokine production by the CTL, as compared to those cultured in media alone. Cytolytic activity of the HCV specific CTL is measured to demonstrate significant increases after culture in IL-21 relative to the same CTL cultured in media.

Example 41

Influenza Model of Acute Viral Infection

A. Preliminary Experiment to test antiviral activity.

To determine the antiviral activity of IL-21 on *Influenza* virus and measure various immune parameters, focusing on cell-mediated and humoral immunity, an *in vivo* study using influenza infected c57B1/6 mice was performed using the following protocol:

Animals: 6 weeks-old female BALB/c mice (Charles River) with 148 mice, 30 per group.
Groups:

(1) Absolute control (not infected) to run in parallel for antibody titre and histopathology (2 animals per group)
(2) Vehicle (i.p.) saline
(3) Amantadine (positive control) 10 mg/day during 5 days (per os) starting 2 hours before infection
(4) IL-21 treated (5 μg, i.p. starting 2 hours after infection)
(5) IL-21 (25 μg, i.p. starting 2 hours after infection)
(6) IL-21 (125 μg, i.p. starting 2 hours after infection)

Day 0 - Except for the absolute controls, all animals infected with Influenza virus
For viral load (10 at LD50)
For immunology workout (LD30)
Day 0 - 9 - daily injections of IL-21 (i.p.)
Body weight and general appearance recorded (3 times/week)
Day 3 - sacrifice of 8 animals per group
Viral load in right lung (TCID50)
Histopathology in left lung
Blood sample for antibody titration
Day 10 - sacrifice of all surviving animals collecting blood samples for antibody titration, isolating lung lymphocytes (4 pools of 3) for direct CTL assay ( in all 5 groups), and quantitative immunophenotyping for the following markers: CD3/CD4, CD3/CD8, CD3/CD8/CD11b, CD8/CD44/CD62L, CD3/DX5, GR-1/F480, and CD19.

Results demonstrated IL-21 treatment enhanced the virus specific cytolytic activity of pulmonary mononuclear cells. This enhanced CTL activity is hypothesized to be important for resolution of human viral disease. The treatment had no significant effect on day 4 viral load, day 10 antibody production or weight loss. The failure to alter day 4 viral load suggests that IL-21 treatment did not significantly enhance NK activity. Day 10 antibody production is an early time point of uncertain significance. The failure to alter weight loss suggests that IL-21 enhancement of CTL activity was inadequate to alter the course of the infection. This could be because the infection was simply too aggressive.

B. Secondary experiment to test for antiviral activity.

Study No 1:

1. $LD_{50}$ determination of a new stock of mouse-adapted influenza virus and analysis of the capacity to induce immune responses in infected C57B1/6 mice. First, two new stocks of human influenza virus are produced and titrated. Second, the last passage of mouse-adapted virus is passaged in in embryonated eggs. Also, a non-adapted virus stock (ATCC frozen stock) passaged in embryonated eggs. Third, an allantoic fluid control stock is prepared in embryonated eggs inoculated with PBS. Both stocks are titrated by HAU (using 0.5% rooster erythrocytes) and $TCID_{50}$ (using MDCK cells). The virus stocks and allantoic fluid control are frozen at -80˚C.

2. Determination of the $LD_{50}$ for mouse-adapted virus stocks in C57B1/6 mice is made using 6 Female C57B1/6 mice (8 weeks-old) (Charles River). The mice are given an intranasal inoculation (20 μl) with a micropipette of anaesthetized animals (Ketamine/xylazine, i.p.), and then given 6 doses of each virus stock (1/10 dilution) + PBS control. There are 8 animals per dose. The numbers of animals are recorded daily, and the body weight measured every other day.

On day 14 post-infection, the number of animals that survived the virus challenge and their body weight are recorded, and the $LD_{30}$ for both virus stocks for C57B1/6 mice is calculated.

3. Immune response induced by mouse-adapted influenza virus in C57BL/6 mice is analyzed.
CTL-mediated cytotoxicity is assayed using effector cells (mononuclear cells from the lungs), 10 days post infection in mice given 1 $LD_{30}$ of virus stock. The target cells are influenza-infected EL-4 (H-$2^b$) tumor cell line ATCC #TIB39 (Brit. J. Cancer 4: 372, 1950) Assays are done in quadruplicates at different E:T ratios starting from 50:1. In addition to compare susceptibility of target cells infected with non-adapted and mouse-adapted A/PR/8/34 influenza virus, uninfected target cells and EL-4 cells infected with B-Lee/40 influenza virus are used for specificity control. Three experiments are done to compare CTL responses in mice infected with the virus stocks.
Immunophenotyping: Preliminary immunophenotyping experiments are done on 3 pools of mononuclear cells from the lungs harvested on Day 10 post-infection from 3 mice infected with 1 $LD_{30}$ of mouse-adapted virus. Lung cell populations of interest are also be determined in normal C57BL/6 mice using pooled cells from 8 mice for each determination. These studies will use 44 animals.

Study No.2:

To determine infecting doses at which clinical signs of illness (loss of weight) are not too severe while still inducing a detectable but suboptimal lung CTL response a dose response study starting with the $LD_{30}$ of mouse-adapted virus is tested. The experiment uses 40 female C57B1/6 mice (8 weeks-old) (Charles River), and the animals are anaesthetized. There are 4 groups of 2 animals for 1 $LD_{30}$ and of 4 animals per dose for lower doses. The doses are 1 LD30, 1/10 dilution, 1/100 dilution of virus.
The number of animals are recorded daily, and body weight is recorded every other day. Day 10 post-infection: Record the number of animals that survived the virus challenge and their body weight. On day 10, the animals are sacrificed and evaluated for CTL induction in 4 pools per dose (2 or 4 animals per pool), using uninfected, A/PR/8/34 and B/Lee/40-infected EL-4 as targets and 7 different E:T ratio (50/1,25/1,12/1,6/1; 3/1,1.5/1; and 0.75/1).

Study No.3

Efficacy study of IL-21 in C57B1/6 mice infected with mouse-adapted virus is done using 8 weeks-old female C57B1/6 mice (Charles River). The groups have 36 animals per group.

Group 1: Vehicle (i.p.)
Group 2: Positive control: Anti-influenza neutralizing antibody (goat anti-influenza A/USSR (H1N1) (Chemicon International, Temecula, CA); 40 μg/mouse at 2 h and 4 h post infection (10 μl intranasal)
Group 3: ZG-01 (5 μg, i.p.)
Group 4: ZG-01 (25 μg, i.p.)
Group 5: ZG-0 (125 μg, i.p.)

Table 11

| Treatment Groups | Sacrificed at Day 10 | |
|---|---|---|
| | Immunology | RNA+IHC+H&E |
| Group 1(28 mice) Vehicle | 24 animals (6 mice/pool) | 4 animals |
| Group 2 (28 mice) +ve control (Neutralizing Ab) | 24animals (6 mice/pool) | 4 animals |
| Group 3 (28 mice) IL-21 (5 μg) | 24 animals (6 mice/pool) | 4 animals |
| Group 4 (28 mice) | 24 animals | 4 animals |

(continued)

| Treatment Groups | Sacrificed at Day 10 | |
|---|---|---|
| | Immunology | RNA+IHC+H&E |
| IL-21 (25 μg) | (6 mice/pool) | |
| Group 5 (28 mice) IL-21 (125 μg) | 24 animals (6 mice/pool) | 4 animals |
| | | |
| Total (140 animals) | 120 | 20 |

Following-life observations and immunological workouts are prepared:

Day 0 - all animals infected with Influenza virus (dose determined in experiment 2)
Day 0 - 9 - daily injections of IL-21 (i.p.)
Body weight and general appearance recorded every other day
Day 10 - sacrifice of surviving animals (24 animals will be used for complete immune evaluation; 4 for DNA isolation and H&E staining)

Isolation of lung lymphocytes (4 pools of 6) for direct CTL assay in the lungs (in all 5 groups) using EL-4 as targets and different E:T ratio (based on best results from experiments 1 and 2).

Tetramer staining: The number of CD8+ T cells binding MHC Class I tetramers containing influenza A nucleoprotein (NP) epitope are assessed using complexes of MHC class I with viral peptides: FLU-$NP_{366-374}/D^b$ (ASNENMETM), (LMCV peptide/$D^b$).

Quantitative immunophenotyping of the following: CD8, tetramer, intracellular IFNγ, NK1.1, CD8, tetramer, CD62L, CD44, CD3(+ or -), NK1.1(+), intracellular IFNγ, CD4, CD8, NK1.1, DX5, CD3 (+ or -), NK1.1, DX5, tetramer, Single colour samples for cytometer adjustment.

Survival/Re-challenge Study

Table 12

| Treatment Groups | Re-challenged with $1LD_{30}$ | Re-challenged with $5LD_{50}$ |
|---|---|---|
| Group 6 (32 mice) Vehicle | Group 6A 12 animals | Group 6B 20 animals |
| Group 7 (32 mice) IL-21 (125 μg) | Group 7A 12 animals | Group 7A 20 animals |
| Total (64 animals) | 24 animals | 40 animals |

Day 30: Survival study (12 animals per group) with mice are treated for 9 days with different doses of IL-21 or with positive anti-influenza antibody control. Body weight and antibody production in individual serum samples (Total, IgG1, IgG2a, IgG2b) are measured.

Re-challenge study:

2 groups of 32 animals are used in this study.
Day 0: Both groups will be infected with A/PR virus (1LD30).
Group 6 will not be treated.
Group 7 will be treated for 9 days with 125 μg of IL-21.
Day 30: Survival study

Body weight and antibody production in individual serum samples (Total, IgG1, IgG2a, IgG2b) are measured.
Day 60: Re-challenge study
Survivors in each group will be divided into 2 subgroups
Group 6A and 7A will be re-challenge with A/PR virus (1 LD30)
Group 6B and 7B will be re-challenge with A/PR virus (1 LD30).

Both groups will be followed up and day of sacrifice will be determined. Body weight and antibody production in individual serum samples (Total, IgG1, IgG2a, IgG2b) are measured.

From the foregoing, it will be appreciated that, although specific embodiments of the invention have been described herein for purposes of illustration, various modifications may be made without deviating from the spirit and scope of the invention. Accordingly, the invention is not limited except as by the appended claims.

## Claims

1. An *in vitro* method for expansion of hematopoietic cells and hematopoietic cell progenitors comprising: culturing bone marrow or peripheral blood cells with a composition comprising an amount of IL-21 sufficient to produce an increase in the number of lymphoid cells in the bone marrow or peripheral blood cells as compared to bone marrow or peripheral blood cells cultured in the absence of IL-21.

2. An *in vitro* method for preparing anti-cancer effector cells for use in adoptive immunotherapy, comprising treating peripheral blood progenitor cells (PBPCs) in culture with IL-21 to differentiate and proliferate the PBPCs into high-density lymphoid cultures.

3. An *in vitro* method according to Claim 1 or 2, wherein the lymphoid cells are selected from NK cells, LAK cells, and tumor-specific T-cells such as cytotoxic T-lymphocytes (CTL).

4. *Any vitro* method according to any of Claims 1 to 3, wherein the bone marrow or peripheral blood cells are cultured with at least one other immunomodulatory agent.

5. An *in vitro* method according to Claim 4, wherein the immunomodulatory agent comprises a cytokine, such as SCF, IL-2, IL-4, IL-7, IL-1b, IL-18, GM-CSF, Flt3 ligand and interferon.

6. An *in vitro* method according to any of Claims 1-5, wherein the IL-21 comprises a polypeptide having at least 80% identity to an IL-21 polypeptide comprising residues 41-148 of SEQ ID NO: 2 or residues 32-162 of SEQ ID NO: 2.

7. Anti-cancer effector cells for use in the treatment of cancer in a patient by adoptive immunotherapy, wherein said anti-cancer effector cells have been obtained from the patient, and wherein the use comprises an initial *ex vivo* step of expanding the anti-cancer effector cells in culture in the presence of IL-21.

8. Anti-cancer effector cells for use according to Claim 7, wherein the anti-cancer effector cells are selected from NK cells, LAK cells, and tumor-specific T-cells such as cytotoxic T-lymphocytes (CTL).

9. Anti-cancer effector cells for use according to Claim 7 or 8, wherein said *ex vivo* expansion step further comprises culturing the anti-cancer effector cells in the presence of one or more other immunomodulatory agents, such as cytokines, preferably SCF, IL-2, IL-4, IL-7, IL-15, IL-18, or interferon.

**10.** Anti-cancer effector cells for use according to any of Claims 7-9, wherein said anti-cancer effector cells are expanded *ex vivo* by a method according to any of Claims 1-6.

**11.** Anti-cancer cells for use according to any of Claims 7-10, in combination with IL-21.

**12.** Anti-cancer cells for use according to any of Claims 7-11, wherein said IL-21 comprises a polypeptide having at least 80% identity to an IL-21 polypeptide comprising residues 41-148 of SEQ ID NO: 2 or residues 32-162 of SEQ ID NO: 2.

**13.** Anti-cancer cells for use according to any of Claims 7-12, for treating cancer in a human.

**14.** Anti-cancer cells for use according to Claim 13, wherein the human cancer is selected from: carcinomas (epithelial tissues); sarcomas of the soft tissues and bone (mesodermal tissues); adenomas (glandular tissues); cancers of the liver (hepatoma), kidney (renal cell carcinomas), or CNS (gliomas, neuroblastoma); hematological cancers; viral associated cancers (eg. associated with retroviral infections, HPV, or hepatitis B or C); lung cancers; endocrine cancers; gastrointestinal cancers (eg. biliary tract cancer, liver cancer, pancreatic cancer, stomach cancer or colorectal cancer); genitourinary cancers (eg. prostate cancer bladder cancer, renal cell carcinoma); gynecologic cancers (eg. uterine cancer, cervical cancer, ovarian cancer); breast cancer or other cancers of the reproductive system; head and neck cancers; and hematopoietic cancers (eg. lymphocytic leukemia, myeloid leukemia, Hodgkin's lymphoma, Non-Hodgkin's lymphomas, chronic lymphocytic leukemia, and other leukemias and lymphomas).

**15.** Use of anti-cancer effector cells for the manufacture of a medicament for treating cancer in a patient by adoptive immunotherapy, wherein said anti-cancer effector cells have been obtained from the patient, and wherein said use comprises an initial *ex vivo* step of expanding the anti-cancer effector cells in culture in the presence of IL-21.

Europäisches Patentamt
European Patent Office
Office européen des brevets

# PARTIAL EUROPEAN SEARCH REPORT

under Rule 62a and/or 63 of the European Patent Convention. This report shall be considered, for the purposes of subsequent proceedings, as the European search report

Application Number
EP 10 17 1625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 00/53761 A2 (ZYMOGENETICS INC [US]) 14 September 2000 (2000-09-14)<br>* see Seq.2, claims 1, 17, 31-34 and [0157]-[0163], examples 40-42 * | 1-6<br>7-15 | INV.<br>A61K35/14<br>A61K35/28<br>C12N5/00<br>A61P35/00 |
| E | WO 03/082212 A2 (US GOV HEALTH & HUMAN SERV [US]; HWU PATRICK [US]; WANG GANG [US]) 9 October 2003 (2003-10-09)<br>* see claims 1-7 ex.4-7, [0021].[0038] * | 1-15 | |
| X,P | WO 03/028630 A2 (WYETH CORP [US]; CARTER LAURA [US]; WHITTERS MATTHEW J [US]; COLLINS M) 10 April 2003 (2003-04-10)<br>* see claims 24, 36, examples 10-11, page s90-92 * | 1-6 | |
| | -/-- | | |

TECHNICAL FIELDS SEARCHED (IPC)

A61K
C12N

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

1

| Place of search | Date of completion of the search | Examiner |
|---|---|---|
| Munich | 19 January 2011 | Merckling-Ruiz, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches Patentamt
European Patent Office
Office européen des brevets

# PARTIAL EUROPEAN SEARCH REPORT

Application Number
EP 10 17 1625

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X<br><br><br><br><br><br><br><br><br><br>Y | PARRISH-NOVAK JULIA ET AL: "Interleukin 21 and its receptor are involved in NK cell expansion and regulation of lymphocyte function", NATURE, NATURE PUBLISHING GROUP, LONDON, GB, vol. 408, no. 6808, 2 November 2000 (2000-11-02), pages 57-63, XP002186602, ISSN: 0028-0836, DOI: DOI:10.1038/35040504<br>* see abstract and pages 60-62 *<br>----- | 1-6<br><br><br><br><br><br><br><br><br><br>7-15 | |
| Y | DUDLEY M E ET AL: "ADOPTIVE TRANSFER OF CLONED MELANOMA-REACTIVE T LYMPHOCYTES FOR THE TREATMENT OF PATIENTS WITH METASTATIC MELANOMA", JOURNAL OF IMMUNOTHERAPY, LIPPINCOTT WILLIAMS & WILKINS, HAGERSTOWN, MD, US, vol. 24, no. 4, 1 January 2001 (2001-01-01), pages 363-373, XP002977245, ISSN: 1524-9557, DOI: DOI:10.1097/00002371-200107000-00012<br>* abstract *<br>----- | 7-15 | TECHNICAL FIELDS SEARCHED (IPC) |

1

EPO FORM 1503 03.82 (P04C10)

Europäisches Patentamt
European Patent Office
Office européen des brevets

**INCOMPLETE SEARCH**
**SHEET C**

Application Number
EP 10 17 1625

Claim(s) completely searchable:
1, 5-7, 9-15

Claim(s) searched incompletely:
2-4, 8

Reason for the limitation of the search:

The present application is a divisional application of application EP 03757366 (parent application)
In claims 2-3, features are combined that are disclosed in two different passages of the parent application (NB : and in no other part of the parent application):
a) page 45 "anti-cancer effector cells", "LAK cells, tumor specific cells"
b) page 47 "blood progenitor cells" "differenciate and proliferate into high-density lymphoid cultures" "NK cells, cytotoxic T lymphocytes (CTLs)"
Page 45 describes embodiment 8) adoptive cell therapy, also called adoptive immunotherapy. This cell therapy consists in taking tumor-infiltrating lymphocytes from the patient, wherein said lymphocytes recognize the tumor antigens of the patients, expand these specific T cells ex vivo and reinject them into the patient so as to obtained an enhanced anti-tumor cellular immune response.
Page 47 is directed t a completely different embodiment, in which bone marrow or peripheral blood lymphoid cells (preferably from the patent) are expanded ex vivo and re-injected e.g. after chemotherapy so as to speed up the recovery of the marrow.
Claims 2-3 and 8 contain a patchwork of features taken either from embodiment a) or b). Technical features from different embodiment cannot be combined so as to obtain new claims. The subject-matter of claims 2-3 and 8 is not explicitly disclosed in the parent application as filed.
As for claim 4, the general concept "immunomodulatory agent" has only been found to be disclosed (in the parent application as filed) in the context of embodiment a) adoptive immunotherapy, but not in the context of embodiment b).
what has been searched is : claims claims 2-4 and 8 taken only in the context of adopive immunotherapy.

## ANNEX TO THE EUROPEAN SEARCH REPORT ON EUROPEAN PATENT APPLICATION NO.

EP 10 17 1625

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2011

| Patent document cited in search report | Publication date | Patent family member(s) | Publication date |
|---|---|---|---|
| WO 0053761 A2 | 14-09-2000 | AT 377076 T | 15-11-2007 |
| | | AU 777842 B2 | 04-11-2004 |
| | | AU 2005200240 A1 | 17-02-2005 |
| | | BR 0008772 A | 07-05-2002 |
| | | CA 2366921 A1 | 14-09-2000 |
| | | CN 1378595 A | 06-11-2002 |
| | | CN 1827639 A | 06-09-2006 |
| | | CN 1927883 A | 14-03-2007 |
| | | CZ 20013184 A3 | 17-04-2002 |
| | | DE 60036930 T2 | 09-10-2008 |
| | | DK 1165791 T3 | 11-02-2008 |
| | | EP 1165791 A2 | 02-01-2002 |
| | | EP 1881070 A2 | 23-01-2008 |
| | | ES 2295019 T3 | 16-04-2008 |
| | | HU 0200418 A2 | 29-05-2002 |
| | | JP 4405686 B2 | 27-01-2010 |
| | | JP 2002537839 T | 12-11-2002 |
| | | MX PA01009074 A | 27-03-2002 |
| | | NO 20014364 A | 09-11-2001 |
| | | NZ 514708 A | 28-01-2005 |
| | | PL 351160 A1 | 24-03-2003 |
| | | PT 1165791 E | 16-01-2008 |
| | | RU 2346951 C2 | 20-02-2009 |
| WO 03082212 A2 | 09-10-2003 | AU 2003226141 A1 | 13-10-2003 |
| | | CA 2481304 A1 | 09-10-2003 |
| | | EP 1589990 A2 | 02-11-2005 |
| | | JP 2005530716 T | 13-10-2005 |
| WO 03028630 A2 | 10-04-2003 | CA 2460916 A1 | 10-04-2003 |
| | | EP 1432431 A2 | 30-06-2004 |
| | | JP 2005508915 T | 07-04-2005 |
| | | JP 2008094853 A | 24-04-2008 |
| | | NZ 532021 A | 30-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 38712702 P
- US 6307024 B
- WO 017235 A
- WO 0177171 A
- US 5223409 A
- WO 9206204 A
- WO 9720078 A
- US 5155027 A
- US 5567584 A
- US 5037743 A, Welch
- US 5143830 A, Holland
- US 5252714 A, Harris
- WO 0117235 A
- EP 0154316 A
- WO 017717 A


### Non-patent literature cited in the description


- **Kelso, A.** *Immun. Cell Biol.,* 1998, vol. 76, 300-317
- Lymphoma Treatments and Managing Their Side Effects. *Lymphoma Res. Found. Of Amer.,* 2001
- **Lanford, R.E. ; Bigger, C.** *Virology,* 2002, vol. 293, 1-9
- Medicine. Scientific American, Inc. 1995, vol. 4, 1-8
- **Nilsson et al.** *EMBO J.,* 1985, vol. 4, 1075
- **Nilsson et al.** *Methods Enzymol.,* 1991, vol. 198 (3
- **Smith ; Johnson.** *Gene,* 1988, vol. 67, 31
- **Grussenmeyer et al.** *Proc. Natl. Acad. Sci. USA,* 1985, vol. 82, 7952-4
- **Hopp et al.** *Biotechnology,* 1988, vol. 6, 1204-10
- **Ford et al.** *Protein Expression and Purification,* 1991, vol. 2, 95-107
- **Dynan ; Tijan.** *Nature,* 1985, vol. 316, 774-78
- **Cosman.** The Hematopoietin Receptor Superfamily. *Cytokine,* 1993, vol. 5 ((2)), 95-106
- **Nicola et al.** *Advances in Protein Chemistry,* 1999, vol. 52, 1-65
- **Kelso, A.** *Immunol. Cell Biol.,* 1998, vol. 76, 300-317
- **Altschul et al.** *Bull. Math. Bio.,* 1986, vol. 48, 603
- **Henikoff ; Henikoff.** *Proc. Natl. Acad. Sci. USA,* 1992, vol. 89, 10915
- **Pearson ; Lipman.** *Proc. Nat'1 Acad. Sci. USA,* 1988, vol. 85, 2444
- **Pearson.** *Meth. Enzymol.,* 1990, vol. 183, 63
- **Barton.** *Current Opin. Struct. Biol.,* 1995, vol. 5, 372-376
- **Cordes et al.** *Current Opin. Struct. Biol.,* 1996, vol. 6, 3-10
- **Lapthorn et al.** *Nat. Struct. Biol.,* 1995, vol. 2, 266-268
- **Bean et al.** *Anal. Biochem.,* 1992, vol. 201, 216-226
- **Gray.** *Protein Sci.,* 1993, vol. 2, 1732-1748
- **Patterson et al.** *Anal. Chem.,* 1994, vol. 66, 3727-3732
- **Johnson.** *Proteins,* 1990, vol. 7, 205-214
- **Schaanan et al.** *Science,* 1992, vol. 257, 961-964
- **Hopp et al.** *Proc. Natl. Acad. Sci.,* 1981, vol. 78, 3824-3828
- **Hopp.** *J. Immun. Meth.,* 1986, vol. 88, 1-18
- **Triquier et al.** *Protein Engineering,* 1998, vol. 11, 153-169
- **Cunningham ; Wells.** *Science,* 1989, vol. 244, 1081
- **Bass et al.** *Proc. Natl Acad. Sci. USA,* 1991, vol. 88, 4498
- Site-Directed Mutagenesis and Protein Engineering. **Corey.** Proteins: Analysis and Design. Academic Press, Inc, 1998, 259-311
- **Hilton et al.** *J. Biol. Chem.,* 1996, vol. 271, 4699
- **Horisberger ; Di Marco.** *Pharmac. Ther.,* 1995, vol. 66, 507
- **Treuter et al.** *Molec. Gen. Genet.,* 1993, vol. 240, 113

- Expression and preliminary deletion analysis of the 42 kDa 2-5A synthetase induced by human interferon. **Content et al.** Biological Interferon Systems, Proceedings of ISIR-TNO Meeting on Interferon Systems. 1987, 65-72
- The EGF Receptor. **Herschman et al.** Control of Animal Cell Proliferation 1. Academic Press, 1985, 169-199
- **Coumailleau et al.** *J. Biol. Chem.,* 1995, vol. 270, 29270
- **Fukunaga et al.** *J. Biol. Chem.,* 1995, vol. 270, 25291
- **Yamaguchi et al.** *Biochem. Pharmacol.,* 1995, vol. 50, 1295
- **Meisel et al.** *Plant Molec. Biol.,* 1996, vol. 30, 1
- **Reidhaar-Olson ; Sauer.** *Science,* 1988, vol. 241, 53
- **Bowie ; Sauer.** *Proc. Nat'1 Acad. Sci. USA,* 1989, vol. 86, 2152
- **Lowman et al.** *Biochem.,* 1991, vol. 30, 10832
- **Derbyshire et al.** *Gene,* 1986, vol. 46, 145
- **Ner et al.** *DNA,* 1988, vol. 7, 127
- **Stemmer.** *Nature,* 1994, vol. 370, 389
- **Stemmer.** *Proc. Natl Acad. Sci. USA,* 1994, vol. 91, 10747
- **Robertson et al.** *J. Am. Chem. Soc.,* 1991, vol. 113, 2722
- **Ellman et al.** *Methods Enzymol.,* 1991, vol. 202, 301
- **Chung et al.** *Science,* 1993, vol. 259, 806
- **Chung et al.** *Proc. Nat'l Acad. Sci. USA,* 1993, vol. 90, 10145
- **Turcatti et al.** *J. Biol. Chem.,* 1996, vol. 271, 19991
- **Koide et al.** *Biochem.,* 1994, vol. 33, 7470
- **Wynn ; Richards.** *Protein Sci.,* 1993, vol. 2, 395
- **Nucci et al.** *Advanced Drug Delivery Reviews,* 1991, vol. 6, 133-155
- **Lu et al.** *Int. J. Peptide Protein Res.,* 1994, vol. 43, 127-138
- **Geysen et al.** *Proc. Nat'l Acad. Sci. USA,* 1983, vol. 81, 3998
- **Sutcliffe et al.** *Science,* 1983, vol. 219, 660
- **Lane ; Stephen.** *Curr. Opin. Immunol.,* 1993, vol. 5, 268
- **Cortese et al.** *Curr. Opin. Biotechnol.,* 1996, vol. 7, 616
- Epitope Mapping. **Mole.** Methods in Molecular Biology. The Humana Press, Inc, 1992, vol. 10, 105-116
- Production and Characterization of Synthetic Peptide-Derived Antibodies. **Price.** Monoclonal Antibodies: Production. Engineering, and Clinical Application. Cambridge University Press, 1995, 60-84
- Current Protocols in Immunology. John Wiley & Sons, 1997, 9.3.1-9.3.59.4.1-9.4.1.1
- **Sambrook et al.** Molecular Cloning: A Laboratory Manual. Cold Spring Harbor Laboratory Press, 1989
- Current Protocols in Molecular Biology. John Wiley and Sons, Inc, 1987
- **Wigler et al.** *Cell,* 1978, vol. 14, 725
- **Corsaro ; Pearson.** *Somatic Cell Genetics,* 1981, vol. 7, 603
- **Graham ; Van der Eb.** *Virology,* 1973, vol. 52, 456
- **Neumann et al.** *EMBO J.,* 1982, vol. 1, 841-5
- **Hawley-Nelson et al.** *Focus,* 1993, vol. 15, 73
- **Ciccarone et al.** *Focus,* 1993, vol. 15, 80
- **Miller ; Rosman.** *BioTechniques,* 1989, vol. 7, 980-90
- **Wag ; Finer.** *Nature Med.,* 1996, vol. 2, 714-6
- Molecular Biology Labfax. Academic Press, 1991
- Bacillus Cloning Methods. **Hardy.** DNA Cloning: A Practical Approach. IRL Press, 1985
- Short Protocols in Molecular Biology. John Wiley & Sons, 1995
- **Wu et al.** Methods in Gene Biotechnology. CRC Press, Inc, 1997
- Antibodies: A Laboratory Manual. Cold Spring Harbor Laboratory Press, 1988
- **Mr6zek et al.** *Blood,* 1996, vol. 87, 2632-2640
- **Robertson.** *Blood,* 1990, vol. 76, 2451-2438
- **Bonnefoix et al.** *Leukemia and Lymphoma,* 1997, vol. 25, 169-178
- **Watt.** *FASEB,* 1991, vol. 5, 281-284
- **Francis.** *Differentiation,* 1994, vol. 57, 63-75
- **Raes.** *Adv. Anim. Cell Biol. Technol. Bioprocesses,* 1989, 161-171
- **Ottensmeier.** *Chemico-Biological Interactions,* 2001, vol. 135-136, 653-664
- Decision Resourses. *Non-Hodgkins Lymphoma, Waltham,* February 2002
- **Jacob et al.** *Blood,* 1990, vol. 75 (5), 1154-1162
- **Hausner et al.** *J. Immunol. Methods,* 2001, vol. 247 (1-2), 175-186
- **Lagneaux et al.** *Br. J. Hematol.,* 2001, vol. 112 (2), 344-352
- **Banyard, J. ; Zetter, B.R.** *Cancer and Metast. Rev.,* 1999, vol. 17, 449-458
- **O'Reilly MS et al.** *Cell,* 1994, vol. 79, 315-328
- **Rusciano D et al.** Murine Models of Liver Metastasis. *Invasion Metastasis,* 1995, vol. 14, 349-361
- **Cattan AR ; Douglas E.** *Leuk. Res.,* 1994, vol. 18, 513-22
- **Flavell, DJ.** *Hematological Oncology,* 1996, vol. 14, 67-82
- **Elsas et al.** *J. Exp. Med.,* 1999, vol. 190, 355-66
- **Shrikant et al.** *Immuni,* 1999, vol. 11, 483-93

- **Shrikant et al.** *J. Immunol.,* 1999, vol. 162, 2858-66
- **Zhang et al.** *Am. J. Pathol.,* 2002, vol. 161, 2295-2309
- **Wigginton et al.** *J. of Nat. Cancer Inst.,* 1996, vol. 88, 38-43
- **Yao et al.** *Cancer Res.,* 2003, vol. 63 (3), 586-586592
- **Mukherjee et al.** *J. Immunotherapy,* 2003, vol. 26, 47-42
- **Kaplan-Lefko et al.** *Prostate,* 2003, vol. 55 (3), 219-237
- **Kwon et al.** *PNAS,* 1999, vol. 96, 15074-15079
- **Arap et al.** *PNAS,* 2002, vol. 99, 1527-1531
- **Atkins.** *Semin. Oncol.,* 2002, vol. 29 (3), 12
- **Burnet FM.** *Lancet,* 1967, vol. 1, 1171-4
- **Klein G.** *Harvey Lect.,* 1975, vol. 69, 71-102
- **Kuper et al.** *J. Intern. Med.,* 2000, vol. 248, 171-83
- **Birkeland et al.** *Lancet,* 2000, vol. 355, 1886-7
- **Penn I.** *Cancer Detect Prev.,* 1994, vol. 18, 241-52
- **Smyth, M. et al.** *J. Exp. Med.,* 2000, vol. 192, 755-760
- **Davidson, W. et al.** *J. Exp. Med.,* 1998, vol. 187, 1825-1838
- **Peng, S et al.** *J. Exp. Med.,* 1996, vol. 184, 1149-1154
- **Kaplan, D. et al.** *Proc. Nat. Acad Sci. USA,* 1998, vol. 95, 7556-7561
- **Shankaran V. et al.** *Nature,* 2001, vol. 410, 1107-1111
- **Boon T. et al.** *Immunol. Today,* 1997, vol. 18, 267-8
- **Stockert E. et al.** *J. Exp. Med.,* 1998, vol. 187, 1349-54
- **Sahin U et al.** *Curr. Opin. Immunol.,* 1997, vol. 9, 709-16
- **Jäger, E. et al.** *Proc. Nat. Acad. Sci. USA,* 2000, vol. 97, 12198-12203
- **Bauer, S et al.** *Science,* 1999, vol. 285, 727-729
- **Coley WB.** The treatment of malignant tumors by repeated inoculations of erysipelas. With a report of ten original cases. *Clin Orthop.,* 1991, vol. 262, 3-11
- Principles and practice of the biologic therapy of cancer. Lippincott Williams & Wilkins, 2000
- **Parrish-Novak J et al.** *Nature,* 2000, vol. 408, 57-63
- Clinical Research Associates Manual. Southwest Oncology Group, 06 October 1998
- **Sievers EL et al.** *J Clin Oncol.,* 2001, vol. 19, 3244-54
- **Bernstein ID.** *Clin. Lymphoma,* 2002, S9-S11
- **Kaminski MS et al.** *J. Clin. Oncol.,* 2001, vol. 19, 3918-28
- **Gordon LI et al.** *Semin. Oncol.,* 2002, 87-92
- **Lynch TJ Jr et al.** *J. Clin. Oncol.,* 1997, vol. 15, 723-34
- **Talpur R et al.** *Leuk. Lymphoma,* 2002, vol. 43, 121-6
- **Rapley R.** *Mol. Biotechnol.,* 1995, vol. 3, 139-54
- **Foss FM.** *Clin. Lymphoma,* 2000, 27-31
- **Atkins MB et al.** *J. Clin. Oncol.,* 1999, vol. 17, 2105-16
- **Fyfe G et al.** *J. Clin. Oncol.,* 1995, vol. 13, 688-96
- **Jonasch E ; Haluska FG.** *Oncologist,* 2001, vol. 6, 34-55
- **Ehrke MJ et al.** *Cancer Immunol. Immunother.,* 1996, vol. 42, 221-30
- **Younes E et al.** *Cell Immunol.,* 1995, vol. 165, 243-51
- **Nishisaka N et al.** *Cytokines Cell Mol Ther.,* 2000, vol. 6, 199-206
- **Porrata LF et al.** *Bone Marrow Transplant.,* 2001, vol. 28, 673-80
- **Slavin S ; Nagler A.** *Cancer J. Sci. Am.,* 1997, 59-67
- **Fefer A et al.** *Cancer J. Sci. Am.,* 1997, 48-53
- **Shinohara H et al.** *Clin. Cancer Res.,* 1999, vol. 5, 2148-56
- **Cao S et al.** *Cancer Res.,* 1998, vol. 58, 3270-4
- **Cao S et al.** *Cancer Res.,* 1998, vol. 58, 1695-9
- **Alpdogan O et al.** *Blood,* 2001, vol. 98, 2256-65
- **Mackall CL et al.** *Blood,* 2001, vol. 97, 1491-7
- **Lord, BI et al.** *Clin. Cancer Res.,* 2001, vol. 7, 2085-90
- **Holmes FA et al.** *J. Clin. Oncol.,* 2002, vol. 20, 727-31
- **Zaki MH et al.** *J. Invest. Dermatol.,* 2002, vol. 118, 366-71
- **Li D et al.** *Arch. Otolaryngol. Head Neck Surg.,* 2001, vol. 127, 1319-24
- **Hiraki A et al.** *Lung Cancer,* 2002, vol. 35, 329-33
- **Wilcox RA et al.** *J. Clin. Invest.,* 2002, vol. 109, 651-9
- **Chambers CA et al.** *Ann. Rev. Immunol.,* 2001, vol. 19, 565-94
- **Takeda K et al.** *J. Exp. Med.,* 2002, vol. 195, 161-9
- **Srivastava RK.** *Neoplasia,* 2001, vol. 3, 535-46
- **Keilholz U et al.** *Leuk. Lymphoma,* 1999, vol. 35, 641-2
- **Ansell SM et al.** *Blood,* 2002, vol. 99, 67-74

- **Carson WE et al.** *J. Immunol.,* 2001, vol. 31, 3016-25
- **Sacchi S et al.** *Haematologica,* 2001, vol. 86, 951-8
- **Dudley ME et al.** *J. Immunother.,* 2001, vol. 24, 363-73
- **Bear HD et al.** *Cancer Immunol. Immunother.,* 2001, vol. 50, 269-74
- **Schultze JL et al.** *Br. J. Haematol.,* 2001, vol. 113, 455-60
- **Antonia SJ et al.** *J.Urol.,* 2002, vol. 167, 1995-2000
- **Schrayer DP et al.** *Clin. Exp. Metastasis,* 2002, vol. 19, 43-53
- **Niethammer AG et al.** *Cancer Res.,* 2001, vol. 61, 6178-84
- **Shimizu K et al.** *Proc. Nat. Acad. Sci U S A,* 1999, vol. 96, 2268-73
- **Tsuchiya et al.** *Int. J. Cancer,* 1980, vol. 26, 171-176
- **Sundstrom et al.** *Int. J. Cancer,* 1976, vol. 17, 565-577
- Fundamental Immunology. Lippincott-Raven, 1999
- Interferons and other cytokines. Fields Fundamental Virology. Lippincott-Raven Publishers Philadelphia, 1996, 341-365
- **Dowdle WR ; Orenstein WA.** *Proc Nat. Acad. Sci. USA.,* 1994, vol. 91, 2464-8
- **Meissner HC.** *J. Pediatr.,* 1994, vol. 124, 17-21, J. Pediatr.
- **Reichman RC et al.** *Ann. Intem. Med.,* 1988, vol. 108, 675-9
- **Davis GL et al.** *New Engl. J. Med.,* 1998, vol. 339, 1493-9
- **Kasaian MT et al.** *Immunity,* 2002, vol. 16, 559-69
- **Perry CM ; Jarvis B.** *Drugs,* 2001, vol. 61, 2263-88
- **Mitsuyasu R.** *J Infect Dis.,* 2002, vol. 185 (2), 5115-22
- **Ross RW et al.** *Expert Opin Biol Ther.,* 2001, vol. 1, 413-24
- **Faro A.** *Springer Semin Immunopathol.,* 1998, vol. 20, 425-36
- **Baiocchi RA et al.** *J Clin. Invest.,* 2001, vol. 108, 887-94
- **Maddrey WC.** *Semin. Liver. Dis.,* 1999, vol. 19 (1), 67-75
- **Yatsuhashi et al.** *J Hepatol Jun.,* 1999, vol. 30 (6), 995-1003
- **Mathai et al.** *J. Interferon Cytokine Res Sep.,* 1999, vol. 19 (9), 1011-8
- **Fukuda et al.** *J Med Virol Mar.,* 2001, vol. 63 (3), 220-7
- **Dupont SA et al.** *J. Interferon Cytokine Res. Apr,* 2002, vol. 22 (4), 491-501
- **Tan, JT et al.** *J Immunol.,* 1999, vol. 163, 4859-68
- **Hasan MS et al.** *J Infect Dis.,* 1999, vol. 180, 2023-6
- **Evans TG et al.** *Clin Nephrol.,* 2000, vol. 54, 138-42
- **Shimizu K et al.** *Proc. Nat. Acad. Sci. U S A.,* 1999, vol. 96, 2268-73
- **Whiteside.** *Curr. Top. Microbiol. Immunol.,* 1998, vol. 230, 221-244
- **Wedemleyer et al.** *J. Immunol.,* 2002, vol. 169, 3447-3458
- **Vrielink et al.** *Transfusion,* 1997, vol. 37, 845-849
- **Pawlotsky et al.** *Hepatology,* 1998, vol. 27, 1700-1702
- **Poynard et al.** *Lancet,* 1998, vol. 352, 1426-1432
- **McHutchinson et al.** *N. Engl. J. Med.,* 1998, vol. 339, 1485-1492
- National Institutes of Health Consensus Development Conference Panel. *Hepatology,* 1997, vol. 26 (1), 2S-10S
- **Yano.** *Hepatology,* 1996, vol. 23, 1334-1340
- **Lauer et al.** *N. Engl. J. Med.,* 2001, vol. 345, 41-52
- **Tannant et al.** *ILAR J.,* 2000, vol. 42 (2), 89-102200
- **Barker et al.** *J. Infect. Dis.,* 1975, vol. 132, 451-458
- **Tabor et al.** *J. Infect. Dis.,* 1983, vol. 147, 531-534
- **Prince et al.** Vaccines. Cold Spring Harbor Laboratory Press, 1997, vol. 97
- **Hirsch et al.** *Adv. Pharmcol.,* 2000, vol. 49, 437-477
- **Nathanson et al.** *AIDS,* 1999, vol. 13 (A), Sl 13-S120
- **Sibal et al.** *ILAR J.,* 2001, vol. 42 (2), 74-84
- Remington: The Science and Practice of Pharmacy. Mack Publishing Co, 1995
- **Delgado et al.** *Critical Reviews in Therapeutic Drug Carrier Systems,* 1992, vol. 9, 249
- **Spreafico.** *Clin. Pharmacokinet.,* 1994, vol. 27, 290
- **Francis et al.** *Int J Hematol,* 1998, vol. 68, 1
- **Hogan, B. et al.** Manipulating the Mouse Embryo. A Laboratory Manual. Cold Spring Harbor Laboratory Press, 1994
- **Donohue JH ; Rosenberg SA.** *J Immunol.,* 1983, vol. 130, 2203
- **Staud, F. et al.** *J. Pharm. Sciences,* 1999, vol. 88, 577-585
- **Lanier, LL.** *Annu. Rev. Immunol.,* 1998, vol. 16, 359-393
- **Heid, CA et al.** *Genome Research,* 1996, vol. 6, 986-994

- **Gibson, UEM et al.** *Genome Research,* 1996, vol. 6, 995-1001
- **Sundaresan, S et al.** *Endocrinology,* 1998, vol. 139, 4756-4764
- *Relative Quantitation of Gene Expression,* 11 December 1997
- **Stirpe et al.** *Biotechnology,* 1992, vol. 10, 405-412
- *J. Biochem.,* 1991, vol. 23, 352-360
- *Biochemistry,* 1991, vol. 30, 1259-1264
- *Biochemistry,* 1996, vol. 35, 11503-11511
- **Lentsch AB et al.** *Cancer Immunol. Immunother.,* 1999, vol. 47, 243
- **Anderson JA et al.** *J. Clin. Invest.,* 1996, vol. 97, 1952
- **Dubinett SM et al.** *Cell. Immunol.,* 1994, vol. 157, 170
- **Anderson TD et al.** *Lab. Invest.,* 1988, vol. 59, 598
- **Gately, MK et al.** *J. Immunol.,* 1988, vol. 141, 189
- **Inaba, K. et al.** *J. Exp. Med.,* 1992, vol. 176, 1693-1702
- **Shrikant, P ; Mescher, M.** *J. Immunology,* 1999, vol. 162, 2858-2866
- **Cerwenka et al.** *Proc. Nat. Acad. Sci.,* 2001, vol. 98, 11521-11526
- **Zhang et al.** *Am. J. of Pathol.,* 2002, vol. 161, 2295-2309
- **Wigginton et al.** *Nat. Cancer Instit.,* 1996, vol. 88, 38-43
- **Yao et al.** *Cancer Res.,* 2003, vol. 63, 586-592
- **Mukherjee et al.** *J. Immunol.,* 2000, vol. 165, 3451-3460
- **Colombo et al.** *Cancer Research,* 2002, vol. 62, 941-946
- **Wherry et al.** *J. Virol.,* 2003, vol. 77, 4911-4927
- **Blattman et al.** *Nature Med.,* 2003, vol. 9 (5), 540-547
- **Hoffman et al.** *J. Immunol,* 2003, vol. 170, 1339-1353
- **Wedemeyer et al.** *J. Immol.,* 2002, vol. 169, 3447-58
- **Gruener et al.** *J. Virol.,* 2001, vol. 75, 5550-58
- **Cramp et al.** *Gastroenterology,* 2000, vol. 118, 346-55
- *Brit. J. Cancer,* 1950, vol. 4, 372

SEQUENCE LISTING

<110> ZymoGenetics, Inc.

<120> USE OF IL-21 IN CANCER AND
OTHER THERAPEUTIC APPLICATIONS

<130> P27150EP-D1-PCT-MRM/PJG

<140> EP 03757366.4
<141> 2003-06-06

<150> US 60/387,127
<151> 2002-06-07

<160> 17

<170> FastSEQ for Windows Version 4.0

<210> 1
<211> 642
<212> DNA
<213> Homo sapiens

<220>
<221> CDS
<222> (47)...(532)

<400> 1
gctgaagtga aaacgagacc aaggtctagc tctactgttg gtactt atg aga tcc 55
Met Arg Ser
1

agt cct ggc aac atg gag agg att gtc atc tgt ctg atg gtc atc ttc 103
Ser Pro Gly Asn Met Glu Arg Ile Val Ile Cys Leu Met Val Ile Phe
5 10 15

ttg ggg aca ctg gtc cac aaa tca agc tcc caa ggt caa gat cgc cac 151
Leu Gly Thr Leu Val His Lys Ser Ser Ser Gln Gly Gln Asp Arg His
20 25 30 35

atg att aga atg cgt caa ctt ata gat att gtt gat cag ctg aaa aat 199
Met Ile Arg Met Arg Gln Leu Ile Asp Ile Val Asp Gln Leu Lys Asn
40 45 50

tat gtg aat gac ttg gtc cct gaa ttt ctg cca gct cca gaa gat gta 247
Tyr Val Asn Asp Leu Val Pro Glu Phe Leu Pro Ala Pro Glu Asp Val
55 60 65

gag aca aac tgt gag tgg tca gct ttt tcc tgt ttt cag aag gcc caa 295
Glu Thr Asn Cys Glu Trp Ser Ala Phe Ser Cys Phe Gln Lys Ala Gln
70 75 80

cta aag tca gca aat aca gga aac aat gaa agg ata atc aat gta tca 343
Leu Lys Ser Ala Asn Thr Gly Asn Asn Glu Arg Ile Ile Asn Val Ser

```
     85                  90                  95

att aaa aag ctg aag agg aaa cca cct tcc aca aat gca ggg aga aga      391
Ile Lys Lys Leu Lys Arg Lys Pro Pro Ser Thr Asn Ala Gly Arg Arg
100                 105                 110                 115

cag aaa cac aga cta aca tgc cct tca tgt gat tct tat gag aaa aaa      439
Gln Lys His Arg Leu Thr Cys Pro Ser Cys Asp Ser Tyr Glu Lys Lys
                120                 125                 130

cca ccc aaa gaa ttc cta gaa aga ttc aaa tca ctt ctc caa aag atg      487
Pro Pro Lys Glu Phe Leu Glu Arg Phe Lys Ser Leu Leu Gln Lys Met
            135                 140                 145

att cat cag cat ctg tcc tct aga aca cac gga agt gaa gat tcc          532
Ile His Gln His Leu Ser Ser Arg Thr His Gly Ser Glu Asp Ser
        150                 155                 160

tgaggatcta acttgcagtt ggacactatg ttacatactc taatatagta gtgaaagtca    592
tttctttgta ttccaagtgg aggagcccta ttaaattata taaagaaata               642
```

<210> 2
<211> 162
<212> PRT
<213> Homo sapiens

<400> 2

```
Met Arg Ser Ser Pro Gly Asn Met Glu Arg Ile Val Ile Cys Leu Met
 1               5                  10                  15
Val Ile Phe Leu Gly Thr Leu Val His Lys Ser Ser Ser Gln Gly Gln
            20                  25                  30
Asp Arg His Met Ile Arg Met Arg Gln Leu Ile Asp Ile Val Asp Gln
        35                  40                  45
Leu Lys Asn Tyr Val Asn Asp Leu Val Pro Glu Phe Leu Pro Ala Pro
    50                  55                  60
Glu Asp Val Glu Thr Asn Cys Glu Trp Ser Ala Phe Ser Cys Phe Gln
65                  70                  75                  80
Lys Ala Gln Leu Lys Ser Ala Asn Thr Gly Asn Asn Glu Arg Ile Ile
                85                  90                  95
Asn Val Ser Ile Lys Lys Leu Lys Arg Lys Pro Pro Ser Thr Asn Ala
            100                 105                 110
Gly Arg Arg Gln Lys His Arg Leu Thr Cys Pro Ser Cys Asp Ser Tyr
        115                 120                 125
Glu Lys Lys Pro Pro Lys Glu Phe Leu Glu Arg Phe Lys Ser Leu Leu
    130                 135                 140
Gln Lys Met Ile His Gln His Leu Ser Ser Arg Thr His Gly Ser Glu
145                 150                 155                 160
Asp Ser
```

<210> 3
<211> 3072
<212> DNA
<213> Mus musculus

<220>

```
<221> CDS
<222> (54)...(491)

<400> 3
gagaaccaga ccaaggccct gtcatcagct cctggagact cagttctggt ggc atg        56
                                                           Met
                                                            1

gag agg acc ctt gtc tgt ctg gta gtc atc ttc ttg ggg aca gtg gcc      104
Glu Arg Thr Leu Val Cys Leu Val Val Ile Phe Leu Gly Thr Val Ala
            5                  10                  15

cat aaa tca agc ccc caa ggg cca gat cgc ctc ctg att aga ctt cgt      152
His Lys Ser Ser Pro Gln Gly Pro Asp Arg Leu Leu Ile Arg Leu Arg
        20                  25                  30

cac ctt att gac att gtt gaa cag ctg aaa atc tat gaa aat gac ttg      200
His Leu Ile Asp Ile Val Glu Gln Leu Lys Ile Tyr Glu Asn Asp Leu
    35                  40                  45

gat cct gaa ctt cta tca gct cca caa gat gta aag ggg cac tgt gag      248
Asp Pro Glu Leu Leu Ser Ala Pro Gln Asp Val Lys Gly His Cys Glu
 50                  55                  60                  65

cat gca gct ttt gcc tgt ttt cag aag gcc aaa ctc aag cca tca aac      296
His Ala Ala Phe Ala Cys Phe Gln Lys Ala Lys Leu Lys Pro Ser Asn
                 70                  75                  80

cct gga aac aat aag aca ttc atc att gac ctc gtg gcc cag ctc agg      344
Pro Gly Asn Asn Lys Thr Phe Ile Ile Asp Leu Val Ala Gln Leu Arg
            85                  90                  95

agg agg ctg cct gcc agg agg gga gga aag aaa cag aag cac ata gct      392
Arg Arg Leu Pro Ala Arg Arg Gly Gly Lys Lys Gln Lys His Ile Ala
        100                 105                 110

aaa tgc cct tcc tgt gat tcg tat gag aaa agg aca ccc aaa gaa ttc      440
Lys Cys Pro Ser Cys Asp Ser Tyr Glu Lys Arg Thr Pro Lys Glu Phe
    115                 120                 125

cta gaa aga cta aaa tgg ctc ctt caa aag atg att cat cag cat ctc      488
Leu Glu Arg Leu Lys Trp Leu Leu Gln Lys Met Ile His Gln His Leu
130                 135                 140                 145

tcc tagaacacat aggacccgaa gattcctgag gatccgagaa gattcccgag           541
Ser


gactgaggag acgccggaca ctatagacgc tcacgaatgc aggagtacat cttgcctctt    601
gggattgcaa gtggagaagt acgatacgtt atgataagaa caactcagaa aagctatagg    661
ttaagatcct ttcgcccatt aactaagcag acattgtggt tccctgcaca gactccatgc    721
tgtcaacatg gaaaatctca actcaacaag agcccagctt cccgtgtcag ggatttctgg    781
tgcttctcaa gctgtggctt catcttattg cccaactgtg acattctttg attggaaggg    841
gaaaactaaa gcttttagca aaaatacagc tagggaattt gtcgatctgc gagagtaaga    901
cctcttatga tcctaacgga atgatgtaag ctggaaataa taagcataag atgaaattga    961
aaattgaagt ctttattctt taagaaaaac tttgtacttg aaagcatgtc tgaagagttt   1021
actcattacc acaaacatct agcatattga taactaacat ctttatactc tacaagagag   1081
```

```
gctttccaga taggtacagt ttttcttctc tattaggtct atcaaaattt aacctattat   1141
gagggtcacc cctggctttc actgtttttc taaagaggca agggtgtagt aagaagcagg   1201
cttaagttgc cttcctccca atgtcaagtt cctttataag ctaatagttt aatcttgtga   1261
agatggcaat gaaagcctgt ggaagtgcaa acctcactat cttctggagc caagtagaat   1321
tttcaagttt gtagctctca cctcaagtgg ttatgggtgt cctgtgatga atctgctagc   1381
tccagcctca gtctcctctc ccacatcctt tcctttcttt cctctttgaa acttctaaga   1441
aaaagcaatc caaacaagtt cagcacttaa gacacattgc atgcacactt ttgataagtt   1501
aaatccaacc atctatttaa aatcaaaatc aggagatgag ccaagagacc agaggttctg   1561
ttccagtttt aaacagactt ttactgaaca tcccaatctt ttaaccacag aggctaaatt   1621
gagcaaatag ttttgccatt tgatataatt tccaacagta tgtttcaatg tcaagttaaa   1681
aagtctacaa agctattttc cctggagtgg tatcatcgct ttgagaattt cttatggtta   1741
aaatggatct gagatccaag catggcctgg gggatggttt tgatctaagg aaaaaggtgt   1801
ctgtacctca cagtgccttt aaaacaagca gagatcccgt gtaccgccct aagatagcac   1861
agactagtgt taactgattc ccagaaaagt gtcacaatca gaaccaacgc attctcttaa   1921
actttaaaaa tatgtattgc aaagaacttg tgtaactgta aatgtgtgac tgttgatgac   1981
attatacaca catagcccac gtaagtgtcc aatggtgcta gcattggttg ctgagtttgc   2041
tgctcgaaag ctgaagcaga gatgcagtcc ttcacaaagc aatgatggac agagagggga   2101
gtctccatgt tttattcttt tgttgtttct ggctgtgtaa ctgttgactt cttgacattg   2161
tgatttttat atttaagaca atgtatttat tttggtgtgt ttattgttct agccttttaa   2221
atcactgaca atttctaatc aagaagtaca aataattcaa tgcagcacag gctaagagct   2281
tgtatcgttt ggaaaagcca gtgaaggctt ctccactagc catgggaaag ctacgcttta   2341
gagtaaacta gacaaaattg cacagcagtc ttgaacctct ctgtgctcaa gactcagcca   2401
gtcctttgac attattgttc actgtgggtg ggaacacatt ggacctgaca cactgttgtg   2461
tgtccatgaa ggttgccact ggtgtaagct ttttttggtt ttcattctct tatctgtaga   2521
acaagaatgt ggggctttcc taagtctatt ctgtatttta ttctgaactt cgtatgtctg   2581
agttttaatg ttttgagtac tcttacagga acacctgacc acacttttga gttaaatttt   2641
atcccaagtg tgatatttag ttgttcaaaa agggaaggga tatacataca tacatacata   2701
catacataca tatatatata tatatataca tatatatata tatatatatg tatatatata   2761
tatatataga gagagagaga gagagagaga gagaaagaga gagaggttgt tgtaggtcat   2821
aggagttcag aggaaatcag ttatggccgt taatactgta gctgaaagtg ttttctttgt   2881
gaataaattc atagcattat tgatctatgt tattgctctg ttttatttac agtcacacct   2941
gagaatttag ttttaatatg aatgatgtac tttataactt aatgattatt tattatgtat   3001
ttggttttga atgtttgtgt tcatggcttc ttatttaaga cctgatcata ttaaatgcta   3061
cccagtccgg a                                                        3072
```

<210> 4
<211> 146
<212> PRT
<213> Mus musculus

<400> 4

```
Met Glu Arg Thr Leu Val Cys Leu Val Val Ile Phe Leu Gly Thr Val
 1               5                  10                  15
Ala His Lys Ser Ser Pro Gln Gly Pro Asp Arg Leu Leu Ile Arg Leu
            20                  25                  30
Arg His Leu Ile Asp Ile Val Glu Gln Leu Lys Ile Tyr Glu Asn Asp
        35                  40                  45
Leu Asp Pro Glu Leu Leu Ser Ala Pro Gln Asp Val Lys Gly His Cys
    50                  55                  60
Glu His Ala Ala Phe Ala Cys Phe Gln Lys Ala Lys Leu Lys Pro Ser
65                  70                  75                  80
Asn Pro Gly Asn Asn Lys Thr Phe Ile Ile Asp Leu Val Ala Gln Leu
                85                  90                  95
Arg Arg Arg Leu Pro Ala Arg Arg Gly Gly Lys Lys Gln Lys His Ile
            100                 105                 110
Ala Lys Cys Pro Ser Cys Asp Ser Tyr Glu Lys Arg Thr Pro Lys Glu
        115                 120                 125
```

```
Phe Leu Glu Arg Leu Lys Trp Leu Leu Gln Lys Met Ile His Gln His
    130                 135                 140
Leu Ser
145


<210> 5
<211> 1614
<212> DNA
<213> Homo sapiens

<220>
<221> CDS
<222> (1)...(1614)

<400> 5
atg ccg cgt ggc tgg gcc gcc ccc ttg ctc ctg ctg ctg ctc cag gga      48
Met Pro Arg Gly Trp Ala Ala Pro Leu Leu Leu Leu Leu Leu Gln Gly
 1               5                  10                  15

ggc tgg ggc tgc ccc gac ctc gtc tgc tac acc gat tac ctc cag acg      96
Gly Trp Gly Cys Pro Asp Leu Val Cys Tyr Thr Asp Tyr Leu Gln Thr
            20                  25                  30


gtc atc tgc atc ctg gaa atg tgg aac ctc cac ccc agc acg ctc acc      144
Val Ile Cys Ile Leu Glu Met Trp Asn Leu His Pro Ser Thr Leu Thr
        35                  40                  45

ctt acc tgg caa gac cag tat gaa gag ctg aag gac gag gcc acc tcc      192
Leu Thr Trp Gln Asp Gln Tyr Glu Glu Leu Lys Asp Glu Ala Thr Ser
    50                  55                  60

tgc agc ctc cac agg tcg gcc cac aat gcc acg cat gcc acc tac acc      240
Cys Ser Leu His Arg Ser Ala His Asn Ala Thr His Ala Thr Tyr Thr
 65                  70                  75                  80

tgc cac atg gat gta ttc cac ttc atg gcc gac gac att ttc agt gtc      288
Cys His Met Asp Val Phe His Phe Met Ala Asp Asp Ile Phe Ser Val
                85                  90                  95

aac atc aca gac cag tct ggc aac tac tcc cag gag tgt ggc agc ttt      336
Asn Ile Thr Asp Gln Ser Gly Asn Tyr Ser Gln Glu Cys Gly Ser Phe
            100                 105                 110

ctc ctg gct gag agc atc aag ccg gct ccc cct ttc aac gtg act gtg      384
Leu Leu Ala Glu Ser Ile Lys Pro Ala Pro Pro Phe Asn Val Thr Val
        115                 120                 125

acc ttc tca gga cag tat aat atc tcc tgg cgc tca gat tac gaa gac      432
Thr Phe Ser Gly Gln Tyr Asn Ile Ser Trp Arg Ser Asp Tyr Glu Asp
    130                 135                 140

cct gcc ttc tac atg ctg aag ggc aag ctt cag tat gag ctg cag tac      480
Pro Ala Phe Tyr Met Leu Lys Gly Lys Leu Gln Tyr Glu Leu Gln Tyr
145                 150                 155                 160
```

```
agg aac cgg gga gac ccc tgg gct gtg agt ccg agg aga aag ctg atc     528
Arg Asn Arg Gly Asp Pro Trp Ala Val Ser Pro Arg Arg Lys Leu Ile
                165                 170                 175

tca gtg gac tca aga agt gtc tcc ctc ctc ccc ctg gag ttc cgc aaa     576
Ser Val Asp Ser Arg Ser Val Ser Leu Leu Pro Leu Glu Phe Arg Lys
            180                 185                 190

gac tcg agc tat gag ctg cag gtg cgg gca ggg ccc atg cct ggc tcc     624
Asp Ser Ser Tyr Glu Leu Gln Val Arg Ala Gly Pro Met Pro Gly Ser
        195                 200                 205

tcc tac cag ggg acc tgg agt gaa tgg agt gac ccg gtc atc ttt cag     672
Ser Tyr Gln Gly Thr Trp Ser Glu Trp Ser Asp Pro Val Ile Phe Gln
    210                 215                 220

acc cag tca gag gag tta aag gaa ggc tgg aac cct cac ctg ctg ctt     720
Thr Gln Ser Glu Glu Leu Lys Glu Gly Trp Asn Pro His Leu Leu Leu
225                 230                 235                 240

ctc ctc ctg ctt gtc ata gtc ttc att cct gcc ttc tgg agc ctg aag     768
Leu Leu Leu Leu Val Ile Val Phe Ile Pro Ala Phe Trp Ser Leu Lys
                245                 250                 255

acc cat cca ttg tgg agg cta tgg aag aag ata tgg gcc gtc ccc agc     816
Thr His Pro Leu Trp Arg Leu Trp Lys Lys Ile Trp Ala Val Pro Ser
            260                 265                 270

cct gag cgg ttc ttc atg ccc ctg tac aag ggc tgc agc gga gac ttc     864
Pro Glu Arg Phe Phe Met Pro Leu Tyr Lys Gly Cys Ser Gly Asp Phe
        275                 280                 285

aag aaa tgg gtg ggt gca ccc ttc act ggc tcc agc ctg gag ctg gga     912
Lys Lys Trp Val Gly Ala Pro Phe Thr Gly Ser Ser Leu Glu Leu Gly
    290                 295                 300

ccc tgg agc cca gag gtg ccc tcc acc ctg gag gtg tac agc tgc cac     960
Pro Trp Ser Pro Glu Val Pro Ser Thr Leu Glu Val Tyr Ser Cys His
305                 310                 315                 320

cca cca cgg agc ccg gcc aag agg ctg cag ctc acg gag cta caa gaa     1008
Pro Pro Arg Ser Pro Ala Lys Arg Leu Gln Leu Thr Glu Leu Gln Glu
                325                 330                 335

cca gca gag ctg gtg gag tct gac ggt gtg ccc aag ccc agc ttc tgg     1056
Pro Ala Glu Leu Val Glu Ser Asp Gly Val Pro Lys Pro Ser Phe Trp
            340                 345                 350

ccg aca gcc cag aac tcg ggg ggc tca gct tac agt gag gag agg gat     1104
Pro Thr Ala Gln Asn Ser Gly Gly Ser Ala Tyr Ser Glu Glu Arg Asp
        355                 360                 365

cgg cca tac ggc ctg gtg tcc att gac aca gtg act gtg cta gat gca     1152
Arg Pro Tyr Gly Leu Val Ser Ile Asp Thr Val Thr Val Leu Asp Ala
    370                 375                 380
```

```
gag ggg cca tgc acc tgg ccc tgc agc tgt gag gat gac ggc tac cca      1200
Glu Gly Pro Cys Thr Trp Pro Cys Ser Cys Glu Asp Asp Gly Tyr Pro
385                 390                 395                 400

gcc ctg gac ctg gat gct ggc ctg gag ccc agc cca ggc cta gag gac      1248
Ala Leu Asp Leu Asp Ala Gly Leu Glu Pro Ser Pro Gly Leu Glu Asp
                405                 410                 415

cca ctc ttg gat gca ggg acc aca gtc ctg tcc tgt ggc tgt gtc tca      1296
Pro Leu Leu Asp Ala Gly Thr Thr Val Leu Ser Cys Gly Cys Val Ser
            420                 425                 430

gct ggc agc cct ggg cta gga ggg ccc ctg gga agc ctc ctg gac aga      1344
Ala Gly Ser Pro Gly Leu Gly Gly Pro Leu Gly Ser Leu Leu Asp Arg
        435                 440                 445

cta aag cca ccc ctt gca gat ggg gag gac tgg gct ggg gga ctg ccc      1392
Leu Lys Pro Pro Leu Ala Asp Gly Glu Asp Trp Ala Gly Gly Leu Pro
    450                 455                 460

tgg ggt ggc cgg tca cct gga ggg gtc tca gag agt gag gcg ggc tca      1440
Trp Gly Gly Arg Ser Pro Gly Gly Val Ser Glu Ser Glu Ala Gly Ser
465                 470                 475                 480

ccc ctg gcc ggc ctg gat atg gac acg ttt gac agt ggc ttt gtg ggc      1488
Pro Leu Ala Gly Leu Asp Met Asp Thr Phe Asp Ser Gly Phe Val Gly
                485                 490                 495

tct gac tgc agc agc cct gtg gag tgt gac ttc acc agc ccc ggg gac      1536
Ser Asp Cys Ser Ser Pro Val Glu Cys Asp Phe Thr Ser Pro Gly Asp
            500                 505                 510

gaa gga ccc ccc cgg agc tac ctc cgc cag tgg gtg gtc att cct ccg      1584
Glu Gly Pro Pro Arg Ser Tyr Leu Arg Gln Trp Val Val Ile Pro Pro
        515                 520                 525

cca ctt tcg agc cct gga ccc cag gcc agc                              1614
Pro Leu Ser Ser Pro Gly Pro Gln Ala Ser
    530                 535


<210> 6
<211> 538
<212> PRT
<213> Homo sapiens

<400> 6
Met Pro Arg Gly Trp Ala Ala Pro Leu Leu Leu Leu Leu Leu Gln Gly
 1               5                  10                  15
Gly Trp Gly Cys Pro Asp Leu Val Cys Tyr Thr Asp Tyr Leu Gln Thr
            20                  25                  30
Val Ile Cys Ile Leu Glu Met Trp Asn Leu His Pro Ser Thr Leu Thr
        35                  40                  45
Leu Thr Trp Gln Asp Gln Tyr Glu Glu Leu Lys Asp Glu Ala Thr Ser
    50                  55                  60
Cys Ser Leu His Arg Ser Ala His Asn Ala Thr His Ala Thr Tyr Thr
65                  70                  75                  80
```

```
Cys His Met Asp Val Phe His Phe Met Ala Asp Asp Ile Phe Ser Val
                85                  90                  95
Asn Ile Thr Asp Gln Ser Gly Asn Tyr Ser Gln Glu Cys Gly Ser Phe
            100                 105                 110
Leu Leu Ala Glu Ser Ile Lys Pro Ala Pro Pro Phe Asn Val Thr Val
        115                 120                 125
Thr Phe Ser Gly Gln Tyr Asn Ile Ser Trp Arg Ser Asp Tyr Glu Asp
    130                 135                 140
Pro Ala Phe Tyr Met Leu Lys Gly Lys Leu Gln Tyr Glu Leu Gln Tyr
145                 150                 155                 160
Arg Asn Arg Gly Asp Pro Trp Ala Val Ser Pro Arg Arg Lys Leu Ile
                165                 170                 175
Ser Val Asp Ser Arg Ser Val Ser Leu Leu Pro Leu Glu Phe Arg Lys
            180                 185                 190
Asp Ser Ser Tyr Glu Leu Gln Val Arg Ala Gly Pro Met Pro Gly Ser
        195                 200                 205
Ser Tyr Gln Gly Thr Trp Ser Glu Trp Ser Asp Pro Val Ile Phe Gln
    210                 215                 220
Thr Gln Ser Glu Glu Leu Lys Glu Gly Trp Asn Pro His Leu Leu Leu
225                 230                 235                 240
Leu Leu Leu Leu Val Ile Val Phe Ile Pro Ala Phe Trp Ser Leu Lys
                245                 250                 255
Thr His Pro Leu Trp Arg Leu Trp Lys Lys Ile Trp Ala Val Pro Ser
            260                 265                 270

Pro Glu Arg Phe Phe Met Pro Leu Tyr Lys Gly Cys Ser Gly Asp Phe
        275                 280                 285
Lys Lys Trp Val Gly Ala Pro Phe Thr Gly Ser Ser Leu Glu Leu Gly
    290                 295                 300
Pro Trp Ser Pro Glu Val Pro Ser Thr Leu Glu Val Tyr Ser Cys His
305                 310                 315                 320
Pro Pro Arg Ser Pro Ala Lys Arg Leu Gln Leu Thr Glu Leu Gln Glu
                325                 330                 335
Pro Ala Glu Leu Val Glu Ser Asp Gly Val Pro Lys Pro Ser Phe Trp
            340                 345                 350
Pro Thr Ala Gln Asn Ser Gly Gly Ser Ala Tyr Ser Glu Glu Arg Asp
        355                 360                 365
Arg Pro Tyr Gly Leu Val Ser Ile Asp Thr Val Thr Val Leu Asp Ala
    370                 375                 380
Glu Gly Pro Cys Thr Trp Pro Cys Ser Cys Glu Asp Asp Gly Tyr Pro
385                 390                 395                 400
Ala Leu Asp Leu Asp Ala Gly Leu Glu Pro Ser Pro Gly Leu Glu Asp
                405                 410                 415
Pro Leu Leu Asp Ala Gly Thr Thr Val Leu Ser Cys Gly Cys Val Ser
            420                 425                 430
Ala Gly Ser Pro Gly Leu Gly Gly Pro Leu Gly Ser Leu Leu Asp Arg
        435                 440                 445
Leu Lys Pro Pro Leu Ala Asp Gly Glu Asp Trp Ala Gly Gly Leu Pro
    450                 455                 460
Trp Gly Gly Arg Ser Pro Gly Gly Val Ser Glu Ser Glu Ala Gly Ser
465                 470                 475                 480
Pro Leu Ala Gly Leu Asp Met Asp Thr Phe Asp Ser Gly Phe Val Gly
                485                 490                 495
Ser Asp Cys Ser Ser Pro Val Glu Cys Asp Phe Thr Ser Pro Gly Asp
            500                 505                 510
Glu Gly Pro Pro Arg Ser Tyr Leu Arg Gln Trp Val Val Ile Pro Pro
        515                 520                 525
```

```
Pro Leu Ser Ser Pro Gly Pro Gln Ala Ser
    530                 535


<210> 7
<211> 153
<212> PRT
<213> Homo sapiens

<400> 7

Met Tyr Arg Met Gln Leu Leu Ser Cys Ile Ala Leu Ser Leu Ala Leu
 1               5                  10                  15
Val Thr Asn Ser Ala Pro Thr Ser Ser Ser Thr Lys Lys Thr Gln Leu
            20                  25                  30
Gln Leu Glu His Leu Leu Leu Asp Leu Gln Met Ile Leu Asn Gly Ile
        35                  40                  45
Asn Asn Tyr Lys Asn Pro Lys Leu Thr Arg Met Leu Thr Phe Lys Phe
    50                  55                  60
Tyr Met Pro Lys Lys Ala Thr Glu Leu Lys His Leu Gln Cys Leu Glu
65                  70                  75                  80
Glu Glu Leu Lys Pro Leu Glu Glu Val Leu Asn Leu Ala Gln Ser Lys
                85                  90                  95
Asn Phe His Leu Arg Pro Arg Asp Leu Ile Ser Asn Ile Asn Val Ile
            100                 105                 110
Val Leu Glu Leu Lys Gly Ser Glu Thr Thr Phe Met Cys Glu Tyr Ala
        115                 120                 125
Asp Glu Thr Ala Thr Ile Val Glu Phe Leu Asn Arg Trp Ile Thr Phe
    130                 135                 140
Cys Gln Ser Ile Ile Ser Thr Leu Thr
145                 150


<210> 8
<211> 153
<212> PRT
<213> Homo sapiens

<400> 8
Met Gly Leu Thr Ser Gln Leu Leu Pro Pro Leu Phe Phe Leu Leu Ala
 1               5                  10                  15
Cys Ala Gly Asn Phe Val His Gly His Lys Cys Asp Ile Thr Leu Gln
            20                  25                  30
Glu Ile Ile Lys Thr Leu Asn Ser Leu Thr Glu Gln Lys Thr Leu Cys
        35                  40                  45
Thr Glu Leu Thr Val Thr Asp Ile Phe Ala Ala Ser Lys Asn Thr Thr
    50                  55                  60
Glu Lys Glu Thr Phe Cys Arg Ala Ala Thr Val Leu Arg Gln Phe Tyr
65                  70                  75                  80
Ser His His Glu Lys Asp Thr Arg Cys Leu Gly Ala Thr Ala Gln Gln
                85                  90                  95
Phe His Arg His Lys Gln Leu Ile Arg Phe Leu Lys Arg Leu Asp Arg
            100                 105                 110

Asn Leu Trp Gly Leu Ala Gly Leu Asn Ser Cys Pro Val Lys Glu Ala
        115                 120                 125
Asn Gln Ser Thr Leu Glu Asn Phe Leu Glu Arg Leu Lys Thr Ile Met
```

```
    130                 135                 140
Arg Glu Lys Tyr Ser Lys Cys Ser Ser
145                 150
```

<210> 9
<211> 162
<212> PRT
<213> Homo sapiens

<400> 9

```
Met Arg Ile Ser Lys Pro His Leu Arg Ser Ile Ser Ile Gln Cys Tyr
 1               5                  10                  15
Leu Cys Leu Leu Leu Asn Ser His Phe Leu Thr Glu Ala Gly Ile His
            20                  25                  30
Val Phe Ile Leu Gly Cys Phe Ser Ala Gly Leu Pro Lys Thr Glu Ala
        35                  40                  45
Asn Trp Val Asn Val Ile Ser Asp Leu Lys Lys Ile Glu Asp Leu Ile
    50                  55                  60
Gln Ser Met His Ile Asp Ala Thr Leu Tyr Thr Glu Ser Asp Val His
65                  70                  75                  80
Pro Ser Cys Lys Val Thr Ala Met Lys Cys Phe Leu Leu Glu Leu Gln
                85                  90                  95
Val Ile Ser Leu Glu Ser Gly Asp Ala Ser Ile His Asp Thr Val Glu
            100                 105                 110
Asn Leu Ile Ile Leu Ala Asn Asn Ser Leu Ser Ser Asn Gly Asn Val
        115                 120                 125
Thr Glu Ser Gly Cys Lys Glu Cys Glu Glu Leu Glu Glu Lys Asn Ile
    130                 135                 140
Lys Glu Phe Leu Gln Ser Phe Val His Ile Val Gln Met Phe Ile Asn
145                 150                 155                 160
Thr Ser
```

<210> 10
<211> 144
<212> PRT
<213> Homo sapiens

<400> 10

```
Met Trp Leu Gln Ser Leu Leu Leu Leu Gly Thr Val Ala Cys Ser Ile
 1               5                  10                  15
Ser Ala Pro Ala Arg Ser Pro Ser Pro Ser Thr Gln Pro Trp Glu His
            20                  25                  30
Val Asn Ala Ile Gln Glu Ala Arg Arg Leu Leu Asn Leu Ser Arg Asp
        35                  40                  45
Thr Ala Ala Glu Met Asn Glu Thr Val Glu Val Ile Ser Glu Met Phe
    50                  55                  60
Asp Leu Gln Glu Pro Thr Cys Leu Gln Thr Arg Leu Glu Leu Tyr Lys
65                  70                  75                  80
Gln Gly Leu Arg Gly Ser Leu Thr Lys Leu Lys Gly Pro Leu Thr Met
                85                  90                  95
Met Ala Ser His Tyr Lys Gln His Cys Pro Pro Thr Pro Glu Thr Ser
            100                 105                 110
Cys Ala Thr Gln Ile Ile Thr Phe Glu Ser Phe Lys Glu Asn Leu Lys
        115                 120                 125
```

Asp Phe Leu Leu Val Ile Pro Phe Asp Cys Trp Glu Pro Val Gln Glu
    130                 135                 140

<210> 11
<211> 22
<212> DNA
<213> Artificial Sequence

<220>
<223> Oligonucleotide primer ZC22281

<400> 11
tgtgaatgac ttggtccctg aa 22

<210> 12
<211> 23
<212> DNA
<213> Artificial Sequence

<220>
<223> Oligonucleotide primer ZC22279

<400> 12
aacaggaaaa agctgaccac tca 23

<210> 13
<211> 31
<212> DNA
<213> Artificial Sequence

<220>
<223> zalpha11 Ligand TaqMan probe, ZG32

<400> 13
tctgccagct ccagaagatg tagagacaaa c 31

<210> 14
<211> 20
<212> DNA
<213> Artificial Sequence

<220>
<223> Oligonucleotide primer ZC22277

<400> 14
ccaggagtgt ggcagctttc 20

<210> 15
<211> 21
<212> DNA
<213> Artificial Sequence

<220>
<223> Oligonucleotide primer ZC22276

<400> 15

```
gcttgccctt cagcatgtag a                                                21

<210> 16
<211> 23
<212> DNA
<213> Artificial Sequence

<220>
<223> zalpha11 TaqMan(r) probe, designated ZG31

<400> 16
cggctccccc tttcaacgtg act                                              23

<210> 17
<211> 8
<212> PRT
<213> Artificial Sequence

<220>
<223> OVA257-264 peptide

<400> 17
Ser Ile Ile Asn Phe Glu Lys Leu
 1               5
```